# EUROPEAN PATENT APPLICATION

(11) **EP 3 591 053 A1**
(43) Date of publication of application: **08.01.2020**
(21) Application number: 19180054.9
(22) Date of filing: 04.07.2014
(51) Int. Cl.: C12N 15/113, C07H 21/02, A61K 48/00, A61P 11/00

(54) **RESPIRATORY DISEASE-RELATED GENE SPECIFIC SIRNA, DOUBLE-HELICAL OLIGO RNA STRUCTURE CONTAINING SIRNA, COMPOSITON CONTAINING SAME FOR PREVENTING OR TREATING RESPIRATORY DISEASE**

(30) Priority: 05.07.2013 KR 20130079311
(62) Divisional of application: 14820458.9
(71) Applicant: Bioneer Corporation, Daejeon 306-220 (KR); Yuhan Corporation, Seoul 06927 (KR)
(72) Inventor: CHAE, Jeiwook, Daejeon 305-761 (KR); PARK, Han Oh, Daejeon 305-761 (KR); YOON, Pyoung Oh, Daejeon 305-751 (KR); HAN, Boram, Gyeonggi-do 422-753 (KR); KIM, Mi Na, Daejeon 305-770 (KR)
(74) Representative: dompatent von Kreisler Selting Werner - Partnerschaft von Patent- und Rechtsanwälten mbB

(57) **Abstract**

The present invention relates to respiratory diseases, especially to gene specific siRNA related to idiopathic pulmonary fibrosis and chronic obstructive pulmonary disease (COPD), and a highly efficient double-helical oligo RNA structure containing the same, wherein the double-helical oligo RNA structure has a structure in which hydrophilic and hydrophobic materials are bonded at the both ends of the double-helical RNA (siRNA) using a simple covalent bond or a linker-mediated covalent bond to be effectively transferred into a cell, and is converted into nanoparticles by the hydrophobic interaction of the double-helical oligo RNA structure in a solution. It is desirable that the siRNA contained in the double-helical oligo RNA structure is a siRNA specific to a CTGF, Cyr61, or Plekhol, which are genes related to respiratory diseases, particularly idiopathic pulmonary fibrosis and COPD. In addition, the present invention relates to a method for producing the double-helical oligo RNA structure and a pharmaceutical composition containing the double-helical oligo RNA structure for preventing or treating respiratory diseases, particularly idiopathic pulmonary fibrosis and COPD.

SEQUENCE LISTING

<110>  BIONEER CORPORATION
       YUHAN CORPORATION

<120>  Respiratory disease-related gene specific siRNA, double-helical
        oligo RNA structure containing siRNA, composition containing
        same for preventing or treating respiratory disease

<130>  PF-B1676-EP-D1

<140>  PCT/KR2014/006033
<141>  2014-07-04

<150>  10-2013-0079311
<151>  2013-07-05

<160>  604

<170>  PatentIn version 3.2

<210>  1
<211>  23
<212>  DNA
<213>  Artificial

<220>
<223>  hCTGF

<400>  1
atgtgcattc tccagccatc aag                                              23


<210>  2
<211>  23
<212>  DNA
<213>  Artificial

<220>
<223>  hCTGF

<400>  2
ttctgaacac cataggtaga atg                                              23


<210>  3
<211>  23
<212>  DNA
<213>  Artificial

<220>
<223>  hCTGF

<400>  3
gtctgatcgt tcaaagcatg aaa                                              23


<210>  4
<211>  23
<212>  DNA
<213>  Artificial

<220>

<223>  hCTGF

<400>  4
tatgactgtt tttcggacag ttt                                              23


<210>  5
<211>  23
<212>  DNA
<213>  Artificial

<220>
<223>  hCTGF

<400>  5
gggaaaagat tcccacccaa ttc                                              23


<210>  6
<211>  23
<212>  DNA
<213>  Artificial

<220>
<223>  hCTGF

<400>  6
gagacatggc atgaagccag aga                                              23


<210>  7
<211>  23
<212>  DNA
<213>  Artificial

<220>
<223>  hCTGF

<400>  7
ttcacatctc atttttccgt aaa                                              23


<210>  8
<211>  23
<212>  DNA
<213>  Artificial

<220>
<223>  hCTGF

<400>  8
tacaactgtc ccggagacaa tga                                              23


<210>  9
<211>  23
<212>  DNA
<213>  Artificial

<220>
<223>  hCTGF

<400>  9

ttgtctgatc gttcaaagca tga                                              23


<210>  10
<211>  23
<212>  DNA
<213>  Artificial

<220>
<223>  hCTGF

<400>  10
atgatttcag tagcacaagt tat                                              23


<210>  11
<211>  23
<212>  DNA
<213>  Artificial

<220>
<223>  hCTGF

<400>  11
taccagcaga aaggttagta tca                                              23


<210>  12
<211>  23
<212>  DNA
<213>  Artificial

<220>
<223>  hCTGF

<400>  12
cggagacatg gcatgaagcc aga                                              23


<210>  13
<211>  23
<212>  DNA
<213>  Artificial

<220>
<223>  hCTGF

<400>  13
ttgatatgac tgtttttcgg aca                                              23


<210>  14
<211>  23
<212>  DNA
<213>  Artificial

<220>
<223>  hCTGF

<400>  14
gagagacatt aactcattag act                                              23

<210>  15
<211>  23
<212>  DNA
<213>  Artificial

<220>
<223>  hCTGF

<400>  15
gagtgagaga cattaactca tta                                              23


<210>  16
<211>  23
<212>  DNA
<213>  Artificial

<220>
<223>  hCTGF

<400>  16
gtcgattaga ctggacagct tgt                                              23


<210>  17
<211>  23
<212>  DNA
<213>  Artificial

<220>
<223>  hCTGF

<400>  17
aagttacatg tttgcacctt tct                                              23


<210>  18
<211>  23
<212>  DNA
<213>  Artificial

<220>
<223>  hCTGF

<400>  18
aaaacattgt gccatgtcaa aca                                              23


<210>  19
<211>  23
<212>  DNA
<213>  Artificial

<220>
<223>  hCTGF

<400>  19
taagacttga cagtggaact aca                                              23


<210>  20
<211>  23
<212>  DNA

<213>  Artificial

<220>
<223>  hCTGF

<400>  20
gaacaccata ggtagaatgt aaa                                               23


<210>  21
<211>  23
<212>  DNA
<213>  Artificial

<220>
<223>  hCTGF

<400>  21
ggacagcttg tggcaagtga att                                               23


<210>  22
<211>  23
<212>  DNA
<213>  Artificial

<220>
<223>  hCTGF

<400>  22
ccctgccggt ggagttcaag tgc                                               23


<210>  23
<211>  23
<212>  DNA
<213>  Artificial

<220>
<223>  hCTGF

<400>  23
gcccagaccc aactatgatt aga                                               23


<210>  24
<211>  23
<212>  DNA
<213>  Artificial

<220>
<223>  hCTGF

<400>  24
aacaccatag gtagaatgta aag                                               23


<210>  25
<211>  23
<212>  DNA
<213>  Artificial

<220>

<223>  hCTGF

<400>  25
gtccttggca ggctgatttc tag                                              23


<210>  26
<211>  23
<212>  DNA
<213>  Artificial

<220>
<223>  hCTGF

<400>  26
catcttcggt ggtacggtgt acc                                              23


<210>  27
<211>  23
<212>  DNA
<213>  Artificial

<220>
<223>  hCTGF

<400>  27
agacttgaca gtggaactac att                                              23


<210>  28
<211>  23
<212>  DNA
<213>  Artificial

<220>
<223>  hCTGF

<400>  28
tttctgaaca ccataggtag aat                                              23


<210>  29
<211>  23
<212>  DNA
<213>  Artificial

<220>
<223>  hCTGF

<400>  29
cagcaccaga atgtatatta agg                                              23


<210>  30
<211>  23
<212>  DNA
<213>  Artificial

<220>
<223>  hCTGF

<400>  30

tagtatcatc agatagcatc tta                                              23

<210>  31
<211>  23
<212>  DNA
<213>  Artificial

<220>
<223>  hCTGF

<400>  31
ctctgacatt ctgattcgaa tga                                              23

<210>  32
<211>  23
<212>  DNA
<213>  Artificial

<220>
<223>  hCTGF

<400>  32
tggcaggctg atttctaggt agg                                              23

<210>  33
<211>  23
<212>  DNA
<213>  Artificial

<220>
<223>  hCTGF

<400>  33
cacagcacca gaatgtatat taa                                              23

<210>  34
<211>  23
<212>  DNA
<213>  Artificial

<220>
<223>  hCTGF

<400>  34
gttcaggaat cggaatcctg tcg                                              23

<210>  35
<211>  23
<212>  DNA
<213>  Artificial

<220>
<223>  hCTGF

<400>  35
catctttgaa tcgctgtact aca                                              23

<210>  36
<211>  23
<212>  DNA
<213>  Artificial

<220>
<223>  hCTGF

<400>  36
ctgatttcta ggtaggaaat gtg                                              23


<210>  37
<211>  23
<212>  DNA
<213>  Artificial

<220>
<223>  hCTGF

<400>  37
agccagagag tgagagacat taa                                              23


<210>  38
<211>  23
<212>  DNA
<213>  Artificial

<220>
<223>  hCTGF

<400>  38
gacagcttgt ggcaagtgaa ttt                                              23


<210>  39
<211>  23
<212>  DNA
<213>  Artificial

<220>
<223>  hCTGF

<400>  39
ttgaagaatg ttaagacttg aca                                              23


<210>  40
<211>  23
<212>  DNA
<213>  Artificial

<220>
<223>  hCTGF

<400>  40
gacagctagg atgtgcattc tcc                                              23


<210>  41
<211>  23
<212>  DNA

<213>  Artificial

<220>
<223>  hCTGF

<400>  41
gagtgtgacc aaaagttaca tgt                                              23


<210>  42
<211>  23
<212>  DNA
<213>  Artificial

<220>
<223>  hCTGF

<400>  42
gcctatcaag tttgagcttt ctg                                              23


<210>  43
<211>  23
<212>  DNA
<213>  Artificial

<220>
<223>  hCTGF

<400>  43
taccgactgg aagacacgtt tgg                                              23


<210>  44
<211>  23
<212>  DNA
<213>  Artificial

<220>
<223>  hCTGF

<400>  44
ccccagtgac agctaggatg tgc                                              23


<210>  45
<211>  23
<212>  DNA
<213>  Artificial

<220>
<223>  hCTGF

<400>  45
ctccagccat caagagactg agt                                              23


<210>  46
<211>  23
<212>  DNA
<213>  Artificial

<220>

<223>  hCTGF

<400>  46
aggctgattt ctaggtagga aat                                              23


<210>  47
<211>  23
<212>  DNA
<213>  Artificial

<220>
<223>  hCTGF

<400>  47
ccagagagtg agagacatta act                                              23


<210>  48
<211>  23
<212>  DNA
<213>  Artificial

<220>
<223>  hCTGF

<400>  48
cagtggaact acattagtac aca                                              23


<210>  49
<211>  23
<212>  DNA
<213>  Artificial

<220>
<223>  hCTGF

<400>  49
tctgaacacc ataggtagaa tgt                                              23


<210>  50
<211>  23
<212>  DNA
<213>  Artificial

<220>
<223>  hCTGF

<400>  50
agtgtccttg gcaggctgat ttc                                              23


<210>  51
<211>  23
<212>  DNA
<213>  Artificial

<220>
<223>  hCTGF

<400>  51

catgtttgca cctttctagt tga                                              23

<210>  52
<211>  23
<212>  DNA
<213>  Artificial

<220>
<223>  hCTGF

<400>  52
tgctcactga cctgcctgta gcc                                              23

<210>  53
<211>  23
<212>  DNA
<213>  Artificial

<220>
<223>  hCTGF

<400>  53
ctccctgcat cttcggtggt acg                                              23

<210>  54
<211>  23
<212>  DNA
<213>  Artificial

<220>
<223>  hCTGF

<400>  54
agcctcaatt tctgaacacc ata                                              23

<210>  55
<211>  23
<212>  DNA
<213>  Artificial

<220>
<223>  hCTGF

<400>  55
tgccattaca actgtcccgg aga                                              23

<210>  56
<211>  23
<212>  DNA
<213>  Artificial

<220>
<223>  hCTGF

<400>  56
acctgtgcct gccattacaa ctg                                              23

<210>  57
<211>  23
<212>  DNA
<213>  Artificial

<220>
<223>  hCTGF

<400>  57
agcagaaagg ttagtatcat cag                                              23


<210>  58
<211>  23
<212>  DNA
<213>  Artificial

<220>
<223>  hCTGF

<400>  58
gtgcagcatg gacgttcgtc tgc                                              23


<210>  59
<211>  23
<212>  DNA
<213>  Artificial

<220>
<223>  hCTGF

<400>  59
ctgcaccagc atgaagacat acc                                              23


<210>  60
<211>  23
<212>  DNA
<213>  Artificial

<220>
<223>  hCTGF

<400>  60
gacctgtgcc tgccattaca act                                              23


<210>  61
<211>  23
<212>  DNA
<213>  Artificial

<220>
<223>  hCTGF

<400>  61
aagagactga gtcaagttgt tcc                                              23


<210>  62
<211>  23
<212>  DNA

<213>  Artificial

<220>
<223>  hCTGF

<400>  62
gtgtccttgg caggctgatt tct                                              23


<210>  63
<211>  23
<212>  DNA
<213>  Artificial

<220>
<223>  hCTGF

<400>  63
ttgagagtgt gaccaaaagt tac                                              23


<210>  64
<211>  23
<212>  DNA
<213>  Artificial

<220>
<223>  hCTGF

<400>  64
gtgtgaccaa aagttacatg ttt                                              23


<210>  65
<211>  23
<212>  DNA
<213>  Artificial

<220>
<223>  hCTGF

<400>  65
ggcaaaaagt gcatccgtac tcc                                              23


<210>  66
<211>  23
<212>  DNA
<213>  Artificial

<220>
<223>  hCTGF

<400>  66
tcctgtcgat tagactggac agc                                              23


<210>  67
<211>  23
<212>  DNA
<213>  Artificial

<220>

<223>  hCTGF

<400>  67
aggaaatgtg gtagcctcac ttt                                              23


<210>  68
<211>  23
<212>  DNA
<213>  Artificial

<220>
<223>  hCTGF

<400>  68
ctgcatcttc ggtggtacgg tgt                                              23


<210>  69
<211>  23
<212>  DNA
<213>  Artificial

<220>
<223>  hCTGF

<400>  69
gcccaaggac caaaccgtgg ttg                                              23


<210>  70
<211>  23
<212>  DNA
<213>  Artificial

<220>
<223>  hCTGF

<400>  70
taactcatta gactggaact tga                                              23


<210>  71
<211>  23
<212>  DNA
<213>  Artificial

<220>
<223>  hCTGF

<400>  71
tgtgcattct ccagccatca aga                                              23


<210>  72
<211>  23
<212>  DNA
<213>  Artificial

<220>
<223>  hCTGF 72

<400>  72

tcgttcaaag catgaaatgg ata                                                23

<210>  73
<211>  23
<212>  DNA
<213>  Artificial

<220>
<223>  hCTGF 73

<400>  73
cagcatgaag acataccgag cta                                                23

<210>  74
<211>  23
<212>  DNA
<213>  Artificial

<220>
<223>  hCTGF

<400>  74
agattcccac ccaattcaaa aca                                                23

<210>  75
<211>  23
<212>  DNA
<213>  Artificial

<220>
<223>  hCTGF

<400>  75
cattgtgcca tgtcaaacaa ata                                                23

<210>  76
<211>  23
<212>  DNA
<213>  Artificial

<220>
<223>  hCTGF

<400>  76
cagtgtcctt ggcaggctga ttt                                                23

<210>  77
<211>  23
<212>  DNA
<213>  Artificial

<220>
<223>  hCTGF

<400>  77
ccctgcatct tcggtggtac ggt                                                23

<210>  78
<211>  23
<212>  DNA
<213>  Artificial

<220>
<223>  hCTGF

<400>  78
gagctaaatt ctgtggagta tgt                                              23


<210>  79
<211>  23
<212>  DNA
<213>  Artificial

<220>
<223>  hCTGF

<400>  79
aagattccca cccaattcaa aac                                              23


<210>  80
<211>  23
<212>  DNA
<213>  Artificial

<220>
<223>  hCTGF

<400>  80
tcccacccaa ttcaaaacat tgt                                              23


<210>  81
<211>  23
<212>  DNA
<213>  Artificial

<220>
<223>  hCTGF

<400>  81
cagcagaaag gttagtatca tca                                              23


<210>  82
<211>  23
<212>  DNA
<213>  Artificial

<220>
<223>  hCTGF

<400>  82
acgagtaata tgcctgctat ttg                                              23


<210>  83
<211>  23
<212>  DNA

<213>  Artificial

<220>
<223>  hCTGF

<400>  83
atcaagagac tgagtcaagt tgt                                              23


<210>  84
<211>  23
<212>  DNA
<213>  Artificial

<220>
<223>  hCTGF

<400>  84
atcggaatcc tgtcgattag act                                              23


<210>  85
<211>  23
<212>  DNA
<213>  Artificial

<220>
<223>  hCTGF

<400>  85
tggtagcctc acttttaatg aac                                              23


<210>  86
<211>  23
<212>  DNA
<213>  Artificial

<220>
<223>  hCTGF

<400>  86
gactgttttt cggacagttt att                                              23


<210>  87
<211>  23
<212>  DNA
<213>  Artificial

<220>
<223>  hCTGF

<400>  87
tacatgtttg cacctttcta gtt                                              23


<210>  88
<211>  23
<212>  DNA
<213>  Artificial

<220>

<223>  hCTGF

<400>  88
aaagttacat gtttgcacct ttc                                              23


<210>  89
<211>  23
<212>  DNA
<213>  Artificial

<220>
<223>  hCTGF

<400>  89
ttctactttg atatgactgt ttt                                              23


<210>  90
<211>  23
<212>  DNA
<213>  Artificial

<220>
<223>  hCTGF

<400>  90
cacccgggtt accaatgaca acg                                              23


<210>  91
<211>  23
<212>  DNA
<213>  Artificial

<220>
<223>  hCTGF

<400>  91
aagtgcatcc gtactcccaa aat                                              23


<210>  92
<211>  23
<212>  DNA
<213>  Artificial

<220>
<223>  hCTGF

<400>  92
aagcctatca agtttgagct ttc                                              23


<210>  93
<211>  23
<212>  DNA
<213>  Artificial

<220>
<223>  hCTGF

<400>  93

agcctatcaa gtttgagctt tct                                                23

<210>  94
<211>  23
<212>  DNA
<213>  Artificial

<220>
<223>  hCTGF

<400>  94
ctgtcccgga gacaatgaca tct                                                23

<210>  95
<211>  23
<212>  DNA
<213>  Artificial

<220>
<223>  hCTGF

<400>  95
gtggaactac attagtacac agc                                                23

<210>  96
<211>  23
<212>  DNA
<213>  Artificial

<220>
<223>  hCTGF

<400>  96
agccatcaag agactgagtc aag                                                23

<210>  97
<211>  23
<212>  DNA
<213>  Artificial

<220>
<223>  hCTGF

<400>  97
ttctgattcg aatgacactg ttc                                                23

<210>  98
<211>  23
<212>  DNA
<213>  Artificial

<220>
<223>  hCTGF

<400>  98
ctgttcagga atcggaatcc tgt                                                23

<210>  99
<211>  23
<212>  DNA
<213>  Artificial

<220>
<223>  hCTGF

<400>  99
tggacagctt gtggcaagtg aat                                              23


<210>  100
<211>  23
<212>  DNA
<213>  Artificial

<220>
<223>  hCTGF

<400>  100
tggcaagtga atttgcctgt aac                                              23


<210>  101
<211>  23
<212>  DNA
<213>  Artificial

<220>
<223>  hCyr61

<400>  101
gccagtgtac agcagcctga aaa                                              23


<210>  102
<211>  23
<212>  DNA
<213>  Artificial

<220>
<223>  hCyr61

<400>  102
ctcaacgagg actgcagcaa aac                                              23


<210>  103
<211>  23
<212>  DNA
<213>  Artificial

<220>
<223>  hCyr61

<400>  103
gggacactcc atgagtgtct gtg                                              23


<210>  104
<211>  23
<212>  DNA

<213>  Artificial

<220>
<223>  hCyr61

<400>  104
gggcagaccc tgtgaatata act                                              23


<210>  105
<211>  23
<212>  DNA
<213>  Artificial

<220>
<223>  hCyr61

<400>  105
gggaaagttt ccagcccaac tgt                                              23


<210>  106
<211>  23
<212>  DNA
<213>  Artificial

<220>
<223>  hCyr61

<400>  106
ttgttcaaac aacttcatgg tcc                                              23


<210>  107
<211>  23
<212>  DNA
<213>  Artificial

<220>
<223>  hCyr61

<400>  107
aagcagcgtt tcccttctac agg                                              23


<210>  108
<211>  23
<212>  DNA
<213>  Artificial

<220>
<223>  hCyr61

<400>  108
ttgtagaaag gaagccttgc tca                                              23


<210>  109
<211>  23
<212>  DNA
<213>  Artificial

<220>

<223>  hCyr61

<400>  109
ttggagcaca tgttactgct tca                                              23


<210>  110
<211>  23
<212>  DNA
<213>  Artificial

<220>
<223>  hCyr61

<400>  110
tggagcttgt ggagttgatg act                                              23


<210>  111
<211>  23
<212>  DNA
<213>  Artificial

<220>
<223>  hCyr61

<400>  111
tcccagtgct caaagacctg tgg                                              23


<210>  112
<211>  23
<212>  DNA
<213>  Artificial

<220>
<223>  hCyr61

<400>  112
aactggtatc tccacacgag tta                                              23


<210>  113
<211>  23
<212>  DNA
<213>  Artificial

<220>
<223>  hCyr61

<400>  113
tggacactaa tgcagccacg att                                              23


<210>  114
<211>  23
<212>  DNA
<213>  Artificial

<220>
<223>  hCyr61

<400>  114

gtgcggatgg acactaatgc agc                                              23


<210>  115
<211>  23
<212>  DNA
<213>  Artificial

<220>
<223>  hCyr61

<400>  115
tagtcgtcac ccttctccac ttg                                              23


<210>  116
<211>  23
<212>  DNA
<213>  Artificial

<220>
<223>  hCyr61

<400>  116
tggtcaaagt taccgggcag tgc                                              23


<210>  117
<211>  23
<212>  DNA
<213>  Artificial

<220>
<223>  hCyr61

<400>  117
agccttgctc attcttgagg agc                                              23


<210>  118
<211>  23
<212>  DNA
<213>  Artificial

<220>
<223>  hCyr61

<400>  118
ttcggtattt ttagaggtgc tcc                                              23


<210>  119
<211>  23
<212>  DNA
<213>  Artificial

<220>
<223>  hCyr61

<400>  119
cacgacattg tatgaagcac aat                                              23

<210>  120
<211>  23
<212>  DNA
<213>  Artificial

<220>
<223>  hCyr61

<400>  120
ctcattcttg aggagcatta agg                                              23


<210>  121
<211>  23
<212>  DNA
<213>  Artificial

<220>
<223>  hCyr61

<400>  121
gtgaaagaaa cccggatttg tga                                              23


<210>  122
<211>  23
<212>  DNA
<213>  Artificial

<220>
<223>  hCyr61

<400>  122
cccgaaccag tcaggtttac tta                                              23


<210>  123
<211>  23
<212>  DNA
<213>  Artificial

<220>
<223>  hCyr61

<400>  123
atgcagccac gattggagaa tac                                              23


<210>  124
<211>  23
<212>  DNA
<213>  Artificial

<220>
<223>  hCyr61

<400>  124
ttcgtccttt gacaaaagta aat                                              23


<210>  125
<211>  23
<212>  DNA

<213>  Artificial

<220>
<223>  hCyr61

<400>  125
gctcaacgag gactgcagca aaa                                              23


<210>  126
<211>  23
<212>  DNA
<213>  Artificial

<220>
<223>  hCyr61

<400>  126
aaaagtaaat gggagggcat tcc                                              23


<210>  127
<211>  23
<212>  DNA
<213>  Artificial

<220>
<223>  hCyr61

<400>  127
gtgtatgcca ttcggtattt tta                                              23


<210>  128
<211>  23
<212>  DNA
<213>  Artificial

<220>
<223>  hCyr61

<400>  128
ctgagtgtat gccattcggt att                                              23


<210>  129
<211>  23
<212>  DNA
<213>  Artificial

<220>
<223>  hCyr61

<400>  129
accctcggct ggtcaaagtt acc                                              23


<210>  130
<211>  23
<212>  DNA
<213>  Artificial

<220>

<223>  hCyr61

<400>  130
cccccgaacc agtcaggttt act                                              23


<210>  131
<211>  23
<212>  DNA
<213>  Artificial

<220>
<223>  hCyr61

<400>  131
ttccaagaac gtcatgatga tcc                                              23


<210>  132
<211>  23
<212>  DNA
<213>  Artificial

<220>
<223>  hCyr61

<400>  132
gtgatgggac tcattgtaga aag                                              23


<210>  133
<211>  23
<212>  DNA
<213>  Artificial

<220>
<223>  hCyr61

<400>  133
gccacgattg gagaatactt tgc                                              23


<210>  134
<211>  23
<212>  DNA
<213>  Artificial

<220>
<223>  hCyr61

<400>  134
aagcttttat tcgtcctttg aca                                              23


<210>  135
<211>  23
<212>  DNA
<213>  Artificial

<220>
<223>  hCyr61

<400>  135

ggacactaat gcagccacga ttg                                              23

<210>  136
<211>  23
<212>  DNA
<213>  Artificial

<220>
<223>  hCyr61

<400>  136
ttcatggtcc cagtgctcaa aga                                              23

<210>  137
<211>  23
<212>  DNA
<213>  Artificial

<220>
<223>  hCyr61

<400>  137
cgccttagtc gtcacccttc tcc                                              23

<210>  138
<211>  23
<212>  DNA
<213>  Artificial

<220>
<223>  hCyr61

<400>  138
ggggacactc catgagtgtc tgt                                              23

<210>  139
<211>  23
<212>  DNA
<213>  Artificial

<220>
<223>  hCyr61

<400>  139
cttgctcatt cttgaggagc att                                              23

<210>  140
<211>  23
<212>  DNA
<213>  Artificial

<220>
<223>  hCyr61

<400>  140
ggccagaaat gtattgttca aac                                              23

<210>  141
<211>  23
<212>  DNA
<213>  Artificial

<220>
<223>  hCyr61

<400>  141
ccagtgtaca gcagcctgaa aaa                                              23


<210>  142
<211>  23
<212>  DNA
<213>  Artificial

<220>
<223>  hCyr61

<400>  142
cccaaccctc ggctggtcaa agt                                              23


<210>  143
<211>  23
<212>  DNA
<213>  Artificial

<220>
<223>  hCyr61

<400>  143
cggatttgtg aggtgcggcc ttg                                              23


<210>  144
<211>  23
<212>  DNA
<213>  Artificial

<220>
<223>  hCyr61

<400>  144
gccttgtgaa agaaacccgg att                                              23


<210>  145
<211>  23
<212>  DNA
<213>  Artificial

<220>
<223>  hCyr61

<400>  145
ccctgagtgc cgccttgtga aag                                              23


<210>  146
<211>  23
<212>  DNA

<213>  Artificial

<220>
<223>  hCyr61

<400>  146
gggtctgtga cgaggatagt atc                                              23


<210>  147
<211>  23
<212>  DNA
<213>  Artificial

<220>
<223>  hCyr61

<400>  147
agcacatgtt actgcttcat ttt                                              23


<210>  148
<211>  23
<212>  DNA
<213>  Artificial

<220>
<223>  hCyr61

<400>  148
accctgtgaa tataactcca gaa                                              23


<210>  149
<211>  23
<212>  DNA
<213>  Artificial

<220>
<223>  hCyr61

<400>  149
gactcattgt agaaaggaag cct                                              23


<210>  150
<211>  23
<212>  DNA
<213>  Artificial

<220>
<223>  hCyr61

<400>  150
caggacttat tgggatacag cag                                              23


<210>  151
<211>  23
<212>  DNA
<213>  Artificial

<220>

<223>  hCyr61

<400>  151
cgagttacca atgacaaccc tga                                              23


<210>  152
<211>  23
<212>  DNA
<213>  Artificial

<220>
<223>  hCyr61

<400>  152
ggtttactta cgctggatgt ttg                                              23


<210>  153
<211>  23
<212>  DNA
<213>  Artificial

<220>
<223>  hCyr61

<400>  153
aagattagtt ggacagttta aag                                              23


<210>  154
<211>  23
<212>  DNA
<213>  Artificial

<220>
<223>  hCyr61

<400>  154
cgaaccagtc aggtttactt acg                                              23


<210>  155
<211>  23
<212>  DNA
<213>  Artificial

<220>
<223>  hCyr61

<400>  155
tgatgggact cattgtagaa agg                                              23


<210>  156
<211>  23
<212>  DNA
<213>  Artificial

<220>
<223>  hCyr61

<400>  156

gatccagtcc tgcaaatgca act                                              23


<210>  157
<211>  23
<212>  DNA
<213>  Artificial

<220>
<223>  hCyr61

<400>  157
ggagcacatg ttactgcttc att                                              23


<210>  158
<211>  23
<212>  DNA
<213>  Artificial

<220>
<223>  hCyr61

<400>  158
ttgtatgaag cacaataaag att                                              23


<210>  159
<211>  23
<212>  DNA
<213>  Artificial

<220>
<223>  hCyr61

<400>  159
cacacctaga caaacaaggg aga                                              23


<210>  160
<211>  23
<212>  DNA
<213>  Artificial

<220>
<223>  hCyr61

<400>  160
tgtatgccat tcggtatttt tag                                              23


<210>  161
<211>  23
<212>  DNA
<213>  Artificial

<220>
<223>  hCyr61

<400>  161
aaccctttac aaggccagaa atg                                              23

<210>  162
<211>  23
<212>  DNA
<213>  Artificial

<220>
<223>  hCyr61

<400>  162
   accctttaca aggccagaaa tgt                                                23


<210>  163
<211>  23
<212>  DNA
<213>  Artificial

<220>
<223>  hCyr61

<400>  163
ctggtatctc cacacgagtt acc                                               23


<210>  164
<211>  23
<212>  DNA
<213>  Artificial

<220>
<223>  hCyr61

<400>  164
ctccacacga gttaccaatg aca                                               23


<210>  165
<211>  23
<212>  DNA
<213>  Artificial

<220>
<223>  hCyr61

<400>  165
ttgtgaaaga aacccggatt tgt                                               23


<210>  166
<211>  23
<212>  DNA
<213>  Artificial

<220>
<223>  hCyr61

<400>  166
tccagggcac acctagacaa aca                                               23


<210>  167
<211>  23
<212>  DNA

<213>  Artificial

<220>
<223>  hCyr61

<400>  167
tgggtgatgg gactcattgt aga                                              23


<210>  168
<211>  23
<212>  DNA
<213>  Artificial

<220>
<223>  hCyr61

<400>  168
tgcagccacg attggagaat act                                              23


<210>  169
<211>  23
<212>  DNA
<213>  Artificial

<220>
<223>  hCyr61

<400>  169
cagccacgat tggagaatac ttt                                              23


<210>  170
<211>  23
<212>  DNA
<213>  Artificial

<220>
<223>  hCyr61

<400>  170
gagtgtatgc cattcggtat ttt                                              23


<210>  171
<211>  23
<212>  DNA
<213>  Artificial

<220>
<223>  hCyr61

<400>  171
atgccattcg gtatttttag agg                                              23


<210>  172
<211>  23
<212>  DNA
<213>  Artificial

<220>

<223>  hCyr61

<400>  172
gggcacacct agacaaacaa ggg                                              23


<210>  173
<211>  23
<212>  DNA
<213>  Artificial

<220>
<223>  hCyr61

<400>  173
ggtgatggga ctcattgtag aaa                                              23


<210>  174
<211>  23
<212>  DNA
<213>  Artificial

<220>
<223>  hCyr61

<400>  174
gaacgtcatg atgatccagt cct                                              23


<210>  175
<211>  23
<212>  DNA
<213>  Artificial

<220>
<223>  hCyr61

<400>  175
ttggacagtt taaagctttt att                                              23


<210>  176
<211>  23
<212>  DNA
<213>  Artificial

<220>
<223>  hCyr61

<400>  176
agcccaactg taaacatcag tgc                                              23


<210>  177
<211>  23
<212>  DNA
<213>  Artificial

<220>
<223>  hCyr61

<400>  177

cacacgagtt accaatgaca acc                                              23

<210>  178
<211>  23
<212>  DNA
<213>  Artificial

<220>
<223>  hCyr61

<400>  178
atgaagcagc gtttcccttc tac                                              23

<210>  179
<211>  23
<212>  DNA
<213>  Artificial

<220>
<223>  hCyr61

<400>  179
gacactaatg cagccacgat tgg                                              23

<210>  180
<211>  23
<212>  DNA
<213>  Artificial

<220>
<223>  hCyr61

<400>  180
aacatgtatt gaacacgaca ttg                                              23

<210>  181
<211>  23
<212>  DNA
<213>  Artificial

<220>
<223>  hCyr61

<400>  181
acgacattgt atgaagcaca ata                                              23

<210>  182
<211>  23
<212>  DNA
<213>  Artificial

<220>
<223>  hCyr61

<400>  182
gacattgtat gaagcacaat aaa                                              23

<210>  183
<211>  23
<212>  DNA
<213>  Artificial

<220>
<223>  hCyr61

<400>  183
agttggacag tttaaagctt tta                                              23


<210>  184
<211>  23
<212>  DNA
<213>  Artificial

<220>
<223>  hCyr61

<400>  184
gaccctgtga atataactcc aga                                              23


<210>  185
<211>  23
<212>  DNA
<213>  Artificial

<220>
<223>  hCyr61

<400>  185
cacctagaca aacaagggag aag                                              23


<210>  186
<211>  23
<212>  DNA
<213>  Artificial

<220>
<223>  hCyr61

<400>  186
ctcattgtag aaaggaagcc ttg                                              23


<210>  187
<211>  23
<212>  DNA
<213>  Artificial

<220>
<223>  hCyr61

<400>  187
aagcatattt tctctaggct ttt                                              23


<210>  188
<211>  23
<212>  DNA

<213>  Artificial

<220>
<223>  hCyr61

<400>  188
gggcattcca tcccttcctg aag                                              23


<210>  189
<211>  23
<212>  DNA
<213>  Artificial

<220>
<223>  hCyr61

<400>  189
ccctttacaa ggccagaaat gta                                              23


<210>  190
<211>  23
<212>  DNA
<213>  Artificial

<220>
<223>  hCyr61

<400>  190
ttgagtgtga agaaataccg gcc                                              23


<210>  191
<211>  23
<212>  DNA
<213>  Artificial

<220>
<223>  hCyr61

<400>  191
gtggacagcc agtgtacagc agc                                              23


<210>  192
<211>  23
<212>  DNA
<213>  Artificial

<220>
<223>  hCyr61

<400>  192
ttacgctgga tgtttgagtg tga                                              23


<210>  193
<211>  23
<212>  DNA
<213>  Artificial

<220>

<223>  hCyr61

<400>  193
tttccagccc aactgtaaac atc                                              23


<210>  194
<211>  23
<212>  DNA
<213>  Artificial

<220>
<223>  hCyr61

<400>  194
aaacatcagt gcacatgtat tga                                              23


<210>  195
<211>  23
<212>  DNA
<213>  Artificial

<220>
<223>  hCyr61

<400>  195
ggtctgtgac gaggatagta tca                                              23


<210>  196
<211>  23
<212>  DNA
<213>  Artificial

<220>
<223>  hCyr61

<400>  196
agcattaagg tatttcgaaa ctg                                              23


<210>  197
<211>  23
<212>  DNA
<213>  Artificial

<220>
<223>  hCyr61

<400>  197
tgggtttcca gggcacacct aga                                              23


<210>  198
<211>  23
<212>  DNA
<213>  Artificial

<220>
<223>  hCyr61

<400>  198

ttgctaagca tattttctct agg                                                23

<210>  199
<211>  23
<212>  DNA
<213>  Artificial

<220>
<223>  hCyr61

<400>  199
cgaggagtgg gtctgtgacg agg                                                23

<210>  200
<211>  23
<212>  DNA
<213>  Artificial

<220>
<223>  hCyr61

<400>  200
cacatgtatt gatggcgccg tgg                                                23

<210>  201
<211>  23
<212>  DNA
<213>  Artificial

<220>
<223>  hPlekho1

<400>  201
ctgaccttgg acttgatcca aga                                                23

<210>  202
<211>  23
<212>  DNA
<213>  Artificial

<220>
<223>  hPlekho1

<400>  202
ttgagtgaaa ccggagctac aaa                                                23

<210>  203
<211>  23
<212>  DNA
<213>  Artificial

<220>
<223>  hPlekho1

<400>  203
agccaagaac cgtatcttgg atg                                                23

<210>  204
<211>  23
<212>  DNA
<213>  Artificial

<220>
<223>  hPlekho1

<400>  204
caagaaccgt atcttggatg agg                                               23


<210>  205
<211>  23
<212>  DNA
<213>  Artificial

<220>
<223>  hPlekho1

<400>  205
cccctgagga accaacctct tgt                                               23


<210>  206
<211>  23
<212>  DNA
<213>  Artificial

<220>
<223>  hPlekho1

<400>  206
aagttgacgc ccacagagaa agg                                               23


<210>  207
<211>  23
<212>  DNA
<213>  Artificial

<220>
<223>  hPlekho1

<400>  207
gagatttgga aaaaccgcta tgt                                               23


<210>  208
<211>  23
<212>  DNA
<213>  Artificial

<220>
<223>  hPlekho1

<400>  208
tgctgagagc tttcgggttg acc                                               23


<210>  209
<211>  23
<212>  DNA

<213>  Artificial

<220>
<223>  hPlekho1

<400>  209
gggtctggaa cttgtcgggt tgg                                              23


<210>  210
<211>  23
<212>  DNA
<213>  Artificial

<220>
<223>  hPlekho1

<400>  210
tgggagaggc atcatcgaat tgg                                              23


<210>  211
<211>  23
<212>  DNA
<213>  Artificial

<220>
<223>  hPlekho1

<400>  211
atcgtggatc aatgccctca act                                              23


<210>  212
<211>  23
<212>  DNA
<213>  Artificial

<220>
<223>  hPlekho1

<400>  212
acctcttgtg ctgagagctt tcg                                              23


<210>  213
<211>  23
<212>  DNA
<213>  Artificial

<220>
<223>  hPlekho1

<400>  213
cacggcaccc aacctgatct tcc                                              23


<210>  214
<211>  23
<212>  DNA
<213>  Artificial

<220>

<223>  hPlekho1

<400>  214
gaggacagct atcttgccca tcc                                               23


<210>  215
<211>  23
<212>  DNA
<213>  Artificial

<220>
<223>  hPlekho1

<400>  215
gacacctaat ggctgtggct tcc                                               23


<210>  216
<211>  23
<212>  DNA
<213>  Artificial

<220>
<223>  hPlekho1

<400>  216
gtcgggttgg acagactctt atc                                               23


<210>  217
<211>  23
<212>  DNA
<213>  Artificial

<220>
<223>  hPlekho1

<400>  217
tcagtaccgg aagagcctga tgt                                               23


<210>  218
<211>  23
<212>  DNA
<213>  Artificial

<220>
<223>  hPlekho1

<400>  218
ttatctccgt gttgctggat aaa                                               23


<210>  219
<211>  23
<212>  DNA
<213>  Artificial

<220>
<223>  hPlekho1

<400>  219

tccgccggat tctgagtcag agc                                                23

<210>  220
<211>  23
<212>  DNA
<213>  Artificial

<220>
<223>  hPlekho1

<400>  220
ctcgagacag ggcaaaaatc cag                                                23

<210>  221
<211>  23
<212>  DNA
<213>  Artificial

<220>
<223>  hPlekho1

<400>  221
aaccaacctc ttgtgctgag agc                                                23

<210>  222
<211>  23
<212>  DNA
<213>  Artificial

<220>
<223>  hPlekho1

<400>  222
caggacctgg tagcaaggaa act                                                23

<210>  223
<211>  23
<212>  DNA
<213>  Artificial

<220>
<223>  hPlekho1

<400>  223
ctgggagagg catcatcgaa ttg                                                23

<210>  224
<211>  23
<212>  DNA
<213>  Artificial

<220>
<223>  hPlekho1

<400>  224
gtccagaaga gaaggaatcg tgg                                                23

<210>  225
<211>  23
<212>  DNA
<213>  Artificial

<220>
<223>  hPlekho1

<400>  225
gccttgagtg aaaccggagc tac                                              23


<210>  226
<211>  23
<212>  DNA
<213>  Artificial

<220>
<223>  hPlekho1

<400>  226
ttggaaaaac cgctatgtgg tgc                                              23


<210>  227
<211>  23
<212>  DNA
<213>  Artificial

<220>
<223>  hPlekho1

<400>  227
gaagttgacg cccacagaga aag                                              23


<210>  228
<211>  23
<212>  DNA
<213>  Artificial

<220>
<223>  hPlekho1

<400>  228
gctgggagag gcatcatcga att                                              23


<210>  229
<211>  23
<212>  DNA
<213>  Artificial

<220>
<223>  hPlekho1

<400>  229
gaacttgtcg ggttggacag act                                              23


<210>  230
<211>  23
<212>  DNA

<213>  Artificial

<220>
<223>  hPlekho1

<400>  230
acagactctt atctccgtgt tgc                                              23


<210>  231
<211>  23
<212>  DNA
<213>  Artificial

<220>
<223>  hPlekho1

<400>  231
aggtcaccgt tgaggaggac agc                                              23


<210>  232
<211>  23
<212>  DNA
<213>  Artificial

<220>
<223>  hPlekho1

<400>  232
tccctggagg agatcctatc tca                                              23


<210>  233
<211>  23
<212>  DNA
<213>  Artificial

<220>
<223>  hPlekho1

<400>  233
gggagctgag agacctgtac aga                                              23


<210>  234
<211>  23
<212>  DNA
<213>  Artificial

<220>
<223>  hPlekho1

<400>  234
ctgagagacc tgtacagaca gat                                              23


<210>  235
<211>  23
<212>  DNA
<213>  Artificial

<220>

<223>  hPlekho1

<400>  235
acagggcaaa aatccagcac tcc                                               23


<210>  236
<211>  23
<212>  DNA
<213>  Artificial

<220>
<223>  hPlekho1

<400>  236
gagctttcgg gttgacctgg aca                                               23


<210>  237
<211>  23
<212>  DNA
<213>  Artificial

<220>
<223>  hPlekho1

<400>  237
cactcgagac agggcaaaaa tcc                                               23


<210>  238
<211>  23
<212>  DNA
<213>  Artificial

<220>
<223>  hPlekho1

<400>  238
gtccggaaat tctgcgggaa agg                                               23


<210>  239
<211>  23
<212>  DNA
<213>  Artificial

<220>
<223>  hPlekho1

<400>  239
tgggatgctg accttggact tga                                               23


<210>  240
<211>  23
<212>  DNA
<213>  Artificial

<220>
<223>  hPlekho1

<400>  240

gaccttggac ttgatccaag agg                                              23

<210>  241
<211>  23
<212>  DNA
<213>  Artificial

<220>
<223>  hPlekho1

<400>  241
aacctcttgt gctgagagct ttc                                              23

<210>  242
<211>  23
<212>  DNA
<213>  Artificial

<220>
<223>  hPlekho1

<400>  242
gaagaagaac aattccgcca agc                                              23

<210>  243
<211>  23
<212>  DNA
<213>  Artificial

<220>
<223>  hPlekho1

<400>  243
ttcaagaggt atttgacctg agt                                              23

<210>  244
<211>  23
<212>  DNA
<213>  Artificial

<220>
<223>  hPlekho1

<400>  244
aatcgtggat caatgccctc aac                                              23

<210>  245
<211>  23
<212>  DNA
<213>  Artificial

<220>
<223>  hPlekho1

<400>  245
cttggacttg atccaagagg aag                                              23

<210>  246
<211>  23
<212>  DNA
<213>  Artificial

<220>
<223>  hPlekho1

<400>  246
gacagactct tatctccgtg ttg                                              23


<210>  247
<211>  23
<212>  DNA
<213>  Artificial

<220>
<223>  hPlekho1

<400>  247
agagaaggaa tcgtggatca atg                                              23


<210>  248
<211>  23
<212>  DNA
<213>  Artificial

<220>
<223>  hPlekho1

<400>  248
ggggtctgga acttgtcggg ttg                                              23


<210>  249
<211>  23
<212>  DNA
<213>  Artificial

<220>
<223>  hPlekho1

<400>  249
cacccgagcc aagaaccgta tct                                              23


<210>  250
<211>  23
<212>  DNA
<213>  Artificial

<220>
<223>  hPlekho1

<400>  250
caccgttgag gaggacagct atc                                              23


<210>  251
<211>  23
<212>  DNA

<213>  Artificial

<220>
<223>  hPlekho1

<400>  251
gagaaggaat cgtggatcaa tgc                                              23


<210>  252
<211>  23
<212>  DNA
<213>  Artificial

<220>
<223>  hPlekho1

<400>  252
cgtgtgtgtg cgagtgcgaa tgc                                              23


<210>  253
<211>  23
<212>  DNA
<213>  Artificial

<220>
<223>  hPlekho1

<400>  253
cctggcagtg agtccagaag aga                                              23


<210>  254
<211>  23
<212>  DNA
<213>  Artificial

<220>
<223>  hPlekho1

<400>  254
cctgaggaac caacctcttg tgc                                              23


<210>  255
<211>  23
<212>  DNA
<213>  Artificial

<220>
<223>  hPlekho1

<400>  255
gctgagagac ctgtacagac aga                                              23


<210>  256
<211>  23
<212>  DNA
<213>  Artificial

<220>

<223>  hPlekho1

<400>  256
acctctacct cggatgggat gct                                              23


<210>  257
<211>  23
<212>  DNA
<213>  Artificial

<220>
<223>  hPlekho1

<400>  257
tggatcaatg ccctcaactc tgc                                              23


<210>  258
<211>  23
<212>  DNA
<213>  Artificial

<220>
<223>  hPlekho1

<400>  258
tcgagacagg gcaaaaatcc agc                                              23


<210>  259
<211>  23
<212>  DNA
<213>  Artificial

<220>
<223>  hPlekho1

<400>  259
tgagtgaaac cggagctaca aag                                              23


<210>  260
<211>  23
<212>  DNA
<213>  Artificial

<220>
<223>  hPlekho1

<400>  260
ctcaactctg ccatcacccg agc                                              23


<210>  261
<211>  23
<212>  DNA
<213>  Artificial

<220>
<223>  hPlekho1

<400>  261

aatcatagca agtttactct tgc                                              23

<210>  262
<211>  23
<212>  DNA
<213>  Artificial

<220>
<223>  hPlekho1

<400>  262
ttggacagac tcttatctcc gtg                                              23

<210>  263
<211>  23
<212>  DNA
<213>  Artificial

<220>
<223>  hPlekho1

<400>  263
tggacagact cttatctccg tgt                                              23

<210>  264
<211>  23
<212>  DNA
<213>  Artificial

<220>
<223>  hPlekho1

<400>  264
ccttgagtga aaccggagct aca                                              23

<210>  265
<211>  23
<212>  DNA
<213>  Artificial

<220>
<223>  hPlekho1

<400>  265
gattttcagg gagatttgga aaa                                              23

<210>  266
<211>  23
<212>  DNA
<213>  Artificial

<220>
<223>  hPlekho1

<400>  266
aaaccgctat gtggtgctga aag                                              23

<210>  267
<211>  23
<212>  DNA
<213>  Artificial

<220>
<223>  hPlekho1

<400>  267
agtaccggaa gagcctgatg tga                                              23


<210>  268
<211>  23
<212>  DNA
<213>  Artificial

<220>
<223>  hPlekho1

<400>  268
cttatctccg tgttgctgga taa                                              23


<210>  269
<211>  23
<212>  DNA
<213>  Artificial

<220>
<223>  hPlekho1

<400>  269
tcacccgagc caagaaccgt atc                                              23


<210>  270
<211>  23
<212>  DNA
<213>  Artificial

<220>
<223>  hPlekho1

<400>  270
ctacctcgga tgggatgctg acc                                              23


<210>  271
<211>  23
<212>  DNA
<213>  Artificial

<220>
<223>  hPlekho1

<400>  271
cccactcgag acagggcaaa aat                                              23


<210>  272
<211>  23
<212>  DNA

<213>  Artificial

<220>
<223>  hPlekho1

<400>  272
tcgggttgga cagactctta tct                                              23


<210>  273
<211>  23
<212>  DNA
<213>  Artificial

<220>
<223>  hPlekho1

<400>  273
agacctgtac agacagatgg acc                                              23


<210>  274
<211>  23
<212>  DNA
<213>  Artificial

<220>
<223>  hPlekho1

<400>  274
cttgagtgaa accggagcta caa                                              23


<210>  275
<211>  23
<212>  DNA
<213>  Artificial

<220>
<223>  hPlekho1

<400>  275
cggaaattct gcgggaaagg gat                                              23


<210>  276
<211>  23
<212>  DNA
<213>  Artificial

<220>
<223>  hPlekho1

<400>  276
caacctcttg tgctgagagc ttt                                              23


<210>  277
<211>  23
<212>  DNA
<213>  Artificial

<220>

<223>  hPlekho1

<400>  277
ctctcccgac cttgggaaaa aac                                              23


<210>  278
<211>  23
<212>  DNA
<213>  Artificial

<220>
<223>  hPlekho1

<400>  278
atctccgtgt tgctggataa agc                                              23


<210>  279
<211>  23
<212>  DNA
<213>  Artificial

<220>
<223>  hPlekho1

<400>  279
tggaacttgt cgggttggac aga                                              23


<210>  280
<211>  23
<212>  DNA
<213>  Artificial

<220>
<223>  hPlekho1

<400>  280
ggaatcgtgg atcaatgccc tca                                              23


<210>  281
<211>  23
<212>  DNA
<213>  Artificial

<220>
<223>  hPlekho1

<400>  281
ggaggacagc tatcttgccc atc                                              23


<210>  282
<211>  23
<212>  DNA
<213>  Artificial

<220>
<223>  hPlekho1

<400>  282

tcggctgggt ccggaaattc tgc                                              23

<210>  283
<211>  23
<212>  DNA
<213>  Artificial

<220>
<223>  hPlekho1

<400>  283
ggacctggta gcaaggaaac tgg                                              23

<210>  284
<211>  23
<212>  DNA
<213>  Artificial

<220>
<223>  hPlekho1

<400>  284
tcccactcga gacagggcaa aaa                                              23

<210>  285
<211>  23
<212>  DNA
<213>  Artificial

<220>
<223>  hPlekho1

<400>  285
ctctacctcg gatgggatgc tga                                              23

<210>  286
<211>  23
<212>  DNA
<213>  Artificial

<220>
<223>  hPlekho1

<400>  286
cggatccagg acctggtagc aag                                              23

<210>  287
<211>  23
<212>  DNA
<213>  Artificial

<220>
<223>  hPlekho1

<400>  287
cagggagctg agagacctgt aca                                              23

<210>  288
<211>  23
<212>  DNA
<213>  Artificial

<220>
<223>  hPlekho1

<400>  288
tgaggtcacc gttgaggagg aca                                              23


<210>  289
<211>  23
<212>  DNA
<213>  Artificial

<220>
<223>  hPlekho1

<400>  289
tcaccgttga ggaggacagc tat                                              23


<210>  290
<211>  23
<212>  DNA
<213>  Artificial

<220>
<223>  hPlekho1

<400>  290
ccaggcaaag agggtgctgc agg                                              23


<210>  291
<211>  23
<212>  DNA
<213>  Artificial

<220>
<223>  hPlekho1

<400>  291
gccaagaacc gtatcttgga tga                                              23


<210>  292
<211>  23
<212>  DNA
<213>  Artificial

<220>
<223>  hPlekho1

<400>  292
gagagacctg tacagacaga tgg                                              23


<210>  293
<211>  23
<212>  DNA

<213>  Artificial

<220>
<223>  hPlekho1

<400>  293
ggaccagctc tacatctctg aga                                              23


<210>  294
<211>  23
<212>  DNA
<213>  Artificial

<220>
<223>  hPlekho1

<400>  294
cctggaggag atcctatctc agc                                              23


<210>  295
<211>  23
<212>  DNA
<213>  Artificial

<220>
<223>  hPlekho1

<400>  295
ccaggacctg gtagcaagga aac                                              23


<210>  296
<211>  23
<212>  DNA
<213>  Artificial

<220>
<223>  hPlekho1

<400>  296
ccactcgaga cagggcaaaa atc                                              23


<210>  297
<211>  23
<212>  DNA
<213>  Artificial

<220>
<223>  hPlekho1

<400>  297
cctgtacaga cagatggacc tgc                                              23


<210>  298
<211>  23
<212>  DNA
<213>  Artificial

<220>

<223>  hPlekho1

<400>  298
cacctctacc tcggatggga tgc                                              23


<210>  299
<211>  23
<212>  DNA
<213>  Artificial

<220>
<223>  hPlekho1

<400>  299
tcccctgagg aaccaacctc ttg                                              23


<210>  300
<211>  23
<212>  DNA
<213>  Artificial

<220>
<223>  hPlekho1

<400>  300
gtcagtaccg gaagagcctg atg                                              23


<210>  301
<211>  19
<212>  DNA
<213>  Artificial

<220>
<223>  mCTGF

<400>  301
acacgaactc attagacta                                                   19


<210>  302
<211>  19
<212>  DNA
<213>  Artificial

<220>
<223>  mCTGF

<400>  302
gaagtaaggg acacgaact                                                   19


<210>  303
<211>  19
<212>  DNA
<213>  Artificial

<220>
<223>  mCTGF

<400>  303

gagcatgtgt cctccacta                                                    19


<210>  304
<211>  19
<212>  DNA
<213>  Artificial

<220>
<223>  mCTGF

<400>  304
gtacggagac atggcgtaa                                                    19


<210>  305
<211>  19
<212>  DNA
<213>  Artificial

<220>
<223>  mCTGF

<400>  305
agtcagattt ctagtagga                                                    19


<210>  306
<211>  19
<212>  DNA
<213>  Artificial

<220>
<223>  mCTGF

<400>  306
gacactggtt tcgagacag                                                    19


<210>  307
<211>  19
<212>  DNA
<213>  Artificial

<220>
<223>  mCTGF

<400>  307
gtgaacaaat ggcctttat                                                    19


<210>  308
<211>  19
<212>  DNA
<213>  Artificial

<220>
<223>  mCTGF

<400>  308
cacgaactca ttagactat                                                    19

<210>  309
<211>  19
<212>  DNA
<213>  Artificial

<220>
<223>  mCTGF

<400>  309
tcaacctcag acactggtt                                                    19


<210>  310
<211>  19
<212>  DNA
<213>  Artificial

<220>
<223>  mCTGF

<400>  310
cctgctgtgc atcctccta                                                    19


<210>  311
<211>  19
<212>  DNA
<213>  Artificial

<220>
<223>  mCTGF

<400>  311
ggccatacaa gtagtctgt                                                    19


<210>  312
<211>  19
<212>  DNA
<213>  Artificial

<220>
<223>  mCTGF

<400>  312
gcctactttt tggagtgta                                                    19


<210>  313
<211>  19
<212>  DNA
<213>  Artificial

<220>
<223>  mCTGF

<400>  313
ggtgagtcct tccaaagca                                                    19


<210>  314
<211>  19
<212>  DNA

<213>  Artificial

<220>
<223>  mCTGF

<400>  314
cagtttacac ttgacagtt                                                    19


<210>  315
<211>  19
<212>  DNA
<213>  Artificial

<220>
<223>  mCTGF

<400>  315
aaactccaaa caccatagg                                                    19


<210>  316
<211>  19
<212>  DNA
<213>  Artificial

<220>
<223>  mCTGF

<400>  316
cttgtctgtt agactggac                                                    19


<210>  317
<211>  19
<212>  DNA
<213>  Artificial

<220>
<223>  mCTGF

<400>  317
acacttgaca gttgttcat                                                    19


<210>  318
<211>  19
<212>  DNA
<213>  Artificial

<220>
<223>  mCTGF

<400>  318
agacactggt ttcgagaca                                                    19


<210>  319
<211>  19
<212>  DNA
<213>  Artificial

<220>

<223>  mCTGF

<400>  319
agctgcaaat accaatgca                                                    19


<210>  320
<211>  19
<212>  DNA
<213>  Artificial

<220>
<223>  mCTGF

<400>  320
tcagacactg gtttcgaga                                                    19


<210>  321
<211>  19
<212>  DNA
<213>  Artificial

<220>
<223>  mCTGF

<400>  321
caccaaagtg agaacgtta                                                    19


<210>  322
<211>  19
<212>  DNA
<213>  Artificial

<220>
<223>  mCTGF

<400>  322
gcatccggac acctaaaat                                                    19


<210>  323
<211>  19
<212>  DNA
<213>  Artificial

<220>
<223>  mCTGF

<400>  323
gtactagctg aggttattt                                                    19


<210>  324
<211>  19
<212>  DNA
<213>  Artificial

<220>
<223>  mCTGF

<400>  324

gacacgaact cattagact                                                    19


<210>  325
<211>  19
<212>  DNA
<213>  Artificial

<220>
<223>  mCTGF

<400>  325
ctgcaaatac caatgcact                                                    19


<210>  326
<211>  19
<212>  DNA
<213>  Artificial

<220>
<223>  mCTGF

<400>  326
ggacacctaa aatcgccaa                                                    19


<210>  327
<211>  19
<212>  DNA
<213>  Artificial

<220>
<223>  mCTGF

<400>  327
gtgaagacat acagggcta                                                    19


<210>  328
<211>  19
<212>  DNA
<213>  Artificial

<220>
<223>  mCTGF

<400>  328
ccaaacacca taggtagga                                                    19


<210>  329
<211>  19
<212>  DNA
<213>  Artificial

<220>
<223>  mCTGF

<400>  329
ctgtaacaag ccagatttt                                                    19

<210>  330
<211>  19
<212>  DNA
<213>  Artificial

<220>
<223>  mCTGF

<400>  330
ggtgaacaaa tggccttta                                                    19


<210>  331
<211>  19
<212>  DNA
<213>  Artificial

<220>
<223>  mCTGF

<400>  331
ggtgagtcct tccaaagca                                                    19


<210>  332
<211>  19
<212>  DNA
<213>  Artificial

<220>
<223>  mCTGF

<400>  332
gcatccggac acctaaaat                                                    19


<210>  333
<211>  19
<212>  DNA
<213>  Artificial

<220>
<223>  mCTGF

<400>  333
ctgcaaatac caatgcact                                                    19


<210>  334
<211>  19
<212>  DNA
<213>  Artificial

<220>
<223>  mCTGF

<400>  334
caaaaagtgc atccggaca                                                    19


<210>  335
<211>  19
<212>  DNA

<213>  Artificial

<220>
<223>  mCTGF

<400>  335
ggacacctaa aatcgccaa                                                    19


<210>  336
<211>  19
<212>  DNA
<213>  Artificial

<220>
<223>  mCTGF

<400>  336
gtgaagacat acagggcta                                                    19


<210>  337
<211>  19
<212>  DNA
<213>  Artificial

<220>
<223>  mCTGF

<400>  337
ccttcccgag aagggtcaa                                                    19


<210>  338
<211>  19
<212>  DNA
<213>  Artificial

<220>
<223>  mCTGF

<400>  338
cgcctgttct aagacctgt                                                    19


<210>  339
<211>  19
<212>  DNA
<213>  Artificial

<220>
<223>  mCTGF

<400>  339
ctgtgcctgc cattacaac                                                    19


<210>  340
<211>  19
<212>  DNA
<213>  Artificial

<220>

<223>  mCTGF

<400>  340
agcggtgagt ccttccaaa                                                    19


<210>  341
<211>  19
<212>  DNA
<213>  Artificial

<220>
<223>  mCTGF

<400>  341
gcaccagtgt gaagacata                                                    19


<210>  342
<211>  20
<212>  DNA
<213>  Artificial

<220>
<223>  mCTGF

<400>  342
gacaatacct tctgcagact                                                   20


<210>  343
<211>  19
<212>  DNA
<213>  Artificial

<220>
<223>  mCTGF

<400>  343
cgcaagatcg gagtgtgca                                                    19


<210>  344
<211>  19
<212>  DNA
<213>  Artificial

<220>
<223>  mCTGF

<400>  344
gcaaaaagtg catccggac                                                    19


<210>  345
<211>  19
<212>  DNA
<213>  Artificial

<220>
<223>  mCTGF

<400>  345

tcttcggtgg gtcggtgta                                                    19

<210>  346
<211>  19
<212>  DNA
<213>  Artificial

<220>
<223>  mCTGF

<400>  346
tcccgagaag ggtcaagct                                                    19

<210>  347
<211>  19
<212>  DNA
<213>  Artificial

<220>
<223>  mCTGF

<400>  347
gtgcatccgg acacctaaa                                                    19

<210>  348
<211>  19
<212>  DNA
<213>  Artificial

<220>
<223>  mCTGF

<400>  348
cgatggcgag atcatgaaa                                                    19

<210>  349
<211>  19
<212>  DNA
<213>  Artificial

<220>
<223>  mCTGF

<400>  349
gatggcgaga tcatgaaaa                                                    19

<210>  350
<211>  19
<212>  DNA
<213>  Artificial

<220>
<223>  mCTGF

<400>  350
ctgtcaagtt tgagctttc                                                    19

<210>  351
<211>  19
<212>  DNA
<213>  Artificial

<220>
<223>  mCTGF

<400>  351
ctcttctgcg atttcggct                                                    19


<210>  352
<211>  19
<212>  DNA
<213>  Artificial

<220>
<223>  mCTGF

<400>  352
gttaccaatg acaatacct                                                    19


<210>  353
<211>  19
<212>  DNA
<213>  Artificial

<220>
<223>  mCTGF

<400>  353
cctgtcaagt ttgagcttt                                                    19


<210>  354
<211>  19
<212>  DNA
<213>  Artificial

<220>
<223>  mCTGF

<400>  354
ggaagatgta cggagacat                                                    19


<210>  355
<211>  19
<212>  DNA
<213>  Artificial

<220>
<223>  mCTGF

<400>  355
catccggaca cctaaaatc                                                    19


<210>  356
<211>  19
<212>  DNA

<213>  Artificial

<220>
<223>  mCTGF

<400>  356
tgtcaagttt gagctttct                                                    19


<210>  357
<211>  19
<212>  DNA
<213>  Artificial

<220>
<223>  mCTGF

<400>  357
caaggaccgc acagcagtt                                                    19


<210>  358
<211>  19
<212>  DNA
<213>  Artificial

<220>
<223>  mCTGF

<400>  358
ggaagacaca tttggccca                                                    19


<210>  359
<211>  19
<212>  DNA
<213>  Artificial

<220>
<223>  mCTGF

<400>  359
ggcaaaaagt gcatccgga                                                    19


<210>  360
<211>  19
<212>  DNA
<213>  Artificial

<220>
<223>  mCTGF

<400>  360
gactggaaga cacatttgg                                                    19


<210>  361
<211>  19
<212>  DNA
<213>  Artificial

<220>

<223>  mCTGF

<400>  361
tgaagacata cagggctaa                                                    19


<210>  362
<211>  19
<212>  DNA
<213>  Artificial

<220>
<223>  mCTGF

<400>  362
gggaaatgct gcgaggagt                                                    19


<210>  363
<211>  19
<212>  DNA
<213>  Artificial

<220>
<223>  mCTGF

<400>  363
acaggaagat gtacggaga                                                    19


<210>  364
<211>  19
<212>  DNA
<213>  Artificial

<220>
<223>  mCTGF

<400>  364
ttggcccaga cccaactat                                                    19


<210>  365
<211>  19
<212>  DNA
<213>  Artificial

<220>
<223>  mCTGF

<400>  365
cctaaaatcg ccaagcctg                                                    19


<210>  366
<211>  19
<212>  DNA
<213>  Artificial

<220>
<223>  mCTGF

<400>  366

gaagacatac agggctaag                                                    19

<210>  367
<211>  19
<212>  DNA
<213>  Artificial

<220>
<223>  mCTGF

<400>  367
actatgatgc gagccaact                                                    19

<210>  368
<211>  19
<212>  DNA
<213>  Artificial

<220>
<223>  mCTGF

<400>  368
agaagggcaa aaagtgcat                                                    19

<210>  369
<211>  19
<212>  DNA
<213>  Artificial

<220>
<223>  mCTGF

<400>  369
atcgccaagc ctgtcaagt                                                    19

<210>  370
<211>  19
<212>  DNA
<213>  Artificial

<220>
<223>  mCTGF

<400>  370
aggaagatgt acggagaca                                                    19

<210>  371
<211>  19
<212>  DNA
<213>  Artificial

<220>
<223>  mCTGF

<400>  371
ccgactggaa gacacattt                                                    19

<210>  372
<211>  19
<212>  DNA
<213>  Artificial

<220>
<223>  mCTGF

<400>  372
gcccagaccc aactatgat                                                    19


<210>  373
<211>  19
<212>  DNA
<213>  Artificial

<220>
<223>  mCTGF

<400>  373
cgccaagcct gtcaagttt                                                    19


<210>  374
<211>  19
<212>  DNA
<213>  Artificial

<220>
<223>  mCTGF

<400>  374
ccgatggcga gatcatgaa                                                    19


<210>  375
<211>  19
<212>  DNA
<213>  Artificial

<220>
<223>  mCTGF

<400>  375
tccggacacc taaaatcgc                                                    19


<210>  376
<211>  18
<212>  DNA
<213>  Artificial

<220>
<223>  mCTGF

<400>  376
tttgagcttt ctggctgc                                                     18


<210>  377
<211>  19
<212>  DNA

<213>  Artificial

<220>
<223>  mCTGF

<400>  377
ggagaactgt gtacggagc                                                    19


<210>  378
<211>  19
<212>  DNA
<213>  Artificial

<220>
<223>  mCTGF

<400>  378
ctgcaccagt gtgaagaca                                                    19


<210>  379
<211>  19
<212>  DNA
<213>  Artificial

<220>
<223>  mCTGF

<400>  379
agcgcctgtt ctaagacct                                                    19


<210>  380
<211>  19
<212>  DNA
<213>  Artificial

<220>
<223>  mCTGF

<400>  380
cgactggaag acacatttg                                                    19


<210>  381
<211>  19
<212>  DNA
<213>  Artificial

<220>
<223>  mCTGF

<400>  381
gagaagggtc aagctgcct                                                    19


<210>  382
<211>  19
<212>  DNA
<213>  Artificial

<220>

<223>  mCTGF

<400>  382
ctacaggaag atgtacgga                                                    19


<210>  383
<211>  19
<212>  DNA
<213>  Artificial

<220>
<223>  mCTGF

<400>  383
gacctggagg aaaacatta                                                    19


<210>  384
<211>  19
<212>  DNA
<213>  Artificial

<220>
<223>  mCTGF

<400>  384
tggagcgcct gttctaaga                                                    19


<210>  385
<211>  19
<212>  DNA
<213>  Artificial

<220>
<223>  mCTGF

<400>  385
tgccattaca actgtcctg                                                    19


<210>  386
<211>  19
<212>  DNA
<213>  Artificial

<220>
<223>  mCTGF

<400>  386
cggagtgtgc actgccaaa                                                    19


<210>  387
<211>  19
<212>  DNA
<213>  Artificial

<220>
<223>  mCTGF

<400>  387

tcggagtgtg cactgccaa                                                    19

<210>  388
<211>  19
<212>  DNA
<213>  Artificial

<220>
<223>  mCTGF

<400>  388
cggacaccta aaatcgcca                                                    19

<210>  389
<211>  19
<212>  DNA
<213>  Artificial

<220>
<223>  mCTGF

<400>  389
gcctgttcta agacctgtg                                                    19

<210>  390
<211>  19
<212>  DNA
<213>  Artificial

<220>
<223>  mCTGF

<400>  390
caggaagatg tacggagac                                                    19

<210>  391
<211>  19
<212>  DNA
<213>  Artificial

<220>
<223>  mCTGF

<400>  391
tgcaccagtg tgaagacat                                                    19

<210>  392
<211>  19
<212>  DNA
<213>  Artificial

<220>
<223>  mCTGF

<400>  392
tgcatccgga cacctaaaa                                                    19

<210>  393
<211>  19
<212>  DNA
<213>  Artificial

<220>
<223>  mCTGF

<400>  393
atggcgagat catgaaaaa                                                    19


<210>  394
<211>  19
<212>  DNA
<213>  Artificial

<220>
<223>  mCTGF

<400>  394
ccaaccgcaa gatcggagt                                                    19


<210>  395
<211>  19
<212>  DNA
<213>  Artificial

<220>
<223>  mCTGF

<400>  395
aacattaaga agggcaaaa                                                    19


<210>  396
<211>  19
<212>  DNA
<213>  Artificial

<220>
<223>  mCTGF

<400>  396
gccattacaa ctgtcctgg                                                    19


<210>  397
<211>  19
<212>  DNA
<213>  Artificial

<220>
<223>  mCTGF

<400>  397
tcgccaagcc tgtcaagtt                                                    19


<210>  398
<211>  19
<212>  DNA

<213>  Artificial

<220>
<223>  mCTGF

<400>  398
agctgggaga actgtgtac                                                    19


<210>  399
<211>  19
<212>  DNA
<213>  Artificial

<220>
<223>  mCTGF

<400>  399
agctgcctac cgactggaa                                                    19


<210>  400
<211>  19
<212>  DNA
<213>  Artificial

<220>
<223>  mCTGF

<400>  400
acagagtgga gcgcctgtt                                                    19


<210>  401
<211>  19
<212>  DNA
<213>  Artificial

<220>
<223>  mCyr61

<400>  401
ctgtgaagtg cgtccttgt                                                    19


<210>  402
<211>  19
<212>  DNA
<213>  Artificial

<220>
<223>  mCyr61

<400>  402
gtgaagatgc ggttccgat                                                    19


<210>  403
<211>  19
<212>  DNA
<213>  Artificial

<220>

<223>  mCyr61

<400>  403
ctgcaaatgt aactacaac                                                    19


<210>  404
<211>  19
<212>  DNA
<213>  Artificial

<220>
<223>  mCyr61

<400>  404
ggatgaatgg tgccttgct                                                    19


<210>  405
<211>  19
<212>  DNA
<213>  Artificial

<220>
<223>  mCyr61

<400>  405
gcagaccctg tgaatataa                                                    19


<210>  406
<211>  19
<212>  DNA
<213>  Artificial

<220>
<223>  mCyr61

<400>  406
gaagtaaatg aaagcgcct                                                    19


<210>  407
<211>  19
<212>  DNA
<213>  Artificial

<220>
<223>  mCyr61

<400>  407
ggacaaccag tgtacagca                                                    19


<210>  408
<211>  19
<212>  DNA
<213>  Artificial

<220>
<223>  mCyr61

<400>  408

gcattaagga ctccctgga                                                    19


<210>  409
<211>  19
<212>  DNA
<213>  Artificial

<220>
<223>  mCyr61

<400>  409
tgtacagcct attcaatga                                                    19


<210>  410
<211>  19
<212>  DNA
<213>  Artificial

<220>
<223>  mCyr61

<400>  410
cgactgtaca gcctattca                                                    19


<210>  411
<211>  19
<212>  DNA
<213>  Artificial

<220>
<223>  mCyr61

<400>  411
tggagcgggc attattgct                                                    19


<210>  412
<211>  19
<212>  DNA
<213>  Artificial

<220>
<223>  mCyr61

<400>  412
gacttcgctt catagtact                                                    19


<210>  413
<211>  19
<212>  DNA
<213>  Artificial

<220>
<223>  mCyr61

<400>  413
gtactggagc gggcattat                                                    19

<210>  414
<211>  19
<212>  DNA
<213>  Artificial

<220>
<223>  mCyr61

<400>  414
ggcattattg ctccatatt                                                    19


<210>  415
<211>  19
<212>  DNA
<213>  Artificial

<220>
<223>  mCyr61

<400>  415
gagttctttt caaccctct                                                    19


<210>  416
<211>  19
<212>  DNA
<213>  Artificial

<220>
<223>  mCyr61

<400>  416
ctgtgaatat aactccaga                                                    19


<210>  417
<211>  19
<212>  DNA
<213>  Artificial

<220>
<223>  mCyr61

<400>  417
cgaagatgga gagatgttt                                                    19


<210>  418
<211>  19
<212>  DNA
<213>  Artificial

<220>
<223>  mCyr61

<400>  418
ccaagaatgt catgatgat                                                    19


<210>  419
<211>  19
<212>  DNA

<213>  Artificial

<220>
<223>  mCyr61

<400>  419
ggagttaacg agaaacaat                                                    19


<210>  420
<211>  19
<212>  DNA
<213>  Artificial

<220>
<223>  mCyr61

<400>  420
cctgcactct aaaactgca                                                    19


<210>  421
<211>  19
<212>  DNA
<213>  Artificial

<220>
<223>  mCyr61

<400>  421
ggcaagaaat gcagcaaga                                                    19


<210>  422
<211>  19
<212>  DNA
<213>  Artificial

<220>
<223>  mCyr61

<400>  422
gccagaaatg catcgttca                                                    19


<210>  423
<211>  19
<212>  DNA
<213>  Artificial

<220>
<223>  mCyr61

<400>  423
gtctgcgcta aacaactca                                                    19


<210>  424
<211>  19
<212>  DNA
<213>  Artificial

<220>

<223>  mCyr61

<400>  424
gcaagcatca tggagacgt                                                    19


<210>  425
<211>  19
<212>  DNA
<213>  Artificial

<220>
<223>  mCyr61

<400>  425
gcgagttctt ttcaaccct                                                    19


<210>  426
<211>  19
<212>  DNA
<213>  Artificial

<220>
<223>  mCyr61

<400>  426
agtactggag cgggcatta                                                    19


<210>  427
<211>  19
<212>  DNA
<213>  Artificial

<220>
<223>  mCyr61

<400>  427
gaatggtgcc ttgctcatt                                                    19


<210>  428
<211>  19
<212>  DNA
<213>  Artificial

<220>
<223>  mCyr61

<400>  428
agaaataccg gcccaaata                                                    19


<210>  429
<211>  19
<212>  DNA
<213>  Artificial

<220>
<223>  mCyr61

<400>  429

cagccgcagt tggagaaga                                                    19


<210>  430
<211>  19
<212>  DNA
<213>  Artificial

<220>
<223>  mCyr61

<400>  430
gagagatgtt ttccaagaa                                                    19


<210>  431
<211>  19
<212>  DNA
<213>  Artificial

<220>
<223>  mCyr61

<400>  431
ctgtgaagtg cgtccttgt                                                    19


<210>  432
<211>  19
<212>  DNA
<213>  Artificial

<220>
<223>  mCyr61

<400>  432
gtgaagatgc ggttccgat                                                    19


<210>  433
<211>  19
<212>  DNA
<213>  Artificial

<220>
<223>  mCyr61

<400>  433
ctgcaaatgt aactacaac                                                    19


<210>  434
<211>  19
<212>  DNA
<213>  Artificial

<220>
<223>  mCyr61

<400>  434
gcagaccctg tgaatataa                                                    19

<210>  435
<211>  19
<212>  DNA
<213>  Artificial

<220>
<223>  mCyr61

<400>  435
gtgtacagca gcctaaaaa                                                    19


<210>  436
<211>  19
<212>  DNA
<213>  Artificial

<220>
<223>  mCyr61

<400>  436
ctgaagaggc ttcctgtct                                                    19


<210>  437
<211>  19
<212>  DNA
<213>  Artificial

<220>
<223>  mCyr61

<400>  437
gtaactacaa ctgcccgca                                                    19


<210>  438
<211>  19
<212>  DNA
<213>  Artificial

<220>
<223>  mCyr61

<400>  438
caagaaatac cggcccaaa                                                    19


<210>  439
<211>  19
<212>  DNA
<213>  Artificial

<220>
<223>  mCyr61

<400>  439
gcattaagga ctccctgga                                                    19


<210>  440
<211>  19
<212>  DNA

<213>  Artificial

<220>
<223>  mCyr61

<400>  440
ggacaaccag tgtacagca                                                    19


<210>  441
<211>  19
<212>  DNA
<213>  Artificial

<220>
<223>  mCyr61

<400>  441
agaaccagtc agatttact                                                    19


<210>  442
<211>  19
<212>  DNA
<213>  Artificial

<220>
<223>  mCyr61

<400>  442
tgtacagcct attcaatga                                                    19


<210>  443
<211>  19
<212>  DNA
<213>  Artificial

<220>
<223>  mCyr61

<400>  443
ccagtgcaca tgtattgat                                                    19


<210>  444
<211>  19
<212>  DNA
<213>  Artificial

<220>
<223>  mCyr61

<400>  444
cgactgtaca gcctattca                                                    19


<210>  445
<211>  19
<212>  DNA
<213>  Artificial

<220>

<223>  mCyr61

<400>  445
ctgtgaatat aactccaga                                                    19


<210>  446
<211>  19
<212>  DNA
<213>  Artificial

<220>
<223>  mCyr61

<400>  446
ggagttaacg agaaacaat                                                    19


<210>  447
<211>  19
<212>  DNA
<213>  Artificial

<220>
<223>  mCyr61

<400>  447
ccaagaatgt catgatgat                                                    19


<210>  448
<211>  19
<212>  DNA
<213>  Artificial

<220>
<223>  mCyr61

<400>  448
gaagaggctt cctgtcttt                                                    19


<210>  449
<211>  19
<212>  DNA
<213>  Artificial

<220>
<223>  mCyr61

<400>  449
cccatggcca gaaatgcat                                                    19


<210>  450
<211>  19
<212>  DNA
<213>  Artificial

<220>
<223>  mCyr61

<400>  450

gccagaaatg catcgttca                                                    19

<210>  451
<211>  19
<212>  DNA
<213>  Artificial

<220>
<223>  mCyr61

<400>  451
ggcaagaaat gcagcaaga                                                    19

<210>  452
<211>  19
<212>  DNA
<213>  Artificial

<220>
<223>  mCyr61

<400>  452
aggcagaccc tgtgaatat                                                    19

<210>  453
<211>  19
<212>  DNA
<213>  Artificial

<220>
<223>  mCyr61

<400>  453
gtctgcgcta aacaactca                                                    19

<210>  454
<211>  19
<212>  DNA
<213>  Artificial

<220>
<223>  mCyr61

<400>  454
gcgagttctt ttcaaccct                                                    19

<210>  455
<211>  19
<212>  DNA
<213>  Artificial

<220>
<223>  mCyr61

<400>  455
gctctgaaag ggatctgca                                                    19

<210>  456
<211>  19
<212>  DNA
<213>  Artificial

<220>
<223>  mCyr61

<400>  456
tgaagagtgg gtttgtgat                                                    19


<210>  457
<211>  19
<212>  DNA
<213>  Artificial

<220>
<223>  mCyr61

<400>  457
agaaataccg gcccaaata                                                    19


<210>  458
<211>  19
<212>  DNA
<213>  Artificial

<220>
<223>  mCyr61

<400>  458
gagagatgtt ttccaagaa                                                    19


<210>  459
<211>  19
<212>  DNA
<213>  Artificial

<220>
<223>  mCyr61

<400>  459
gactgtacag cctattcaa                                                    19


<210>  460
<211>  19
<212>  DNA
<213>  Artificial

<220>
<223>  mCyr61

<400>  460
agttaccaat gacaaccca                                                    19


<210>  461
<211>  19
<212>  DNA

<213>  Artificial

<220>
<223>  mCyr61

<400>  461
ccggatctgt gaagtgcgt                                                    19


<210>  462
<211>  19
<212>  DNA
<213>  Artificial

<220>
<223>  mCyr61

<400>  462
atgcagcaag accaagaaa                                                    19


<210>  463
<211>  19
<212>  DNA
<213>  Artificial

<220>
<223>  mCyr61

<400>  463
ctaaacaact caacgagga                                                    19


<210>  464
<211>  19
<212>  DNA
<213>  Artificial

<220>
<223>  mCyr61

<400>  464
gaaataccgg cccaaatac                                                    19


<210>  465
<211>  19
<212>  DNA
<213>  Artificial

<220>
<223>  mCyr61

<400>  465
ctgtaaggtc tgcgctaaa                                                    19


<210>  466
<211>  19
<212>  DNA
<213>  Artificial

<220>

<223>  mCyr61

<400>  466
gaaccgcgag ttcttttca                                                    19


<210>  467
<211>  19
<212>  DNA
<213>  Artificial

<220>
<223>  mCyr61

<400>  467
gatgctccag tgtcaagaa                                                    19


<210>  468
<211>  19
<212>  DNA
<213>  Artificial

<220>
<223>  mCyr61

<400>  468
gcgaagatgg agagatgtt                                                    19


<210>  469
<211>  19
<212>  DNA
<213>  Artificial

<220>
<223>  mCyr61

<400>  469
gatccagtcc tgcaaatgt                                                    19


<210>  470
<211>  19
<212>  DNA
<213>  Artificial

<220>
<223>  mCyr61

<400>  470
cctgcaaatg taactacaa                                                    19


<210>  471
<211>  19
<212>  DNA
<213>  Artificial

<220>
<223>  mCyr61

<400>  471

ttccgactgt acagcctat                                                    19

<210>  472
<211>  19
<212>  DNA
<213>  Artificial

<220>
<223>  mCyr61

<400>  472
aagtgcgtcc ttgtggaca                                                    19

<210>  473
<211>  19
<212>  DNA
<213>  Artificial

<220>
<223>  mCyr61

<400>  473
acaactgccc gcatcccaa                                                    19

<210>  474
<211>  19
<212>  DNA
<213>  Artificial

<220>
<223>  mCyr61

<400>  474
gctgtaaggt ctgcgctaa                                                    19

<210>  475
<211>  19
<212>  DNA
<213>  Artificial

<220>
<223>  mCyr61

<400>  475
gagttaatcg caattggaa                                                    19

<210>  476
<211>  19
<212>  DNA
<213>  Artificial

<220>
<223>  mCyr61

<400>  476
ccttgtggac aaccagtgt                                                    19

<210>  477
<211>  19
<212>  DNA
<213>  Artificial

<220>
<223>  mCyr61

<400>  477
gttccgatgc gaagatgga                                                    19


<210>  478
<211>  19
<212>  DNA
<213>  Artificial

<220>
<223>  mCyr61

<400>  478
gatggagaga tgttttcca                                                    19


<210>  479
<211>  19
<212>  DNA
<213>  Artificial

<220>
<223>  mCyr61

<400>  479
ggagagatgt tttccaaga                                                    19


<210>  480
<211>  19
<212>  DNA
<213>  Artificial

<220>
<223>  mCyr61

<400>  480
gcctattcaa tgacatcca                                                    19


<210>  481
<211>  19
<212>  DNA
<213>  Artificial

<220>
<223>  mCyr61

<400>  481
ccatggccag aaatgcatc                                                    19


<210>  482
<211>  19
<212>  DNA

<213>  Artificial

<220>
<223>  mCyr61

<400>  482
tcaagaaata ccggcccaa                                                    19


<210>  483
<211>  19
<212>  DNA
<213>  Artificial

<220>
<223>  mCyr61

<400>  483
cccaactgta aacaccagt                                                    19


<210>  484
<211>  19
<212>  DNA
<213>  Artificial

<220>
<223>  mCyr61

<400>  484
ctgtaaacac cagtgcaca                                                    19


<210>  485
<211>  19
<212>  DNA
<213>  Artificial

<220>
<223>  mCyr61

<400>  485
ggaggtggag ttaacgaga                                                    19


<210>  486
<211>  19
<212>  DNA
<213>  Artificial

<220>
<223>  mCyr61

<400>  486
tccagcccaa ctgtaaaca                                                    19


<210>  487
<211>  19
<212>  DNA
<213>  Artificial

<220>

<223>  mCyr61

<400>  487
tgcagcaaga ccaagaaat                                                    19


<210>  488
<211>  19
<212>  DNA
<213>  Artificial

<220>
<223>  mCyr61

<400>  488
aagaaatacc ggcccaaat                                                    19


<210>  489
<211>  19
<212>  DNA
<213>  Artificial

<220>
<223>  mCyr61

<400>  489
catgtattga tggcgccgt                                                    19


<210>  490
<211>  19
<212>  DNA
<213>  Artificial

<220>
<223>  mCyr61

<400>  490
tgaagaggct tcctgtctt                                                    19


<210>  491
<211>  19
<212>  DNA
<213>  Artificial

<220>
<223>  mCyr61

<400>  491
tcctgcaaat gtaactaca                                                    19


<210>  492
<211>  19
<212>  DNA
<213>  Artificial

<220>
<223>  mCyr61

<400>  492

ccaccgctct gaaagggat                                                    19


<210>  493
<211>  19
<212>  DNA
<213>  Artificial

<220>
<223>  mCyr61

<400>  493
cccaaatact gcggctcct                                                    19


<210>  494
<211>  19
<212>  DNA
<213>  Artificial

<220>
<223>  mCyr61

<400>  494
ctctgcagac cagaactgt                                                    19


<210>  495
<211>  19
<212>  DNA
<213>  Artificial

<220>
<223>  mCyr61

<400>  495
ggttggaatg caatttcgg                                                    19


<210>  496
<211>  19
<212>  DNA
<213>  Artificial

<220>
<223>  mCyr61

<400>  496
aacaccagtg cacatgtat                                                    19


<210>  497
<211>  19
<212>  DNA
<213>  Artificial

<220>
<223>  mCyr61

<400>  497
agagtgggtt tgtgatgaa                                                    19

<210>  498
<211>  19
<212>  DNA
<213>  Artificial

<220>
<223>  mCyr61

<400>  498
agcccaactg taaacacca                                                    19


<210>  499
<211>  19
<212>  DNA
<213>  Artificial

<220>
<223>  mCyr61

<400>  499
cgcaattgga aaaggcagc                                                    19


<210>  500
<211>  19
<212>  DNA
<213>  Artificial

<220>
<223>  mCyr61

<400>  500
gaatgcaatt tcggcgcca                                                    19


<210>  501
<211>  19
<212>  DNA
<213>  Artificial

<220>
<223>  mPlekho1

<400>  501
gagaaggagt catggatca                                                    19


<210>  502
<211>  19
<212>  DNA
<213>  Artificial

<220>
<223>  mPlekho1

<400>  502
gatttggaaa aaccgctat                                                    19


<210>  503
<211>  19
<212>  DNA

<213>  Artificial

<220>
<223>  mPlekho1

<400>  503
ggatgctaac attagacct                                                    19


<210>  504
<211>  19
<212>  DNA
<213>  Artificial

<220>
<223>  mPlekho1

<400>  504
cacaagcact gaagaagtt                                                    19


<210>  505
<211>  19
<212>  DNA
<213>  Artificial

<220>
<223>  mPlekho1

<400>  505
tgtttgacct gagtgacta                                                    19


<210>  506
<211>  19
<212>  DNA
<213>  Artificial

<220>
<223>  mPlekho1

<400>  506
cgccctgagt tctgccatt                                                    19


<210>  507
<211>  19
<212>  DNA
<213>  Artificial

<220>
<223>  mPlekho1

<400>  507
cattagacct gatccaaga                                                    19


<210>  508
<211>  19
<212>  DNA
<213>  Artificial

<220>

<223>  mPlekho1

<400>  508
ggacctggta gcaaggaaa                                                    19


<210>  509
<211>  19
<212>  DNA
<213>  Artificial

<220>
<223>  mPlekho1

<400>  509
ggaaactgga gaagactca                                                    19


<210>  510
<211>  19
<212>  DNA
<213>  Artificial

<220>
<223>  mPlekho1

<400>  510
tggagaagac tcaggagct                                                    19


<210>  511
<211>  19
<212>  DNA
<213>  Artificial

<220>
<223>  mPlekho1

<400>  511
ctctacctca gatgggatg                                                    19


<210>  512
<211>  19
<212>  DNA
<213>  Artificial

<220>
<223>  mPlekho1

<400>  512
cctatcccag agggacact                                                    19


<210>  513
<211>  19
<212>  DNA
<213>  Artificial

<220>
<223>  mPlekho1

<400>  513

ggaaaaaccg ctatgtggt                                                    19

<210>  514
<211>  19
<212>  DNA
<213>  Artificial

<220>
<223>  mPlekho1

<400>  514
gtttgacctg agtgactat                                                    19

<210>  515
<211>  19
<212>  DNA
<213>  Artificial

<220>
<223>  mPlekho1

<400>  515
ctcagatggg atgctaaca                                                    19

<210>  516
<211>  19
<212>  DNA
<213>  Artificial

<220>
<223>  mPlekho1

<400>  516
gagctgagag acctgtaca                                                    19

<210>  517
<211>  19
<212>  DNA
<213>  Artificial

<220>
<223>  mPlekho1

<400>  517
ctcgagacag agcaaaaat                                                    19

<210>  518
<211>  19
<212>  DNA
<213>  Artificial

<220>
<223>  mPlekho1

<400>  518
agtcatggat caacgccct                                                    19

<210>  519
<211>  19
<212>  DNA
<213>  Artificial

<220>
<223>  mPlekho1

<400>  519
aaagagagtg ctgcaggaa                                                    19


<210>  520
<211>  19
<212>  DNA
<213>  Artificial

<220>
<223>  mPlekho1

<400>  520
actatgagaa gtgcgaaga                                                    19


<210>  521
<211>  19
<212>  DNA
<213>  Artificial

<220>
<223>  mPlekho1

<400>  521
gactcccacc tcagacaga                                                    19


<210>  522
<211>  19
<212>  DNA
<213>  Artificial

<220>
<223>  mPlekho1

<400>  522
cgtgagtcct gaagagaag                                                    19


<210>  523
<211>  19
<212>  DNA
<213>  Artificial

<220>
<223>  mPlekho1

<400>  523
tgaaaggcga ccagctcta                                                    19


<210>  524
<211>  19
<212>  DNA

<213>  Artificial

<220>
<223>  mPlekho1

<400>  524
cctcttgtgc tgagagctt                                                    19


<210>  525
<211>  19
<212>  DNA
<213>  Artificial

<220>
<223>  mPlekho1

<400>  525
ccacaagcac tgaagaagt                                                    19


<210>  526
<211>  19
<212>  DNA
<213>  Artificial

<220>
<223>  mPlekho1

<400>  526
cacagtcaat accggaaga                                                    19


<210>  527
<211>  19
<212>  DNA
<213>  Artificial

<220>
<223>  mPlekho1

<400>  527
cctactcgag acagagcaa                                                    19


<210>  528
<211>  19
<212>  DNA
<213>  Artificial

<220>
<223>  mPlekho1

<400>  528
ccgtgagtcc tgaagagaa                                                    19


<210>  529
<211>  19
<212>  DNA
<213>  Artificial

<220>

<223>  mPlekho1

<400>  529
ctaaaaaccg tatcttgga                                                    19


<210>  530
<211>  19
<212>  DNA
<213>  Artificial

<220>
<223>  mPlekho1

<400>  530
gtgtttgacc tgagtgact                                                    19


<210>  531
<211>  19
<212>  DNA
<213>  Artificial

<220>
<223>  mPlekho1

<400>  531
gagaaggagt catggatca                                                    19


<210>  532
<211>  19
<212>  DNA
<213>  Artificial

<220>
<223>  mPlekho1

<400>  532
ccagagctaa aaaccgtat                                                    19


<210>  533
<211>  19
<212>  DNA
<213>  Artificial

<220>
<223>  mPlekho1

<400>  533
ctatcttgcc caccctact                                                    19


<210>  534
<211>  19
<212>  DNA
<213>  Artificial

<220>
<223>  mPlekho1

<400>  534

gatttggaaa aaccgctat                                                    19


<210>  535
<211>  19
<212>  DNA
<213>  Artificial

<220>
<223>  mPlekho1

<400>  535
ctgagtgact atgagaagt                                                    19


<210>  536
<211>  19
<212>  DNA
<213>  Artificial

<220>
<223>  mPlekho1

<400>  536
ggatgctaac attagacct                                                    19


<210>  537
<211>  19
<212>  DNA
<213>  Artificial

<220>
<223>  mPlekho1

<400>  537
ctccggatgg aaaccatca                                                    19


<210>  538
<211>  19
<212>  DNA
<213>  Artificial

<220>
<223>  mPlekho1

<400>  538
caccttcaag ctgaggaat                                                    19


<210>  539
<211>  19
<212>  DNA
<213>  Artificial

<220>
<223>  mPlekho1

<400>  539
ccgctatgtg gtgctgaaa                                                    19

<210>  540
<211>  19
<212>  DNA
<213>  Artificial

<220>
<223>  mPlekho1

<400>  540
ggaggtaaaa gatgagaaa                                                    19


<210>  541
<211>  19
<212>  DNA
<213>  Artificial

<220>
<223>  mPlekho1

<400>  541
tgtttgacct gagtgacta                                                    19


<210>  542
<211>  19
<212>  DNA
<213>  Artificial

<220>
<223>  mPlekho1

<400>  542
cgccctgagt tctgccatt                                                    19


<210>  543
<211>  19
<212>  DNA
<213>  Artificial

<220>
<223>  mPlekho1

<400>  543
cattagacct gatccaaga                                                    19


<210>  544
<211>  19
<212>  DNA
<213>  Artificial

<220>
<223>  mPlekho1

<400>  544
ggacctggta gcaaggaaa                                                    19


<210>  545
<211>  19
<212>  DNA

<213>  Artificial

<220>
<223>  mPlekho1

<400>  545
ggaaactgga gaagactca                                                    19


<210>  546
<211>  19
<212>  DNA
<213>  Artificial

<220>
<223>  mPlekho1

<400>  546
tggagaagac tcaggagct                                                    19


<210>  547
<211>  19
<212>  DNA
<213>  Artificial

<220>
<223>  mPlekho1

<400>  547
ctctacctca gatgggatg                                                    19


<210>  548
<211>  19
<212>  DNA
<213>  Artificial

<220>
<223>  mPlekho1

<400>  548
cgtatcttgg atgaggtca                                                    19


<210>  549
<211>  19
<212>  DNA
<213>  Artificial

<220>
<223>  mPlekho1

<400>  549
ggaaaaaccg ctatgtggt                                                    19


<210>  550
<211>  19
<212>  DNA
<213>  Artificial

<220>

<223>  mPlekho1

<400>  550
cctatcccag agggacact                                                    19


<210>  551
<211>  19
<212>  DNA
<213>  Artificial

<220>
<223>  mPlekho1

<400>  551
gtcaccgttg aggaggaca                                                    19


<210>  552
<211>  19
<212>  DNA
<213>  Artificial

<220>
<223>  mPlekho1

<400>  552
ctttctcggc cttgggaaa                                                    19


<210>  553
<211>  19
<212>  DNA
<213>  Artificial

<220>
<223>  mPlekho1

<400>  553
gagctgagag acctgtaca                                                    19


<210>  554
<211>  19
<212>  DNA
<213>  Artificial

<220>
<223>  mPlekho1

<400>  554
agaaggaggt aaaagatga                                                    19


<210>  555
<211>  19
<212>  DNA
<213>  Artificial

<220>
<223>  mPlekho1

<400>  555

agtcatggat caacgccct                                                    19


<210>  556
<211>  19
<212>  DNA
<213>  Artificial

<220>
<223>  mPlekho1

<400>  556
actatgagaa gtgcgaaga                                                    19


<210>  557
<211>  19
<212>  DNA
<213>  Artificial

<220>
<223>  mPlekho1

<400>  557
aaagagagtg ctgcaggaa                                                    19


<210>  558
<211>  19
<212>  DNA
<213>  Artificial

<220>
<223>  mPlekho1

<400>  558
cctcccagca cagtcaata                                                    19


<210>  559
<211>  19
<212>  DNA
<213>  Artificial

<220>
<223>  mPlekho1

<400>  559
cgtgagtcct gaagagaag                                                    19


<210>  560
<211>  19
<212>  DNA
<213>  Artificial

<220>
<223>  mPlekho1

<400>  560
tgaaaggcga ccagctcta                                                    19

<210>  561
<211>  19
<212>  DNA
<213>  Artificial

<220>
<223>  mPlekho1

<400>  561
cgggtcgatc tggacaagt                                                    19


<210>  562
<211>  19
<212>  DNA
<213>  Artificial

<220>
<223>  mPlekho1

<400>  562
ccggaaattc tgcgggaaa                                                    19


<210>  563
<211>  19
<212>  DNA
<213>  Artificial

<220>
<223>  mPlekho1

<400>  563
gtgtttgacc tgagtgact                                                    19


<210>  564
<211>  19
<212>  DNA
<213>  Artificial

<220>
<223>  mPlekho1

<400>  564
ccgtgagtcc tgaagagaa                                                    19


<210>  565
<211>  19
<212>  DNA
<213>  Artificial

<220>
<223>  mPlekho1

<400>  565
ctaaaaaccg tatcttgga                                                    19


<210>  566
<211>  19
<212>  DNA

<213>  Artificial

<220>
<223>  mPlekho1

<400>  566
cgttgaggag gacagctat                                                    19


<210>  567
<211>  19
<212>  DNA
<213>  Artificial

<220>
<223>  mPlekho1

<400>  567
cctactcgag acagagcaa                                                    19


<210>  568
<211>  19
<212>  DNA
<213>  Artificial

<220>
<223>  mPlekho1

<400>  568
cctcttgtgc tgagagctt                                                    19


<210>  569
<211>  19
<212>  DNA
<213>  Artificial

<220>
<223>  mPlekho1

<400>  569
ccacaagcac tgaagaagt                                                    19


<210>  570
<211>  19
<212>  DNA
<213>  Artificial

<220>
<223>  mPlekho1

<400>  570
cacagtcaat accggaaga                                                    19


<210>  571
<211>  19
<212>  DNA
<213>  Artificial

<220>

<223>  mPlekho1

<400>  571
tgactatgag aagtgcgaa                                                    19


<210>  572
<211>  19
<212>  DNA
<213>  Artificial

<220>
<223>  mPlekho1

<400>  572
caggaggtgt ttgacctga                                                    19


<210>  573
<211>  19
<212>  DNA
<213>  Artificial

<220>
<223>  mPlekho1

<400>  573
cctctacctc agatgggat                                                    19


<210>  574
<211>  19
<212>  DNA
<213>  Artificial

<220>
<223>  mPlekho1

<400>  574
ggccttggga aaaaccaga                                                    19


<210>  575
<211>  19
<212>  DNA
<213>  Artificial

<220>
<223>  mPlekho1

<400>  575
gatgggatgc taacattag                                                    19


<210>  576
<211>  19
<212>  DNA
<213>  Artificial

<220>
<223>  mPlekho1

<400>  576

agaaggagtc atggatcaa                                                    19

<210>  577
<211>  19
<212>  DNA
<213>  Artificial

<220>
<223>  mPlekho1

<400>  577
acctcccagc acagtcaat                                                    19

<210>  578
<211>  19
<212>  DNA
<213>  Artificial

<220>
<223>  mPlekho1

<400>  578
ccgttgagga ggacagcta                                                    19

<210>  579
<211>  19
<212>  DNA
<213>  Artificial

<220>
<223>  mPlekho1

<400>  579
ccaccctact cgagacaga                                                    19

<210>  580
<211>  19
<212>  DNA
<213>  Artificial

<220>
<223>  mPlekho1

<400>  580
gacaggatcc agccctctt                                                    19

<210>  581
<211>  19
<212>  DNA
<213>  Artificial

<220>
<223>  mPlekho1

<400>  581
ctactcgaga cagagcaaa                                                    19

<210>  582
<211>  19
<212>  DNA
<213>  Artificial

<220>
<223>  mPlekho1

<400>  582
tcaccttcaa gctgaggaa                                                    19


<210>  583
<211>  19
<212>  DNA
<213>  Artificial

<220>
<223>  mPlekho1

<400>  583
cggactcaga caggatcca                                                    19


<210>  584
<211>  19
<212>  DNA
<213>  Artificial

<220>
<223>  mPlekho1

<400>  584
ggcttcgacc tctacctca                                                    19


<210>  585
<211>  19
<212>  DNA
<213>  Artificial

<220>
<223>  mPlekho1

<400>  585
gagctaaaaa ccgtatctt                                                    19


<210>  586
<211>  19
<212>  DNA
<213>  Artificial

<220>
<223>  mPlekho1

<400>  586
ccgtatcttg gatgaggtc                                                    19


<210>  587
<211>  19
<212>  DNA

<213>  Artificial

<220>
<223>  mPlekho1

<400>  587
tcaagctgag gaatcccta                                                    19


<210>  588
<211>  19
<212>  DNA
<213>  Artificial

<220>
<223>  mPlekho1

<400>  588
tgagagacct gtacaggca                                                    19


<210>  589
<211>  19
<212>  DNA
<213>  Artificial

<220>
<223>  mPlekho1

<400>  589
ggacagctat cttgcccac                                                    19


<210>  590
<211>  19
<212>  DNA
<213>  Artificial

<220>
<223>  mPlekho1

<400>  590
ctgtggcttc gacctctac                                                    19


<210>  591
<211>  19
<212>  DNA
<213>  Artificial

<220>
<223>  mPlekho1

<400>  591
ttttcaggga gatttggaa                                                    19


<210>  592
<211>  19
<212>  DNA
<213>  Artificial

<220>

<223>  mPlekho1

<400>  592
accgctatgt ggtgctgaa                                                    19


<210>  593
<211>  19
<212>  DNA
<213>  Artificial

<220>
<223>  mPlekho1

<400>  593
tggatcaacg ccctgagtt                                                    19


<210>  594
<211>  19
<212>  DNA
<213>  Artificial

<220>
<223>  mPlekho1

<400>  594
aaaaccgtat cttggatga                                                    19


<210>  595
<211>  19
<212>  DNA
<213>  Artificial

<220>
<223>  mPlekho1

<400>  595
agacctgatc caagaggaa                                                    19


<210>  596
<211>  19
<212>  DNA
<213>  Artificial

<220>
<223>  mPlekho1

<400>  596
acctgatcca agaggaaga                                                    19


<210>  597
<211>  19
<212>  DNA
<213>  Artificial

<220>
<223>  mPlekho1

<400>  597

gaggtaaaag atgagaaaa                                                    19

<210>  598
<211>  19
<212>  DNA
<213>  Artificial

<220>
<223>  mPlekho1

<400>  598
acgtctccga gaaggaggt                                                    19

<210>  599
<211>  19
<212>  DNA
<213>  Artificial

<220>
<223>  mPlekho1

<400>  599
agagaaggag tcatggatc                                                    19

<210>  600
<211>  19
<212>  DNA
<213>  Artificial

<220>
<223>  mPlekho1

<400>  600
acctctacct cagatggga                                                    19

<210>  601
<211>  19
<212>  DNA
<213>  Artificial

<220>
<223>  SiCON

<400>  601
cuuacgcuga guacuucga                                                    19

<210>  602
<211>  23
<212>  DNA
<213>  Artificial

<220>
<223>  hCTGF

<400>  602
ttacaactgt cccggagaca atg                                               23

<210>  603
<211>  23
<212>  DNA
<213>  Artificial

<220>
<223>  hCTGF

<400>  603
aagggcaaaa agtgcatccg tac                                              23


<210>  604
<211>  23
<212>  DNA
<213>  Artificial

<220>
<223>  hCTGF

<400>  604
ttgaatcgct gtactacagg aag                                              23

## Description

### Technical Field

The present invention relates to a respiratory disease-related gene-specific siRNA and a high efficient structure comprising double helical-oligo RNA ('double helical-oligo RNA structure') containing the siRNA. The double helical-oligo RNA structure has a structure in which a hydrophilic material and a hydrophobic material are conjugated to both ends of double helical RNA (siRNA) by using a simple covalent bond or a linker-mediated covalent bond so as to be effectively delivered into cells, wherein the structure may be converted into a nanoparticle form by hydrophobic interactions of the double helical-oligo RNA structures in an aqueous solution. The siRNA included in the double helical-oligo RNA structure is preferably a siRNA specific to CTGF, Cyr61, or Plekho1 (hereinafter, referred to as a CTGF, Cyr61 or Plekho1-specific siRNA), which is a gene related with respiratory diseases, particularly, idiopathic pulmonary fibrosis and chronic obstructive pulmonary disease (COPD).

Further, the present invention relates to a method for producing the double-helical oligo RNA structure and a pharmaceutical composition containing the double-helical oligo RNA structure for preventing or treating respiratory diseases, particularly, idiopathic pulmonary fibrosis and COPD.

### Background Art

Technologies for inhibiting gene expression are important tools in the development of a therapeutic agent and target validation for treating diseases. Among the technologies, since a role of an RNA interference (hereinafter, referred to as 'RNAi') had been found, it was found that the RNA interference acts on a sequence-specific mRNA in various kinds of mammalian cells (Silence of the transcripts: RNA interference in medicine. J. Mol. Med. (2005) 83: 764-773).When a long-chain double-stranded RNA is delivered into a cell, the delivered double-stranded RNA is converted into a small interfering RNA (hereinafter, referred to as 'siRNA') which is processed to 21 to 23 base pairs (bp) by Dicer endonuclease. siRNA is bonded to an RNA-induced silencing complex (RISC), whereby a guide (antisense) strand recognizes and decomposes a target mRNA to sequence-specifically inhibit expression of a target gene (NUCLEIC-ACID THERAPEUTICS: BASIC PRINCIPLES AND RECENT APPLICATIONS. Nature Reviews Drug Discovery. 2002. 1, 503-514) .

Bertrand et al., found that as compared to an antisense oligonucleotide (ASO) on the same target gene, siRNA has an effect of significantly inhibiting mRNA expression in vitro and in vivo, and the corresponding effect is maintained for a long time (Comparison of antisense oligonucleotides and siRNAs in cell culture and in vivo. Biochem. Biophys. Res. Commun. 2002. 296: 1000-1004).In addition, since siRNA is complementarily coupled to a target mRNA to sequence-specifically regulate an expression of the target gene, a mechanism of the siRNA has an advantage in that a target to be applicable may be remarkably increased as compared to the existing antibody-based medical product or chemical pharmaceuticals (small molecular drug) (Progress Towards in Vivo Use of siRNAs. MOLECULAR THERAPY. 2006 13(4):664-670).

In order to develop the siRNA as a therapeutic agent even with excellent effect and variously usable range of the siRNA, the siRNA needs to be effectively delivered to a target cell with improved stability and a more efficient cell delivery of the siRNA (Harnessing In Vivo siRNA Delivery for Drug Discovery and Therapeutic Development. Drug Discov. Today. 2006 Jan; 11(1-2):67-73.

In order to solve the problems, research into modification of some nucleotides or backbone of siRNA to have a nucleic acid lyase resistance, or use of carriers such as viral vectors, liposomes or nanoparticles, etc. for improving stability in vivo, has been actively attempted.

Delivery systems using viral vectors such as adenovirus, retrovirus, etc, have high transfection efficacy, and also have high immunogenicity and oncogenicity. Meanwhile, a non-viral delivery system including nanoparticles has a low cell delivery efficiency as compared to the viral delivery system, but has high stability in vivo and is possible to be target-specifically delivered, has highly improved delivery effects such as uptake, internalization, etc., of RNAi oligonucleotide into cells or tissues, and rarely has cytotoxicity and immune stimulation, such that the non-viral delivery system is currently evaluated as a viable delivery method as compared to the viral delivery systems (Nonviral delivery of synthetic siRNA s in vivo. J Clin Invest. 2007 December 3; 117 (12) : 3623-3632).

In a method using a nanocarrier in the non-viral delivery systems, various polymers such as liposomes, cationic polymer composites, etc., are used to form nanoparticles, and a siRNA is supported on the nanoparticles, that is, nanocarrier, to be delivered into the cells. Among the methods using nanocarriers, a method using polymeric nanoparticle, polymer micelle, lipoplex, or the like, is mainly used, wherein the lipoplex consists of cationic lipid to interact with anionic lipid of endosome of a cell, thereby eliciting a destabilization effect of the endosome to deliver the siRNA into a cell (Proc. Natl. Acad. Sci. 15; 93 (21) :11493-8, 1996).

In addition, it is known that chemical materials, etc., are connected to end portions of a siRNA passenger (sense) strand to provide promoted pharmacokinetics characteristics, such that high efficacy may be induced in vivo (Nature 11; 432(7014):173-8, 2004). Here, stability of the siRNA may vary depending on properties of the chemical materials bonded to ends of the siRNA sense (passenger) or antisense (guide) strand. For example, a siRNA to which a polymer compound such as polyethylene glycol (PEG) is conjugated, interacts with an anionic phosphate group of siRNA in the presence of cationic materials to form a complex, thereby being a carrier having an improved siRNA stability (J. Control Release 129(2):107-16, 2008). In particular, micelle consisting of polymer complexes has an extremely small size, significantly uniform distribution, and is spontaneously form, thereby being easy to manage quality of formulation and secure reproducibility, as compared to other systems used as a drug delivery vehicle, such as microsphere, nanoparticle, etc.

Further, in order to improve an intracellular delivery efficiency of siRNA, technology of using a siRNA conjugate in which hydrophilic material which is a biocompatible polymer (for example, polyethylene glycol (PEG)) is conjugated to the siRNA by a simple covalent bond or a linker-mediated covalent bond, to thereby secure stability of siRNA and have effective cell membrane permeability was developed (see Korean Patent Publication No. 883471). However, the chemical modification of the siRNA and the conjugation with the polyethylene glycol (PEG) (PEGylation) still have disadvantages that stability in vivo is low and delivery into a target organ is not smooth. In order to solve the disadvantages, a double-helical oligo RNA structure in which a hydrophilic material and a hydrophobic material are bonded to oligonucleotides, in particular, double helical RNA such as siRNA, was developed, wherein the double-helical oligo RNA structure forms a self assembled nanoparticle named a self assembled micelle inhibitory RNA (SAMiRNA™) by a hydrophobic interaction of a hydrophobic material (see Korean Patent Publication No. 1224828), the SAMiRNA™ technology has an advantage in that homogenous nanoparticles having a significantly small size are capable of being obtained as compared to the existing delivery technologies.

As a specific example of the SAMiRNA™ technology, PEG (polyethylene glycol) is used as a hydrophilic material, wherein PEG is synthetic polymer, which is used for increasing solubility of pharmaceuticals, particularly, protein, and for controlling pharmacokinetics. PEG is a polydisperse material, a polymer in one batch consists of the sum of different number of monomers, a molecular weight is shown in the Gaussian curve, and polydispersity values (Mw/Mn) express the homogeneity degree of a material. That is, when PEG has a low molecular weight (3 to 5 kDa), the polydisperse value is about 1.01, and when PEG has a high molecular weight (20 kDa), the polydisperse value is about 1.2, which is high, such that as the molecular weight is higher, the homogeneity of the material is relatively low (F. M. Veronese. Peptide and protein PEGylation: a review of problems and solutions. Biomaterials (2001) 22:405-417). Accordingly, when the PEG is combined to the pharmaceuticals, polydispersed characteristic of PEG is reflected on the conjugate, such that it is not easy to perform verification of single material, and accordingly, production of materials having a low polydisperse value through synthesis of PEG and improvement of purification processes is on a rising trend, but has still problems due to polydispersed characteristic of the material, particularly, when PEG is combined to a material having a small molecular weight, there is difficult in confirming whether or not the combination is easily performed, etc. (Francesco M. Veronese and Gianfranco Pasut. PEGylation, successful approach to drug delivery. DRUG DISCOVERY TODAY (2005) 10 (21) :1451-1458) .

Accordingly, recently, as an improved technology of SAMiRNA™ which is the existing self-assembled nanoparticles, a technology of a new form of carrier having a significantly small size as compared to the existing SAMiRNA™ and remarkably improved polydispersity has been developed by blocking the hydrophilic material of the double helical RNA structure configuring SAMiRNA™ as a base unit including 1 to 15 uniform monomers having a predetermined molecular weight and a linker as needed, and using the appropriate number of blocked hydrophilic materials as needed.

Meanwhile, since biopharmaceuticals specifically act to a target gene sequence or a protein structure, in order to evaluate the efficacy and safety in a non-clinical surrogate model, a material acting with the same mechanism as the corresponding biopharmaceuticals in human is additionally required even in species of the surrogate model. Therefore, in order to avoid difficulty in additionally finding the material including the same mechanism as human, a material acting with the same mechanism both in human (treatment target) in the mouse (the non-clinical surrogate model), is required to be developed.

Idiopathic Pulmonary Fibrosis (hereinafter, abbreviated as 'IPF') is a disease in which chronic inflammatory cells penetrate into a wall of an alveolar wall (lung alveolus) to make the lung become hard, causing severe structural change in lung tissue, such that lung function is gradually reduced to induce death. However, an effective treatment thereof does not exist yet, and Idiopathic Pulmonary Fibrosis is generally diagnosed when symptoms appear at last, and has extremely bad prognosis since a median survival time is only about three to five years. It is reported that the incidence frequency of the foreign countries is about 3-5 people per 100,000, and it is known that the incidence is generally high after the age of 50, and men have a two-times higher incidence than women. The cause of IPF has not been clearly identified yet, and it is merely reported that high frequency is shown in smokers, antidepressants, chronic pulmonary inhalation due to gastroesophageal reflux, metal dust, wood dust, solvent inhalation, etc., are regarded as risk factors related with IPF occurrence. However, definitive causal factors cannot be found in the majority of patients.

It is known that IPF is continuously worsen with no treatment, and about 50% of patients die within 3-5 years, and once a lung is completely hardened by fibrosis as the disease progresses, there is no improvement despite any treatment, and accordingly, it is predicted that an early treatment increases efficacy. As the currently used therapeutic agent, a combination therapy method using steroid and azathioprine or cyclophosphamide, has been known, but it is difficult to say that there are special effects, and attempts of several fibrosis inhibitors in animal experiments and small group of patients failed in proving clear effects. In particular, there is no other effective treatment in patients with end-stage IPF, in addition to lung transplantation. Therefore, development of more effective therapeutic agent against IPF is desperately required.

Meanwhile, COPD, one of representative lung diseases together with asthma, is different from asthma in that it is accompanied by irreversible airway obstruction, and is a respiratory disease which is accompanied by abnormal inflammatory response of lung caused by repeated infection, harmful particles, gas inlet or smoking, and is not fully reversible, but shows increasingly progressed airflow limitation (Pauwels et al, Am J Respir Crit Care Med, 163:1256-1276, 2001). COPD is a disease caused by pathological changes of bronchioles and lung parenchyma by airway and lung parenchyma inflammation, and is characterized by obstructive bronchiolitis and emphysema (destruction of lung parenchyma). Types of COPD include chronic obstructive bronchitis, chronic bronchiolitis and emphysema. In COPD, the number of neutrophils is increased, and secretion of cytokines such as GM-CSF, TNF-α, IL-8, and MIP-2 is increased. Further, airway inflammation, a thickened muscle wall, and bronchial closure due to increased mucus secretion are also shown. When bronchus is closed, alveoli are expanded and damaged, such that an exchange ability of oxygen and carbon dioxide is damaged to increase respiratory failure.

The severity of COPD is emerging around the world since it is predicted that COPD ranked 6^{th} in 1990 among causes of death due to disease, but will rank in third place in 2020, and is the only disease of which the incidence is increased in 10 diseases. COPD has a high prevalence rate, causes a respiratory disorder, and requires a large amount of direct medical costs required for diagnosis and treatment of COPD, and a significant amount of indirect expenses such as losses due to dyspnea and leave of absence or loss due to premature death, which is a social and economic problem in the world (Chronic obstructive pulmonary disease (COPD) treatment guidelines 2005. Chronic obstructive airway disease Clinical Research Center. p.30-31).

Existing therapeutic drugs have not been confirmed as relieving reduction in long time lung function which is a characteristic of COPD. Accordingly, drug treatment in COPD has a main purpose of reducing symptoms or complications. Among the therapeutic drugs, a bronchodilator is a typical COPD allopathic drug, and an anti-inflammatory drug or corticosteroid is usually prescribed, but the effect is not significant, the application range is narrow, and there is great concern for side effects. As other drugs, it is known that only the influenza vaccine reduces serious symptoms and death by about 50% in patients with COPD (chronic obstructive pulmonary disease (COPD) treatment guidelines 2005. Chronic obstructive airway disease Clinical Research Center. p.52-58).

Meanwhile, many genetic factors are estimated as increasing (or decreasing) the risk of individual COPD. A genetic risk factor which has been demonstrated so far is genetic deficiency of α1-antitrypsin. Smoking significantly increases the risk of COPD, but occurrence of panlobular emphysema and reduction in lung function that rapidly progress at a young age are shown by both of smokers and non-smokers having significant genetic deficiency. Although other genes confirmed to be related with the COPD pathogenesis do not exist yet in addition to α1-antitrypsin, attempt for identifying biomarkers of the disease to be utilized for diagnosis through research into cellular, molecular, and genetic abnormal states that are basically shown in patients with COPD or attempt for finding a new treatment method is being progressed (P.J. Barnes and R.A. Stockely. COPD: current therapeutic interventions and future approaches. Eur Respir J. (2005) 25:1084-1106) . In particular, research into diagnosis of COPD and selection of target for treatment through methods such as gene microarray, proteomics, etc., has been actively conducted, and analyses of genetic factors for obtaining COPD sensitivity (susceptibility) and causes of the worsen COPD symptoms induced by smoking have been mainly conducted (Peter J. Castaldi et al. The COPD genetic association compendium. Human Molecular Genetics, 2010, Vol. 19, No. 3 526-534) .

CTGF (connective tissue growth factor; CCN2), which is one of matricellular proteins included in CCN family, is known to be a secretion cytokine involved in various biological processes such as cell adhesion, migration, proliferation, angiogenesis, wound repair, etc. Over-expression of CTGF is regarded as being main cause of symptoms such as scleroderma, fibrotic disease, and scar formation (Brigstock DR. Connective tissue growth factor (CCN2, CTGF) and organ fibrosis: lessons from transgenic animals. J Cell Commun Signal (2010) 4 (1): 1-4).In particular, regarding fibrosis, CTGF is known to cause sustained fibrosis together with TGF-β (Transforming growth factor-β) or to promote production of ECM (extracelluar matrix) under a condition in which fiber formation is caused, and recently, it is known to be capable of treating ocular disorders or muscular dystrophy caused by abnormal expression of CTGF by treating samples or materials that hinder expression of CTGF or inhibiting action thereof, but relevancy with a respiratory disease has not been suggested (U.S. Patent No. 7,622,454, and U.S Patent Application Publication No. 20120164151).

CYR61 (Cysteine-rich angiogenic inducer 61) is an extracelluar matrix (ECM)-related signaling protein included in CCN family, which is known to control various cellular activities such as cell adhesion, migration, proliferation, differentiation, apoptosis, etc. The purified CYR61 promotes attachment and spreading of endothelial cells in a similar manner to fibronectin, and does not have mitogenic activity, but acts to reinforce a mitogen effect of a fibroblast growth factor (MARIA L. KIREEVA et al. Cyr61, a Product of a Growth Factor-Inducible Immediate-Early Gene, Promotes Cell Proliferation, Migration, and Adhesion. MOLECULAR AND CELLULAR BIOLOGY, Apr. 1996, p. 1326-1334).

It is reported that Plekho1 (Pleckstrin homology domain-containing family 0 member 1) is present in a plasma membrane or nucleus, acts as a non-enzymatic regulator of protein kinase CK2α1(Casein kinase 2, alpha 1), and is involved in apoptosis by inhibition of AP-1 action through C-terminal piece produced when being decomposed by Caspase 3 (Denis G. Bosc et al. Identification and Characterization of CKIP-1, a Novel Pleckstrin Homology Domain-containing Protein That Interacts with Protein Kinase CK2. The Journal of Biological Chemistry(2000) 275, 14295-14306; Lingqiang Zhang et al. Role for the pleckstrin homology domain containing protein CKIP-1 in AP-1 regulation and apoptosis. The EMBO Journal (2005) 24, 766-778).

As described above, prevalence of the respiratory diseases, particularly, idiopathic pulmonary fibrosis and COPD has increased, but therapeutic agents being capable of basically preventing and treating the idiopathic pulmonary fibrosis and COPD do not exist yet. Therefore, the demand for the therapeutic agent for the idiopathic pulmonary fibrosis and COPD showing high level of prevention and treatment effects without side effects is significantly largely present in the market.

### DISCLOSURE OF INVENTION

An object of the present invention is to provide a novel siRNA specific to CTGF, Cyr61 or Plekho1 (hereinafter, referred to as a CTGF, Cyr61 or Plekho1-specific siRNA), capable of inhibiting expression thereof at a significantly high efficiency, a double helical-oligo RNA structure containing the siRNA, and a method for producing the double-helical oligo RNA structure.

Another object of the present invention is to provide a pharmaceutical composition including the CTGF, Cyr61 or Plekho1-specific siRNA or the double-helical oligo RNA structure containing the siRNA as an effective component, for preventing or treating respiratory diseases, particularly, idiopathic pulmonary fibrosis and COPD.

Another object of the present invention is to provide a method for preventing or treating respiratory diseases, particularly, idiopathic pulmonary fibrosis and COPD, by using the CTGF, Cyr61 or Plekho1-specific siRNA or the double-helical oligo RNA structure containing the siRNA.

### BRIEF Description of THE Drawings

FIG. 1 is a schematic diagram of a nanoparticle formed of a double-helical oligo polymer structure according to the present invention.
FIG. 2 is a graph showing an inhibition amount of target gene expression, confirmed after transforming a human fibroblast cell line with siRNA having sequence of SEQ ID NOs: 1 to 10, 101 to 110, and 201 to 210 as a sense strand according to the present invention.
   A : Graph showing CTGF expression amount according to treatment of 5 or 20 nM of siRNA having sequence of SEQ ID NOs: 1 to 10 as a sense strand.
   B : Graph showing Cyr61 expression amount according to treatment of 5 or 20 nM of siRNA having sequence of SEQ ID NOs: 101 to 110 as a sense strand.
   C : Graph showing Plekho1 expression amount according to treatment of 5 or 20 nM of siRNA having sequence of SEQ ID NOs: 201 to 210 as a sense strand.

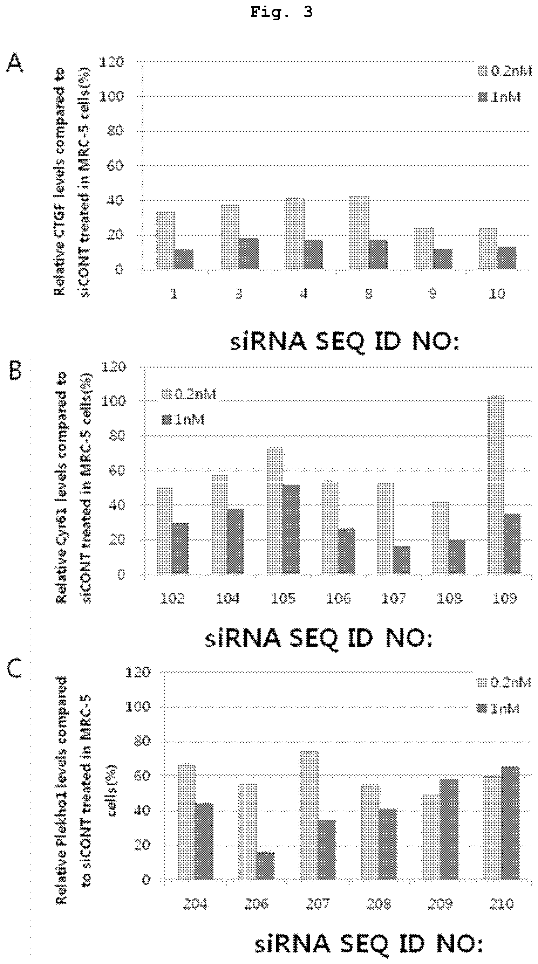

FIG. 3 is a graph showing an inhibition amount of target gene expression, confirmed after transforming a human fibroblast cell line with siRNA having sequence of SEQ ID NO: 1, 3, 4, 8, 9, 10, 102, 104, 105, 106, 107, 108, 109, 204, 206, 207, 208, 209 or 210 as a sense strand according to the present invention.
   A : Graph showing CTGF expression amount according to treatment of 0.2 or 1 nM of siRNA having sequence of SEQ ID NO: 1, 3, 4, 8, 9 or 10 as a sense strand.
   B : Graph showing Cyr61 expression amount according to treatment of 0.2 or 1 nM of siRNA having sequence of SEQ ID NO: 102, 104, 105, 106, 107, 108 or 109 as a sense strand.
   C : Graph showing Plekho1 expression amount according to treatment of 0.2 or 1 nM of siRNA having sequence of SEQ ID NO: 204, 206, 207, 208, 209, or 210 as a sense strand.

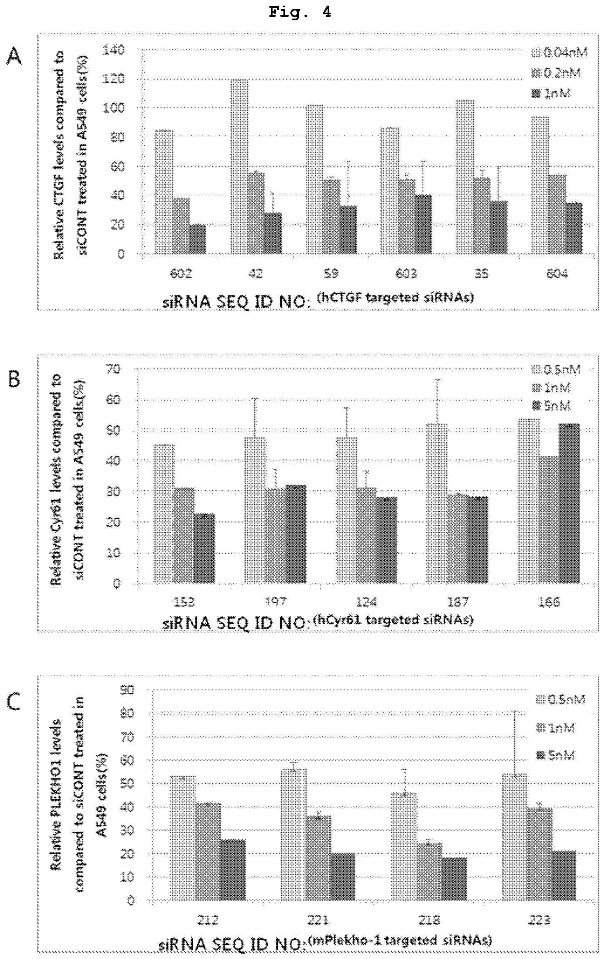

FIG. 4 is a graph showing an inhibition amount of target gene expression, confirmed after transforming a human lung cancer cell line with siRNA having sequence of SEQ ID NO: 35, 42, 59, 602, 603, 604, 124, 153, 166, 187, 197, 212, 218, 221 or 223 as a sense strand according to the present invention.
   A : Graph showing CTGF expression amount according to treatment of 0.04, 0.2 or 1 nM of siRNA having sequence of SEQ ID NO: 35, 42, 59, 602, 603, or 604 as a sense strand.
   B : Graph showing Cyr61 expression amount according to treatment of 0.5, 1 or 5 nM of siRNA having sequence of SEQ ID NO: 124, 153, 166, 187, or 197 as a sense strand.
   C : Graph showing Plekho1 expression amount according to treatment of 0.5, 1 or 5 nM of siRNA having sequence of SEQ ID NO: 212, 218, 221 or 223 as a sense strand.

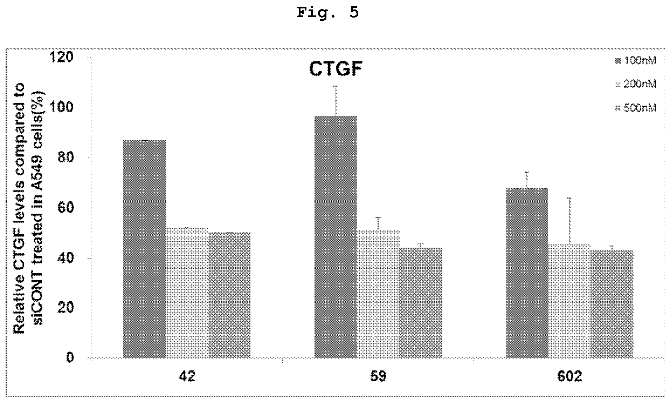

FIG. 5 is a graph showing an inhibition amount of target gene expression, confirmed after transforming a human lung cancer cell line with SAMiRNA having sequence of SEQ ID NO: 42, 59, or 602 as a sense strand according to the present invention.

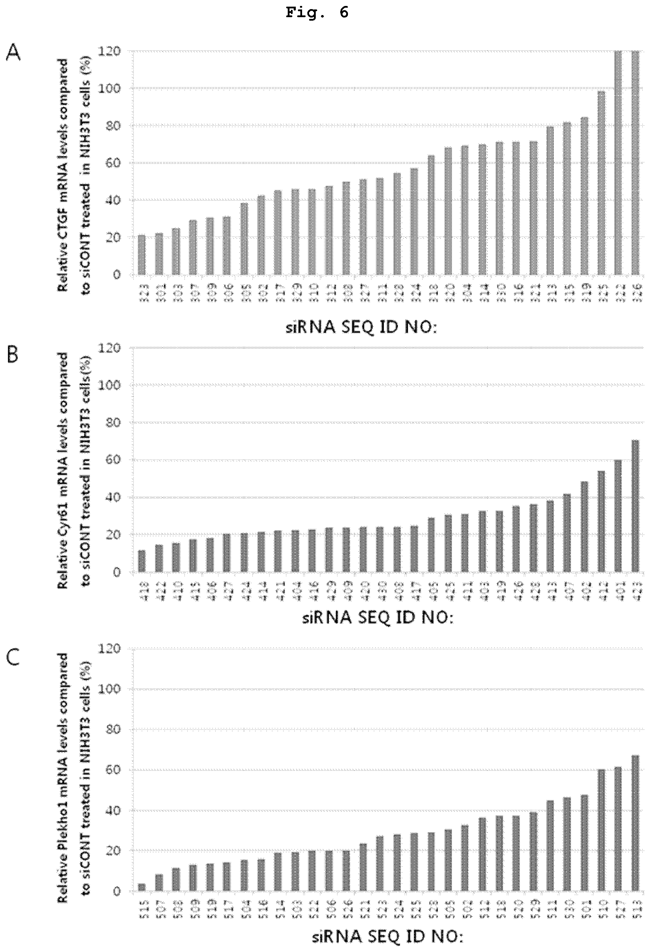

FIG. 6 is a graph showing an inhibition amount of target gene expression, confirmed after transforming a mouse fibroblast cell line with siRNA having sequence of SEQ ID NOs: 301 to 330, 401 to 430, 501 to 530 as a sense strand according to the present invention.
   A : Graph showing CTGF expression amount according to treatment of 20 nM of siRNA having sequence of SEQ ID NOs: 301 to 330 as a sense strand.
   B : Graph showing Cyr61 expression amount according to treatment of 20 nM of siRNA having sequence of SEQ ID NOs: 401 to 430 as a sense strand.
   C : Graph showing Plekho1 expression amount according to treatment of 20 nM of siRNA having sequence of SEQ ID NOs: 501 to 530 as a sense strand.

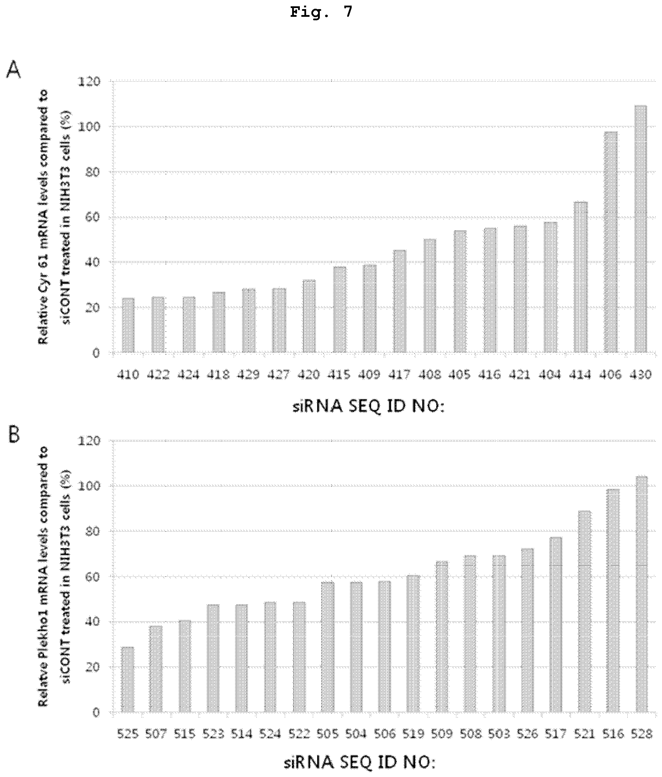

FIG. 7 is a graph showing an inhibition amount of target gene expression, confirmed after transforming a mouse fibroblast cell line with siRNA having sequence of SEQ ID NO: 404 to 406, 408 to 410, 414 to 418, 420 to 422, 424, 427, 429, 430, 503 to 509, 514 to 517, 519, 521 to 526 or 528 as a sense strand according to the present invention.
   A: Graph showing Cyr61 expression amount according to treatment of 5 nM of siRNA having sequence of SEQ ID NOs: 404 to 406, 408 to 410, 414 to 418, 420 to 422, 424, 427, 429, or 430 as a sense strand.
   B : Graph showing Plekho1 expression amount according to treatment of 5 nM of siRNA having sequence of SEQ ID NO: 503 to 509, 514 to 517, 519, 521 to 526 or 528 as a sense strand.

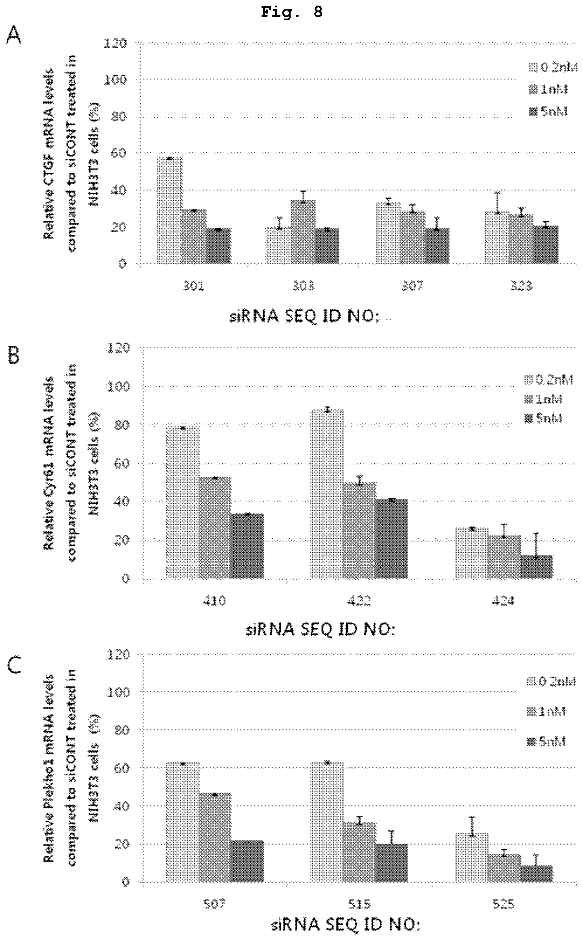

FIG. 8 is a graph showing an inhibition amount of target gene expression, confirmed after transforming a mouse fibroblast cell line with siRNA having sequence of SEQ ID NO: 301, 303, 307, 323, 410, 422, 424, 507, 515 or 525 as a sense strand according to the present invention.
   A : Graph showing CTGF expression amount according to treatment of 0.2, 1, or 5 nM of siRNA having sequence of SEQ ID NO: 301, 303, 307 or 323 as a sense strand.
   B : Graph showing Cyr61 expression amount according to treatment of 0.2, 1, or 5 nM of siRNA having sequence of SEQ ID NO: 410, 422 or 424 as a sense strand.
   C : Graph showing Plekho1 expression amount according to treatment of 0.2, 1 or 5 nM of siRNA having sequence of SEQ ID NO: 507, 515 or 525 as a sense strand.

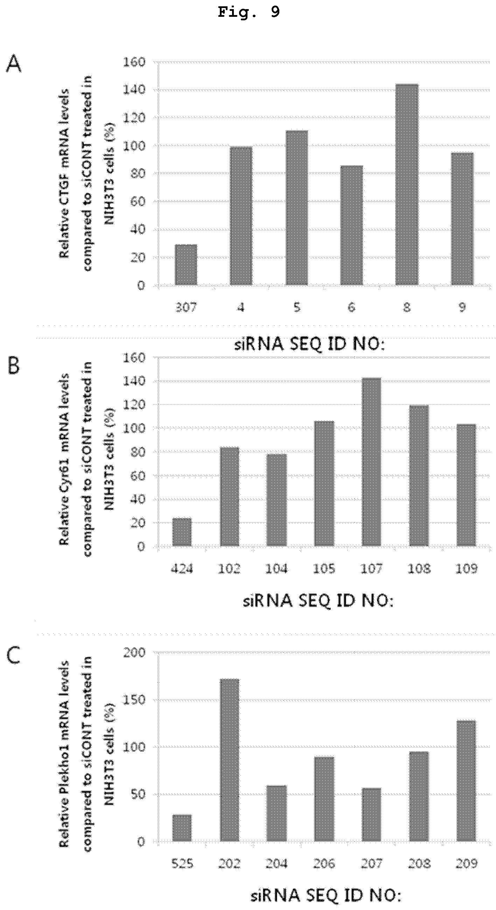

FIG. 9 is a graph showing an inhibition amount of target gene expression, confirmed after transforming a mouse fibroblast cell line with siRNA having a SEQ ID NO: 4, 5, 6, 8, 9, 102, 104, 105, 107, 108, 109, 202, 204, 206 to 209, 307, 424 or 525 as a sense strand according to the present invention.
   A : Graph showing CTGF expression amount according to treatment of 5 nM of siRNA having sequence of SEQ ID NO: 4, 5, 6, 8, 9 or 307 as a sense strand.
   B : Graph showing Cyr61 expression amount according to treatment of 5 nM of siRNA having sequence of SEQ ID NO: 102, 104, 105, 107, 108, 109 or 424 as a sense strand.
   C : Graph showing Plekho1 expression amount according to treatment of 5 nM of siRNA having sequence of SEQ ID NO: 202, 204, 206 to 209 or 525 as a sense strand.

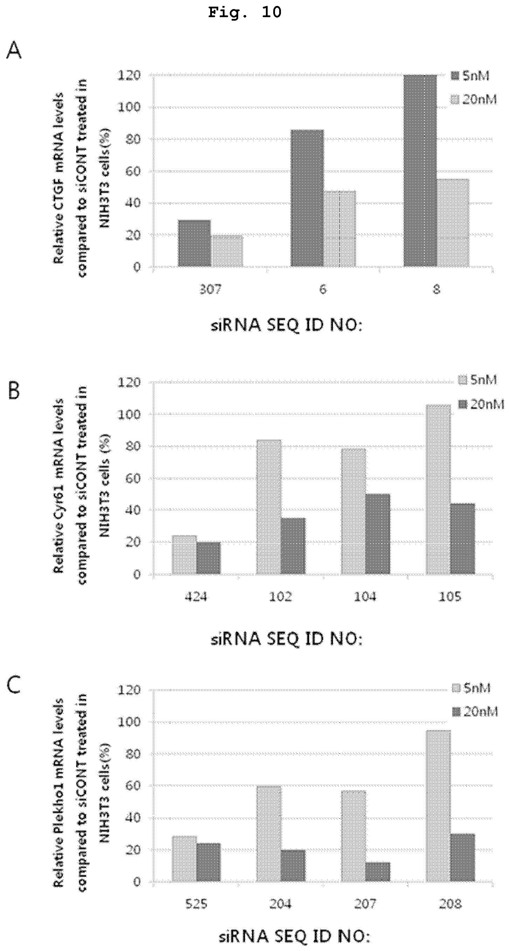

FIG. 10 is a graph showing an inhibition amount of target gene expression, confirmed after transforming a mouse fibroblast cell line with siRNA having sequence of SEQ ID NO: 6, 8, 102, 104, 105, 204, 207, 208, 307, 424 or 525 as a sense strand according to the present invention.
   A : Graph showing CTGF expression amount according to treatment of 5 or 20 nM of siRNA having sequence of SEQ ID NO: 6, 8 or 307 as a sense strand.
   B : Graph showing Cyr61 expression amount according to treatment of 5 or 20 nM of siRNA having sequence of SEQ ID NO: 102, 104, 105 or 424 as a sense strand.
   C : Graph showing Plekho1 expression amount according to treatment of 5 or 20 nM of siRNA having sequence of SEQ ID NO: 204, 207, 208 or 525 as a sense strand.

### BEST MODE FOR CARRYING OUT THE INVENTION

In order to achieve the foregoing objects, the present invention provides a CTGF, Cyr61 or Plekho1 (respiratory diseases-related gene)-specific siRNA consisting of first oligonucleotide which is a sense strand including any one sequence selected from the group consisting of SEQ ID NOs: 1 to 600 and 602 to 604 and a second oligonucleotide which is an antisense strand including a complementary sequence thereto.

The siRNA in the present invention includes all materials having a general RNAi (RNA interference) action, and accordingly, it is obvious to a person skilled in the art that CTGF, Cyr61 or Plekho1-specific siRNA includes CTGF, Cyr61 or Plekho1-specific shRNA, etc.

SEQ ID NOs: 1 to 100, or 602 to 604 are sense strand sequences of CTGF (Homo sapiens)-specific siRNA, SEQ ID NOs: 101 to 200 are sense strand sequences of Cyr61 (Homo sapiens)-specific siRNA, SEQ ID NOs: 201 to 300 are sense strand sequences of Plekho1 (Homo sapiens)-specific siRNA, SEQ ID NOs: 301 to 400 are sense strand sequences of CTGF (Mus musculus)-specific siRNA, SEQ ID NOs: 401 to 500 are sense strand sequences of *Cyr61*(*Mus musculus*)-specific siRNA, and SEQ ID NOs: 501 to 600 are sense strand sequences of Plekho1(*Mus musculus*)-specific siRNA.

The siRNA according to the present invention is preferably CTGF-specific siRNA including any one sequence selected from the group consisting of SEQ ID NOs: 1 to 10, 35, 42, 59, 602, 603, 604, 301 to 303, 305 to 307, 309, 317, 323 and 329, as a sense strand,

Cyr61-specific siRNA including any one sequence selected from the group consisting of SEQ ID NOs: 101 to 110, 124, 153, 166, 187, 197, 409, 410, 415, 417, 418, 420, 422, 424, 427 and 429, as a sense strand, or

Plekho1-specific siRNA including any one sequence selected from the group consisting of SEQ ID NOs: 201 to 210, 212, 218, 221, 223, 504 to 507, 514, 515 and 522 to 525, as a sense strand.

More preferably, the siRNA according to the present invention is CTGF-specific siRNA including any one sequence selected from the group consisting of SEQ ID NOs: 4, 5, 8, 9, 35, 42, 59, 601, 602, 604, 301, 303, 307 and 323, as a sense strand,

Cyr61-specific siRNA including any one sequence selected from the group consisting of SEQ ID NOs: 102, 104, 107, 108, 124, 153, 166, 187, 197, 410, 422 and 424, as a sense strand, or

Plekho1-specific siRNA including any one sequence selected from the group consisting of SEQ ID NOs: 206 to 209, 212, 218, 221, 223, 507, 515 and 525, as a sense strand.

Most preferably, the siRNA according to the present invention is CTGF-specific siRNA including any one sequence selected from the group consisting of SEQ ID NO: 42, 59, 602 and 323, as a sense strand,

Cry61-specific siRNA including any one sequence selected from the group consisting of SEQ ID NO: 124, 153, 187, 197 and 424, as a sense strand, or Plekho1-specific siRNA including any one sequence selected from the group consisting of SEQ ID NO: 212, 218, 221, 223 and 525, as a sense strand.

In particular, it was confirmed that some of human CTGF, Cyr61 or Plekho1-specific siRNA according to the present invention is capable of simultaneously inhibiting expression of mouse CTGF, Cyr61 or Plekho1.

The siRNA capable of simultaneously inhibiting the expression of human and mouse CTGF, Cyr61 or Plekho1 preferably includes a sense strand of CTGF-specific siRNA according to SEQ ID NO: 6 or 8, a sense strand of Cyr61-specific siRNA according to SEQ ID NO: 102, 104 or 105, or a sense strand of Plekho1-specific siRNA according to SEQ ID NO: 204, 207 or 208.

Most preferably, the siRNA includes a sense strand of CTGF-specific siRNA according to SEQ ID NO: 6, a sense strand of Cyr61-specific siRNA according to SEQ ID NO: 102, or a sense strand of Plekho1-specific siRNA according to SEQ ID NO: 207.

The sense strand or the antisense strand of the siRNA according to the present invention preferably has 19 to 31 nucleotides, and the siRNA includes a sense strand including any one sequence selected from SEQ ID NOs: 1 to 604 and an antisense strand complementary thereto.

Since the CTGF, Cyr61 or Plekho1-specific siRNA according to the present invention has a base sequence designed to be capable of being complementarily bonded to mRNA encoding the corresponding gene, expression of the corresponding gene is capable of being effectively inhibited. Further, the CTGF, Cyr61 or Plekho1-specific siRNA according to the present invention may include overhang which is a structure including one or two or more unpaired nucleotide(s) at 3' end of the siRNA, and
may include various modifications of the siRNA to provide nucleic acid lyase resistance, and to decrease non-specific immune response for improving stability in vivo. The modification of the first oligonucleotide or the second oligonucleotide configuring the siRNA may be one or more combinations selected from modification in which -OH group at 2' carbon in a sugar structure in one or more nucleotides is substituted with -CH₃(methyl), -OCH₃ (methoxy), -NH₂, -F(fluorine), -O-2-methoxyethyl, -O-propyl, -O-2-methylthioethyl, -0-3-aminopropyl, -0-3-dimethylaminopropyl, -O-N-methylacetamido or -O-dimethylamidooxyethyl; modification in which oxygen in a sugar structure in nucleotides is substituted with sulfur; and modification to phosphorothioate or boranophosphate, methyl phosphonate bindings from nucleotides bindings, or may be modification to peptide nucleic acid (PNA), modification to locked nucleic acid (LNA), or modification to unlocked nucleic acid (UNA) (Ann. Rev. Med. 55, 61-65 2004; US 5,660,985; US 5,958,691; US 6,531,584; US 5,808,023; US 6,326,358; US 6,175,001; Bioorg. Med. Chem. Lett. 14:1139-1143, 2003; RNA, 9:1034-1048, 2003; Nucleic Acid Res. 31:589-595, 2003; Nucleic Acids Research, 38(17) 5761-5773, 2010; Nucleic Acids Research, 39(5) 1823-1832, 2011) .

The CTGF, Cyr61 and/or Plekho1-specific siRNA according to the present invention may inhibit expression of the corresponding gene, and may remarkably inhibit expression of the corresponding protein.

The present invention also provides a conjugate in which a hydrophilic material and a hydrophobic material are conjugated to both ends of siRNA, for effective in vivo delivery and for improving stability, of the respiratory diseases-related gene-specific siRNA, particularly, the CTGF, Cyr61 or Plekho1-specific siRNA.

In the siRNA conjugate in which a hydrophilic material and a hydrophobic material are bonded to the siRNA, a self-assembled nanoparticle is formed by a hydrophobic interaction of the hydrophobic material (see Korean Patent Publication No. 1224828), wherein the self-assembled nanoparticle has advantages in that internal delivery efficiency and stability in vivo are significantly excellent, and uniformity of a particle size is excellent, such that quality control (QC) is easily performed, whereby a process for producing drugs is easy.

In one preferable exemplary embodiment, a double-helical oligo RNA structure containing the CTGF, Cyr61 or Plekho1-specific siRNA according to the present invention preferably has a structure represented by the following Structural Formula (1):

Structural Formula 1 A-X-R-Y-B

In Structural Formula (1), A is a hydrophilic material, B is a hydrophobic material, X and Y are each independently a simple covalent bond or a linker-mediated covalent bond, and R is CTGF, Cyr61, or Plekho1-specific siRNA.

More preferably, the double-helical oligo RNA structure containing the CTGF, Cyr61 or Plekho1-specific siRNA according to the present invention has a structure represented by the following Structural Formula (2):

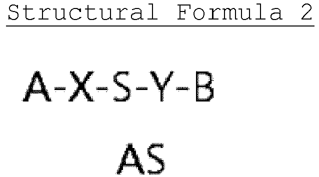

In Structural Formula (2), A, B, X and Y are the same as being defined in Structural Formula (1), S is a sense strand of the CTGF, Cyr61 or Plekho1-specific siRNA, and AS is an antisense strand of the CTGF, Cyr61 or Plekho1-specific siRNA.

More preferably, the double-helical oligo RNA structure containing the CTGF, Cyr61 or Plekho1-specific siRNA according to the present invention has a structure represented by the following Structural Formula (3) or (4):

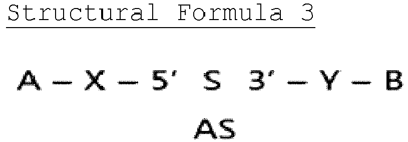

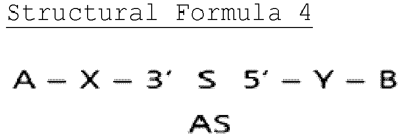

In Structural Formulas (3) and (4), A, B, S, AS, X and Y are the same as being defined in Structural Formula (1), and 5' and 3' mean 5' end and 3' end of the sense strand of the CTGF, Cyr61 or Plekho1-specific siRNA.

It is obvious to a person skilled in the art that in Structural Formulas (1) to (4), one to three phosphate group(s) may be bonded to 5' end of the antisense strand of the double-helical oligo RNA structure containing the CTGF, Cyr61 or Plekho1-specific siRNA, and shRNA is capable of being used instead of using siRNA.

The hydrophilic material in Structural Formulas (1) to (4) is preferably a cationic or non-ionic polymer material having a molecular weight of 200 to 10,000, more preferably, a non-ionic polymer material having a molecular weight of 1,000 to 2,000. For example, non-ionic hydrophilic polymer compounds such as polyethylene glycol, polyvinyl pyrrolidone, polyoxazoline, etc., are preferably used as the hydrophilic polymer material, but the present invention is not necessarily limited thereto.

In particular, the hydrophilic material (A) in Structural Formulas (1) to (4) may be used in a hydrophilic material block form represented by the following Structural Formula (5) or (6), and by using an appropriate number (n in Structural Formula (5) or (6)) of hydrophilic material blocks as needed, problems caused by polydispersibility that may occur in a case of using a general synthetic polymer material, etc., may be largely improved.

Structural Formula 5 (A'ₘ-J)ₙ

Structural Formula 6 (J-A'ₘ)ₙ

In Structural Formula (5), A' is a hydrophilic material monomer, J is a linker for connecting hydrophilic material monomers (the sum is m) therebetween or a linker for connecting hydrophilic material monomers (the sum is m) to siRNA, m is an integer of 1 to 15, n is an integer of 1 to 10, and the repeating unit represented by (A'ₘ-J) or (J-A'ₘ) corresponds to a base unit of the hydrophilic material block.

When the hydrophilic material block is represented by Structural Formula (5) or (6), the double-helical oligo RNA structure containing the CTGF, Cyr61 or Plekho1-specific siRNA according to the present invention may have a structure represented by the following Structural Formula (7) or (8) :

Structural Formula 7 (A'ₘ-J)ₙ-X-R-Y-B

Structural Formula 8 (J-A'ₘ₎ₙ-X-R-Y-B

In Structural Formulas (7) and (8), X, R, Y and B are the same as being defined in Structural Formula (1), and A', J, m and n are the same as being defined in Structural Formulas (5) and (6).

In Structural Formulas (5) and (6), the hydrophilic material monomer A' is usable without limitation as long as it meets the objects of the present invention among the monomers of the non-ionic hydrophilic polymer. The hydrophilic material monomer A' is preferably a monomer selected from the following compounds (1) to (3) shown in Table 1, more preferably, a monomer of the compound (1), and G in the compound (1) may be preferably selected from CH₂, O, S and NH.

In particular, among the hydrophilic material monomers, the monomer of Compound (1) may have various functional groups introduced thereinto and good affinity in vivo, and induce a little immune response, thereby having excellent bio-compatibility, and further, may increase stability in vivo of the oligonucleotide included in the structure of Structural Formula (7) or (8), and may increase delivery efficiency, which is significantly suitable for producing the structure according to the present invention.

**[Table 1] Monomer structure of hydrophilic material in the present invention**

| Compound (1) | Compound (2) | Compound (3) |
|---|---|---|
| 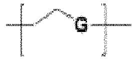 | 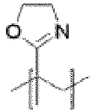 | 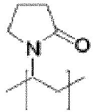 |
| G is CH₂, O, S or NH | | |

In particular, the hydrophilic material in Structural Formulas (5) to (8) preferably has total molecular weight of 1,000 to 2,000. Therefore, for example, when hexaethylene glycol represented by Compound (1), that is, G is O, and m is 6, is used in Structural Formulas (7) and (8), a molecular weight of hexaethylene glycol spacer is 344, such that the repeating number (n) is preferred to be 3 to 5. In particular, the repeating unit of the hydrophilic group, that is, hydrophilic material block, represented by (A_{'m}-J) or (J-A'ₘ)ₙ in Structural Formulas (5) and (6), is capable of being used in an appropriate number represented by n, as needed. A which is the hydrophilic material monomer and J which is the linker included in each hydrophilic material block may be independently the same as each other or be different from each other. That is, when 3 hydrophilic material blocks are used (n = 3), the hydrophilic material monomer of the compound (1) is used in the first block, the hydrophilic material monomer of the compound (2) is used in the second block, the hydrophilic material monomer of the compound (3) is used in the third block, etc. That is, different hydrophilic material monomers for all hydrophilic material blocks may be used, and any one hydrophilic material monomer selected from the hydrophilic material monomers of compounds (1) to (3) may also be equally used in all hydrophilic material blocks. Similarly, linkers mediating the combination of the hydrophilic material monomer may also be same as each other or different from each other for all hydrophilic material blocks. Further, m which is the number of hydrophilic material monomers may be the same as each other or different from each other among all hydrophilic material blocks. That is, 3 hydrophilic material monomers (m=3) are connected in the first hydrophilic material block, 5 hydrophilic material monomers (m=5) are connected in the second hydrophilic material block, 4 hydrophilic material monomers (m=4) are connected in the third hydrophilic material block, etc. That is, hydrophilic material monomers each having different numbers or each having the same number may be used in all hydrophilic material blocks.

Further, in the present invention, the linker (J) is preferably selected from the group consisting of PO₃⁻, SO₃ and CO₂, but the present invention is not limited thereto. It is obvious to a person skilled in the art that any linker may be used depending on the used hydrophilic material monomer, etc., as long as it meets the objects of the present invention.

The hydrophobic material (B) in Structural Formulas (1) to (4), (7) and (8) serves to form a nanoparticle formed of the oligonucleotide structures according to Structural Formulas (1) to (4), (7) and (8) through hydrophobic interaction. The hydrophobic material preferably has a molecular weight of 250 to 1,000, and may include a steroid derivative, a glyceride derivative, glycerol ether, polypropylene glycol, C₁₂ to C₅₀ unsaturated or saturated hydrocarbon, diacylphosphatidylcholine, fatty acid, phospholipid, lipopolyamine, etc., but the present invention is not limited thereto. It is obvious to a person skilled in the art that any hydrophobic material may be used as long as it meets the objects of the present invention.

The steroid derivative may be selected from the group consisting of cholesterol, cholestanol, cholic acid, cholesteryl formate, cholestanyl formate, and cholesteryl amine, and the glyceride derivative may be selected from mono-, di- and tri-glyceride, etc., wherein the fatty acid of the glyceride is preferred to be C₁₂ to C₅₀ unsaturated or saturated fatty acid.

In particular, among the hydrophobic materials, saturated or unsaturated hydrocarbon or cholesterol is preferred since it is capable of easily being bonded in a synthetic step of the oligonucleotide structure according to the present invention, and C₂₄ hydrocarbon, particularly, tetradocosane including a disulfide bond is the most preferred.

The hydrophobic material is bonded to the distal end of the hydrophilic material, and it does not matter if the hydrophobic material is bonded to any position of the sense strand or the antisense strand of the siRNA.

The hydrophilic material or the hydrophobic material in Structural Formulas (1) to (4), (7) and (8) according to the present invention is bonded to the CTGF, Cyr61, or Plekho1-specific siRNA by a simple covalent bond or a linker-mediated covalent bond (X or Y). In addition, the linker mediating the covalent bond is covalently bonded to the hydrophilic material or the hydrophobic material at the end of the CTGF, Cyr61 or Plekho1-specific siRNA, and is not particularly limited as long as the bond that is possible to be decomposed in a specific environment is provided as needed. Therefore, any compound for the binding to activate the CTGF, Cyr61 or Plekho1-specific siRNA and/or the hydrophilic material (or the hydrophobic material) in production of the double-helical oligo RNA structure according to the present invention, may be used as the linker. The covalent bond may be any one of a non-degradable bond or a degradable bond. Here, examples of the non-degradable bond may include an amide bond or a phosphorylation bond, and examples of the degradable bond may include a disulfide bond, an acid degradable bond, an ester bond, an anhydride bond, a biodegradable bond or an enzymatically degradable bond, and the like, but the present invention is not limited thereto.

In addition, any CTGF, Cyr61 or Plekho1-specific siRNA represented by R (or S and AS) in Structural Formulas (1) to (4), (7), and (8) is usable without limitation as long as it is siRNA capable of being specifically bonded to CTGF, Cyr61 or Plekho1. Preferably, the CTGF, Cyr61 or Plekho1-specific siRNA consists of a sense strand including any one sequence selected from SEQ ID NOs: 1 to 600 and 602 to 604 and an antisense strand including a complementary sequence thereto.

In particular, the siRNA included in Structural Formulas (1) to (4), (7) and (8) according to the present invention is preferably CTGF-specific siRNA including any one sequence selected from the group consisting of SEQ ID NOs: 1 to 10, 35, 42, 59, 602 to 604 or 301 to 303, 305 to 307, 309, 317, 323 and 329, as a sense strand,

Cyr61-specific siRNA including any one sequence selected from the group consisting of SEQ ID NOs: 101 to 110, 124, 153, 166, 187, 197, 409, 410, 415, 417, 418, 420, 422, 424, 427 and 429, as a sense strand, or

Plekho1-specific siRNA including any one sequence selected from the group consisting of SEQ ID NOs: 201 to 210, 212, 218, 221, 223, 504 to 507, 514, 515 and 522 to 525, as a sense strand.

More preferably, the siRNA according to the present invention is CTGF-specific siRNA including any one sequence selected from the group consisting of SEQ ID NOs: 4, 5, 8, 9, 35, 42, 59, 602, 603, 604, 301, 303, 307 and 323, as a sense strand,

Cyr61-specific siRNA including any one sequence selected from the group consisting of SEQ ID NOs: 102, 104, 107, 108, 124, 153, 166, 187, 197, 410, 422 and 424, as a sense strand, or

Plekho1-specific siRNA including any one sequence selected from the group consisting of SEQ ID NOs: 206 to 209, 212, 218, 221, 223, 507, 515 and 525, as a sense strand.

Most preferably, the siRNA according to the present invention is CTGF-specific siRNA including any one sequence of SEQ ID NO: 42, 59, 602 or 323, as a sense strand,

Cry61-specific siRNA including any one sequence of SEQ ID NO: 124, 153, 187, 197 or 424, as a sense strand, or

Plekho1-specific siRNA including any one sequence of SEQ ID NO: 212, 218, 221, 223 or 525, as a sense strand.

In addition, siRNA including human and mouse CTGF-specific siRNA sense strand according to SEQ ID NO: 6 or 8, siRNA including human and mouse Cyr61-specific siRNA sense strand according to SEQ ID NO: 102, 104 or 105, and siRNA including human and mouse Plekho1-specific siRNA sense strand according to SEQ ID NO: 204, 207 or 208, are particularly preferred, which is because the siRNA having the sequence as the sense strand has an effect of simultaneously inhibiting expression of human and mouse CTGF, Cyr61 or Plekho1, such that siRNA including human and mouse CTGF-specific siRNA sense strand according to SEQ ID NO: 6, siRNA including human and mouse Cyr61-specific siRNA sense strand according to SEQ ID NO: 102, and siRNA including human and mouse Plekho1-specific siRNA sense strand according to SEQ ID NO: 207 are the most preferred.

In the double helical-oligo RNA structure containing the CTGF, Cyr61 or Plekho1-specific siRNA according to the present invention, an amine group or a polyhistidine group may be additionally introduced into a portion of the distal end bonded with the siRNA of the hydrophilic material in the structure.

This makes it easy to perform intercellular introduction and escape from the endosome of the double helical-oligo RNA structure containing the CTGF, Cyr61 or Plekho1-specific siRNA according to the present invention, and a possibility of introducing an amine group and using a polyhistidine group to easily perform the intercellular introduction and the escape from the endosome of the carriers such as Quantum dot, Dendrimer, liposome, etc., and the effect thereof, have been reported.

Specifically, it is known that the primary amine group expressed at the end or the outside of the carrier is protonated in vivo pH to form a conjugate with a negatively charged gene by an electrostatic interaction, and the escape from the endosome is easily performed due to internal tertiary amines having a buffer effect at a low pH of the endosome after being introduced into the cells, thereby being capable of protecting the carrier from decomposition of lysosome (gene delivery and expression inhibition using a polymer-based hybrid material. Polymer Sci. Technol., Vol. 23, No.3, pp254-259).

In addition, it is known that histidine which is one of non-essential amino acids, has an imidazole ring (pKa3 6.04) at a residue (-R) to increase a buffer capacity in the endosome and the lysosome, such that histidine expression may be used to increase an escape efficiency from the endosome in non-viral gene carriers including liposome (Novel histidine-conjugated galactosylated cationic liposomes for efficient hepatocyte selective gene transfer in human hepatoma HepG2 cells. J. Controlled Release 118, pp262-270).

The amine group or the polyhistidine group may be linked to the hydrophilic material or the hydrophilic material block through at least one linker.

When the amine group or the polyhistidine group is introduced into the hydrophilic material of the double-helical oligo RNA structure represented by Structural Formula (1), the double helical-oligo RNA structure may have a structure represented by Structural Formula (9) below:

Structural Formula 9 P-J₁-J₂-A-X-R-Y-B

In Structural Formula (9), A, B, R, X and Y are the same as being defined in Structural Formula (1),
P is an amine group or a polyhistidine group. J₁ and J₂ are linkers and may be each independently selected from a simple covalent bond, PO₃⁻, SO₃, CO₂, C₂₋₁₂ alkyl, alkenyl, and alkynyl, but the present invention is not limited thereto. It is obvious to a person skilled in the art that any linker is usable as J₁ and J₂ as long as it meets the objects of the present invention depending on the used hydrophilic material.

Preferably, when the amine group is introduced, J₂ is preferably a simple covalent bond or PO₃⁻, and J₁ is preferably C₆ alkyl, but the present invention is not limited thereto.

Further, when the polyhistidine group is introduced, in Structural Formula (9), J₂ is preferably a simple covalent bond or PO₃⁻, and J₁ is preferably the following Compound (4), but the present invention is not limited thereto.

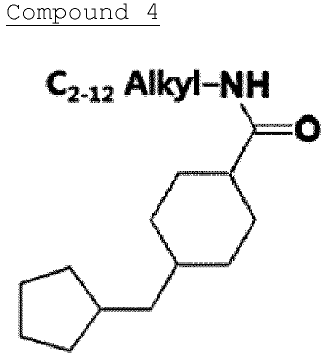

Further, when the hydrophilic material of the double-helical oligo RNA structure represented by Structural Formula (9) is a hydrophilic material block represented by Structural Formula (5) or (6), and the amine group or the polyhistidine group is introduced, the double-helical oligo RNA structure may be represented by Structural Formula (10) or (11):

Structural Formula 10 P-J₁-J₂-(A'ₘ-J)ₙ-X-R-Y-B

Structural Formula 11 P-J₁-J₂-(J-A'ₘ)ₙ-X-R-Y-B

In Structural Formulas (10) and (11), X, R, Y, B, A', J, m and n are the same as being defined in Structural Formula (5) or (6), and P, J1 and J2 are the same as being defined in Structural Formula (9).

In particular, in Structural Formulas (10) and (11), the hydrophilic material is preferably bonded to 3' end of the sense strand of the CTGF, Cyr61 or Plekho1-specific siRNA, and in this case, Structural Formulas (9) to (11) may be represented by the following Structural Formulas (12) to (14): 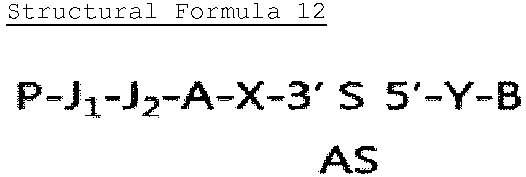 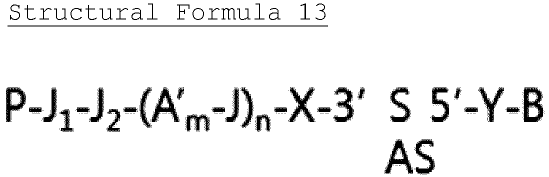 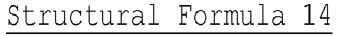 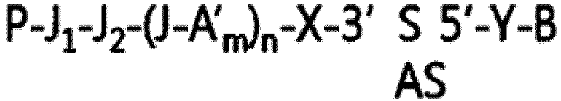 In Structural Formulas (12) to (14), X, R, Y, B, A, A' J, m, n, P, J₁ and J₂ are the same as being defined in Structural Formulas (9) to (11), and 5' and 3' mean 5' end and 3' end of the sense strand of the CTGF, Cyr61 or Plekho1-specific siRNA.

The amine group that may be introduced in the present invention may be primary to tertiary amine groups, and the primary amine group is particularly preferred. The introduced amine group may be present as an amine salt. For example, the salt of the primary amine group may be present in a form of NH₃₊.

Further, the polyhistidine group that may be introduced in the present invention preferably includes 3 to 10 histidines, more preferably, 5 to 8 histidines, and the most preferably, 6 histidines. In addition to histidine, at least one cystein may be additionally included.

Meanwhile, when a targeting moiety is provided in the double-helical oligo RNA structure containing the CTGF, Cyr61, or Plekho1-specific siRNA according to the present invention and the nanoparticle formed therefrom, delivery to the target cell is effectively promoted to achieve delivery to the target cell even in a relatively low concentration of dosage, thereby showing a high control function for target gene expression, and thereby preventing non-specific delivery of the CTGF, Cyr61, or Plekho1-specific siRNA into other organs and cells.

Accordingly, the present invention provides a double-helical oligo RNA structure in which a ligand (L), particularly, a ligand specifically bonded to a receptor that promotes target cell internalization through receptor-mediated endocytosis (RME), is additionally bonded to the structure according to Structural Formulas (1) to (4), (7) and (8). For example, the form in which the ligand is bonded to the double-helical oligo RNA structure according to Structural Formula (1), has a structure represented by the following Structural Formula (15):

Structural Formula 15 (Lᵢ-Z)-A-X-R-Y-B

In Structural Formula (15), A, B, X and Y are the same as being defined in Structural Formula (1), L is a ligand specifically bonded to a receptor that promotes target cell internalization through receptor-mediated endocytosis (RME), and i represents an integer from 1 to 5, preferably, an integer of 1 to 3.

The ligand in Structural Formula (15) may be preferably selected from the group consisting of a target receptor-specific antibody or aptamer, peptide that has properties of RME for promoting cell internalization in a target cell-specific manner; or folate (generally, folate and folic acid are intersectionally used, and the folate in the present invention means folate in a natural state or in an activated state in a human body), chemicals such as sugar, carbohydrate, etc., including hexoamines such as N-acetyl galactosamine (NAG), etc., glucose, mannose, but the present invention is not limited thereto.

Further, the hydrophilic material A in Structural Formula (15) may be used in the form of the hydrophilic material block represented by Structural Formulas (5) and (6) .

The present invention also provides a method for producing the double-helical oligo RNA structure containing the CTGF, Cyr61, or Plekho1-specific siRNA.

The method for producing the double-helical oligo RNA structure containing the CTGF, Cyr61, or Plekho1-specific siRNA according to the present invention may include the following steps:
(1) binding a hydrophilic material based on a solid support;
(2) synthesizing an RNA single strand based on the solid support containing the hydrophilic material bonded thereto;
(3) covalently binding a hydrophobic material to 5' end of the RNA single strand;
(4) synthesizing an RNA single strand having a complementary sequence to a sequence of the RNA single strand;
(5) separating and purifying an RNA-polymer structure and the RNA single strand from the solid support when the synthesizing of the RNA single strand is completed; and
(6) producing the double-helical oligo RNA structure by annealing the RNA-polymer structure and an RNA single strand having a complementary sequence thereto.

The solid support in the present invention is preferably a controlled pore glass (CPG), but the present invention is not limited thereto, but may be polystyrene, silica gel, cellulose paper, etc. In the CPG, a diameter is preferably 40 to 180 *µ*m, and a pore size is preferably 500 to 3000Å. After step (5) above, when the production is completed, it may be confirmed whether the purified RNA-polymer structure and the purified RNA single strand are produced as the desired RNA-polymer structure and the desired RNA single strand by measuring molecular weights by MALDI-TOF mass spectrometer. In the production method, Step (4) which is a step of synthesizing the RNA single strand having a complementary sequence to a sequence of the RNA single strand synthesized in Step (2) may be performed before Step (1) or may be performed during any one step of Steps (1) to (5).

In addition, the RNA single strand having a complementary sequence to the RNA single strand synthesized in Step (2) may contain a phosphate group bonded to 5' end thereof.

Meanwhile, the present invention provides a method for producing a double-helical oligo RNA structure in which a ligand is additionally bonded to the double-helical oligo RNA structure containing the CTGF, Cyr61, or Plekho1-specific siRNA.

The method for producing the double-helical oligo RNA structure containing the ligand-bonded CTGF, Cyr61, or Plekho1-specific siRNA according to the present invention may include the following steps:
(1) binding a hydrophilic material to a solid support containing a functional group bonded thereto;
(2) synthesizing an RNA single strand based on the solid support containing a functional group-hydrophilic material bonded thereto;
(3) covalently binding a hydrophilic material to 5' end of the RNA single strand;
(4) synthesizing an RNA single strand having a complementary sequence to a sequence of the RNA single strand;
(5) separating a functional group-RNA-polymer structure and the RNA single strand having the complementary sequence from the solid support when the synthesizing of the RNA single strand is completed;
(6) producing a ligand-RNA-polymer structure single strand by binding a ligand to an end of the hydrophilic material using the functional group; and
(7) producing a ligand-double-helical oligo RNA structure by annealing the ligand-RNA-polymer structure and an RNA single strand having a complementary sequence thereto.

After step (6) above, when the production is completed, whether the desired ligand-double-helical oligo RNA structure and the desired RNA single strand having a complementary sequence thereto are prepared may be confirmed by separating and purifying the ligand-RNA-polymer structure and the RNA single strand having a complementary sequence thereto, and measuring molecular weights by MALDI-TOF mass spectrometer. The ligand-double-helical oligo RNA structure may be produced by annealing the prepared ligand-RNA-polymer structure and the RNA single strand having a complementary sequence thereto. In the production method, Step (4) which is a step of synthesizing the RNA single strand having a complementary sequence to a sequence of the RNA single strand synthesized in Step (3), is an independent synthesis process, and may be performed before Step (1) or may be performed during any one step of Steps (1) to (6).

The present invention also provides a nanoparticle including the double-helical oligo RNA structure containing the CTGF, Cyr61, or Plekho1-specific siRNA.

As described above, the double-helical oligo RNA structure containing the CTGF, Cyr61, or Plekho1-specific siRNA is amphipathic structure containing both of hydrophobic materials and hydrophilic materials, wherein the hydrophilic materials have affinity through an interaction such as a hydrogen bond, etc., with water molecules present in the body to face toward the outside, and the hydrophobic materials face toward the inside through a hydrophobic interaction therebetween, thereby forming a thermodynamically stable nanoparticle. That is, the hydrophobic materials are positioned in the center of the nanoparticle, and the hydrophilic materials are positioned in the outside direction of the CTGF, Cyr61, or Plekho1-specific siRNA to form nanoparticles protecting the CTGF, Cyr61, or Plekho1-specific siRNA. These formed nanoparticles improve intracellular delivery of the CTGF, Cyr61, and/or Plekho1-specific siRNA and improve siRNA effect.

The nanoparticles according to the present invention may be formed of only the double-helical oligo RNA structure containing siRNAs each having the same sequence as each other, or may be formed of the double-helical oligo RNA structure containing siRNAs each having different sequence, wherein it is construed in the present invention that the siRNAs each having different sequence includes siRNA having different target genes, for example, CTGF, Cyr61, or Plekho1-specific siRNA, or siRNA having the same target gene-specificity, but having different sequence.

Further, the double-helical oligo RNA structure containing other respiratory diseases-related gene-specific siRNA in addition to the CTGF, Cyr61, or Plekho1-specific siRNA may also be included in the nanoparticle according to the present invention.

Further, the present invention provides a composition for preventing or treating respiratory diseases, particularly, idiopathic pulmonary fibrosis and COPD, including: the CTGF, Cyr61, or Plekho1-specific siRNA, the double-helical oligo RNA structure containing the siRNA, and/or the nanoparticle formed of the double-helical oligo RNA structure.

The composition including the CTGF, Cyr61, or Plekho1-specific siRNA according to the present invention, the double-helical oligo RNA structure containing the siRNA, and/or the nanoparticle formed of the double-helical oligo RNA structure as effective components, inhibits pulmonary artery remodeling and airway remodeling, such that the CTGF, Cyr61, or Plekho1-specific siRNA or the composition containing the siRNA has an effect of preventing or treating the respiratory diseases.

In particular, the composition for preventing or treating respiratory diseases, including the double-helical oligo RNA structure according to the present invention may include:
a double-helical oligo RNA structure including a CTGF-specific siRNA that includes a sense strand including any one sequence selected from the group consisting of SEQ ID NOs: 1 to 100 or 602 to 604 and 301 to 400, preferably, any one sequence selected from the group consisting of SEQ ID NOs: 1 to 10, 35, 42, 59, 602 to 604, 301 to 303, 305 to 307, 309, 317, 323 and 329, more preferably, any one sequence selected from the group consisting of SEQ ID NOs: 4, 5, 8, 9, 35, 42, 59, 602 to 604, 301, 303, 307 and 323, the most preferably, any one sequence of SEQ ID NO: 42, 59, 602 or 323, and an antisense strand including a complementary sequence thereto;
a double-helical oligo RNA structure including a Cyr61-specific siRNA that includes a sense strand including any one sequence selected from the group consisting of SEQ ID NOs: 101 to 200 and 401 to 500, preferably, any one sequence selected from the group consisting of SEQ ID NOs: 101 to 110, 124, 153, 166, 187, 197, 409, 410, 415, 417, 418, 420, 422, 424, 427 and 429, more preferably, any one sequence selected from the group consisting of SEQ ID NOs: 102, 104, 107, 108, 124, 153, 166, 187, 197, 410, 422 and 424, the most preferably, any one sequence of SEQ ID NO: 124, 153, 187, 197 or 424, and an antisense strand including a complementary sequence thereto; or
a double-helical oligo RNA structure including a Plekho1-specific siRNA that includes a sense strand including any one sequence selected from the group consisting of SEQ ID NOs: 201 to 300 and 501 to 600, preferably, any one sequence selected from the group consisting of SEQ ID NOs: 201 to 210, 212, 218, 221, 223, 504 to 507, 514, 515 and 522 to 525, more preferably, any one sequence selected from the group consisting of SEQ ID NOs: 206 to 209, 212, 218, 221, 223, 507, 515, and 525, the most preferably, any one sequence of SEQ ID NO: 212, 218, 221, 223 or 525, and an antisense strand including a complementary sequence thereto.

In addition, the composition for preventing or treating respiratory diseases, including the double-helical oligo RNA structure according to the present invention may include: a double-helical oligo RNA structure including a CTGF-specific siRNA that includes a sense strand of human and mouse CTGF-specific siRNA according to sequence of SEQ ID NO: 6 or 8, preferably, SEQ ID NO: 6 and an antisense strand including a complementary sequence thereto;
a double-helical oligo RNA structure including a Cyr61-specific siRNA that includes a sense strand of human and mouse Cyr61-specific siRNA according to any one sequence of SEQ ID NO: 102, 104 and 105, preferably, SEQ ID NO: 102 and an antisense strand including a complementary sequence thereto; or
a double-helical oligo RNA structure including a Plekho1-specific siRNA that includes a sense strand of human and mouse Plekho1-specific siRNA according to any one sequence of SEQ ID NO: 204, 207 and 208, preferably, SEQ ID NO: 207 and an antisense strand including a complementary sequence thereto.

In addition, a double-helical oligo RNA structure containing the CTGF-specific siRNA, a double-helical oligo RNA structure containing the Cyr61-specific siRNA, and/or a double-helical oligo RNA structure containing the Plekho1-specific siRNA may be mixed and included in the composition, and additionally, siRNA specific to the other respiratory diseases-related gene in addition to CTGF, Cry61 or Plekho1 or a double-helical oligo RNA structure containing the other respiratory diseases-related gene-specific siRNA may also be included in the composition according to the present invention.

At the time of using a composition for preventing or treating respiratory diseases additionally including the double-helical oligo RNA structure containing the other respiratory diseases-related gene-specific siRNA together with the CTGF, Cyr61, and/or Plekho1-specific siRNA, or the double-helical oligo RNA structure containing the CTGF, Cyr61, and/or Plekho1-specific siRNA, a synergistic effect like that of a combination therapy may be obtained.

Examples of the respiratory diseases capable of being prevented or treated by the composition of the present invention include idiopathic pulmonary fibrosis, asthma, chronic obstructive pulmonary disease (COPD), acute or chronic bronchitis, allergic rhinitis, cough and phlegm, acute lower respiratory tract infection, bronchitis, and bronchiolitis, acute upper respiratory tract infection, pharyngitis, tonsillitis, laryngitis, etc., but the present invention is not limited thereto.

Further, the nanoparticle included in the composition for preventing or treating the respiratory disease, including the nanoparticle formed of the double-helical oligo RNA structure according to the present invention may purely consist of only any one structure selected from the double-helical oligo RNA structure containing the CTGF, Cyr61, or Plekho1-specific siRNA, or may be configured in a form in which two or more kinds of the double-helical oligo RNA structures including the CTGF, Cyr61, or Plekho1-specific siRNA are mixed with each other.

The composition of the present invention may be prepared by additionally including at least one pharmaceutically acceptable carrier in addition to the effective components. The pharmaceutically acceptable carrier is required to be compatible with the effective components of the present invention, and may be used by mixing one or more components selected from saline, sterile water, Ringer's solution, buffered saline, dextrose solution, maltodextrin solution, glycerol and ethanol, and other conventional additives such as antioxidant, buffer, fungistat, and the like, may be added thereto as needed. In addition, the composition may be prepared as a formulation for injection, such as an aqueous solution, suspension, emulsion, and the like, by additionally adding diluent, dispersant, surfactant, binder and lubricant thereto. In particular, it is preferred to provide the composition prepared as a lyophilized formulation. To prepare the lyophilized formulation, any method which is generally known in the technical field of the present invention may be used, wherein a stabilizer for lyophlization may be added thereto. In addition, appropriate methods in the art or a method disclosed in Remington's pharmaceutical Science, Mack Publishing Company, or Easton PA may be preferably used for formulation depending on each disease or component.

The dosage and administration method of the effective components, etc, included in the pharmaceutical composition of the present invention may be determined based on symptoms of the general patient and severity of the disease by general experts in the art. In addition, the composition may be formulated with various types such as powder, tablet, capsule, solution, injection, ointment, syrup, and the like, and may be provided as a unit-dose or a multi-dose container, for example, a sealed ampoule, bottle, and the like.

The pharmaceutical composition of the present invention may be orally or parenterally administered. Examples of an administration route of the pharmaceutical composition according to the present invention may include oral, intravenous, intramuscular, intra-arterial, intramedullary, intradural, intracardiac, transdermal, subcutaneous, intraperitoneal, intestinal, sublingual or topical administration, but the present invention is not limited thereto. Particularly, the administration route may also include administration into lung via drip infusion in respiratory organs for treatment of respiratory diseases. The administration amount of the composition may have various ranges depending on weight, age, gender, health condition, diet, administration time, method, excretion rate, the severity of disease, and the like, of a patient, and may be easily determined by a general expert in the art. In addition, the composition of the present invention may be formulated into an appropriate dosage form by using known technologies for clinical administration.

Further, the present invention provides a method for preventing or treating respiratory diseases, particularly, idiopathic pulmonary fibrosis and COPD, including: administrating the double-helical oligo RNA structure according to the present invention and the nanoparticle including the double-helical oligo RNA structure to a patient requiring such treatment.

### EXAMPLES

Hereinafter, the present invention will be described in detail with reference to the following Examples. These examples are only for exemplifying the present invention, and it will be obvious to those skilled in the art that the scope of the present invention is not construed to be limited to these examples.

### Example 1. Design of target sequence of CTGF, Cyr61 or Plekho1 and production of siRNA

604 types of target sequences (sense strands) capable of being bonded to mRNA sequence of *CTGF*(*Homo sapiens*) gene (NM_001901), mRNA sequence of Cyr61(*Homo sapiens*) gene (NM_001554), mRNA sequence of Plekho1(*Homo sapiens*) gene (NM_016274), mRNA sequence of CTGF(*Mus musculus*) gene (NM_010217), mRNA sequence of Cyr61(*Mus musculus*) gene (NM_010516), or mRNA sequence of Plekho1 (Mus musculus) gene (NM_023320) were designed, and siRNAs of antisense strands having complementary sequences to the target sequences were produced.

First, a gene design program (Turbo si-Designer) developed by Bioneer Co., was used to design target sequence that the siRNA is capable of being bonded from mRNA sequences of the corresponding genes. The siRNA for respiratory disease-related genes according to the present invention has a double stranded structure including a sense strand consisting of 19 nucleotides and an antisense strand complementary thereto. Further, siCONT (having sequence of SEQ ID NO: 601 as a sense strand) which is siRNA having a sequence in which expression of any gene is not inhibited, was produced. The siRNA was produced by linking phosphodiester bonds forming an RNA backbone structure by using β-cyanoethyl phosphoramidite (Nucleic Acids Research, 12:4539-4557, 1984). Specifically, a reaction product including RNA having a desired length was obtained by repeating a series of processes of deblocking, coupling, oxidation and capping, on a solid support to which nucleotide is attached, using RNA synthesizer (384 Synthesizer, BIONEER, Korea). RNA of the reaction product was separated and purified by HPLC LC918(Japan Analytical Industry, Japan) equipped with Daisogel C₁₈ (Daiso, Japan) column, and it was confirmed whether or not the purified RNA meets the target sequence by MALDI-TOF mass spectrometer (Shimadzu, Japan). Then, the desired doublestranded siRNA (SEQ ID NOs: 1 to 604) was produced by binding the RNA sense strand to the RNA antisense strand.

### Example 2. Production of double-helical oligo RNA structure (PEG-SAMiRNA).

The double-helical oligo RNA structure (PEG-SAMiRNA) produced in the present invention has a structure represented by the following Structural Formula (16):

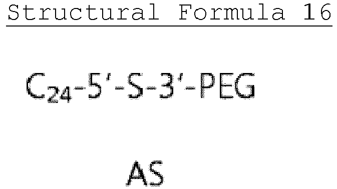

In Structural Formula (16), S is a sense strand of siRNA; AS is an antisense strand of siRNA; PEG is a hydrophilic material, that is, polyethylene glycol; C₂₄ is a hydrophobic material and tetradocosane including a disulfide bond; and 5' and 3' mean directions of the double-helical oligo RNA end.

The sense strand of siRNA of Structural Formula (16) was produced by synthesizing a double-helical oligo RNA-hydrophilic material structure of a sense strand in which polyethylene glycol is bonded to 3' end by the above-described method in which phosphodiester bonds forming an RNA backbone structure are linked by using β-cyanoethyl phosphoramidite, based on 3' polyethylene glycol (PEG, Mn=2,000)-CPG produced by Example 1 of Korean Patent Laid-Open Publication No. 10-2012-0119212, as a supporter, and binding tetradocosane including a disulfide bond to 5' end, thereby producing a sense strand of a desired RNA-polymer structure. For an antisense strand in which annealing is performed with the sense strand, the antisense strand having a complementary sequence to the sense strand was produced by the above-described reaction.

When the synthesis was completed, the RNA single strand and the RNA-polymer structure synthesized by treating 28%(v/v) ammonia in water bath at 60°C were separated from CPG and protecting moieties were removed therefrom by a deprotection reaction, respectively. The RNA single strand and the RNA-polymer structure from which the protecting moieties were removed were treated with N-methylpyrrolidone, triethylamine and triethylaminetrihydrofluoride at a volume ratio of 10:3:4 in an oven at 70°C, to remove 2' TBDMS(tertbutyldimethylsilyl).

RNA of the reaction product was separated and purified by HPLC LC918 (Japan Analytical Industry, Japan) equipped with Daisogel C18(Daiso, Japan) column, and it was confirmed whether or not the purified RNA meets the target sequence by MALDI-TOF mass spectrometer (Shimadzu, Japan). Then, to produce each double-helical oligo RNA structure, the sense strand and the antisense strand each having the same amount were mixed with each other, put in 1X annealing buffer (30 mM HEPES, 100 mM potassium acetate, 2 mM magnesium acetate, pH 7.0 ∼7.5), and reacted in a constant-temperature water bath at 90°C for 3 minutes, and reacted again at 37°C, thereby producing each double-helical oligo RNA structure including siRNA having sequence of SEQ ID NO: 42, 59, 602, 124, 153, 187, 197, 212, 218, 221, 223, 323, 424, 525 or 601 as the sense strand (hereinafter, referred to as SAMiRNALP-hCTGF, SAMiRNALP-hCyr, SAMiRNALP-hPlek, SAMiRNALP-mCTGF, SAMiRNALP-mCyr, and SAMiRNALP-mPlek SAMiRNALP-CONT, respectively). It was confirmed that the produced double-helical oligo RNA structure was annealed by electrophoresis.

### Example 3. Production of improved double-helical oligo RNA structure (Mono-HEG-SAMiRNA).

The improved double-helical oligo RNA structure produced in the present invention is obtained by using [PO₃⁻-hexaethylene glycol]₄ (hereinafter, referred to as 'Mono-HEG-SAMiRNA', see Structural Formula (17)) which is the hydrophilic material block instead of using PEG which is the hydrophilic material, and has the following Structure Formula (17):

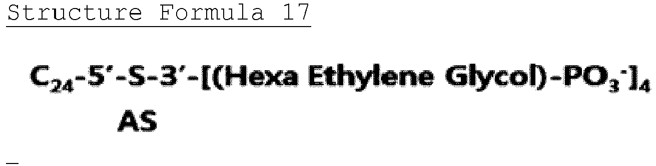

In Structural Formula (17), S is a sense strand of siRNA; AS is an antisense strand of siRNA; [Hexa Ethylene Glycol]₄ is a hydrophilic material monomer; C₂₄ is a hydrophobic material and tetradocosane including a disulfide bond; and 5 'and 3' mean directions of the double-helical oligo RNA sense strand end.

The structure of Mono-HEG SAMiRNA according to Structural Formula (17) may be represented by the following Structural Formula (18):

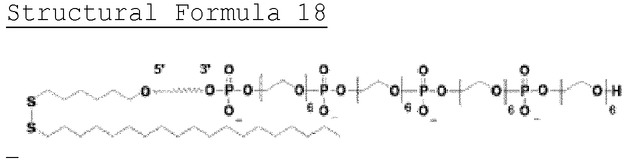

RNA of the reaction product was separated and purified by HPLC LC918 (Japan Analytical Industry, Japan) equipped with Daisogel C₁₈(Daiso, Japan) column, and it was confirmed whether or not the purified RNA meets the target sequence by MALDI-TOF mass spectrometer (Shimadzu, Japan). Then, to produce each double-helical oligo RNA structure, the sense strand and the antisense strand each having the same amount were mixed with each other, put in 1X annealing buffer (30 mM HEPES, 100 mM potassium acetate, 2 mM magnesium acetate, pH 7.0 ∼7.5), and reacted in a constant-temperature water bath at 90°C for 3 minutes, and reacted again at 37°C, thereby producing each double-helical oligo RNA structure including siRNA having sequence of SEQ ID NO: 42, 59, 602, 124, 153, 187, 197, 212, 218, 221, 223, 323, 424, 525 or 601 as the sense strand (hereinafter, referred to as Mono-HEG-SAMiRNALP-hCTGF, Mono-HEG-SAMiRNALP-hCyr, Mono-HEG-SAMiRNALP-hPlek, Mono-HEG-SAMiRNALP-mCTGF, Mono-HEG-SAMiRNALP-mCyr, Mono-HEG-SAMiRNALP-mPlek, and Mono-HEG-SAMiRNALP-CONT, respectively). It was confirmed that the produced double-helical oligo RNA structure was annealed by electrophoresis.

### Example 4. Production of nanoparticles formed of improved double-helical oligo RNA structure (Mono-HEG-SAMiRNA) and measurement of size thereof

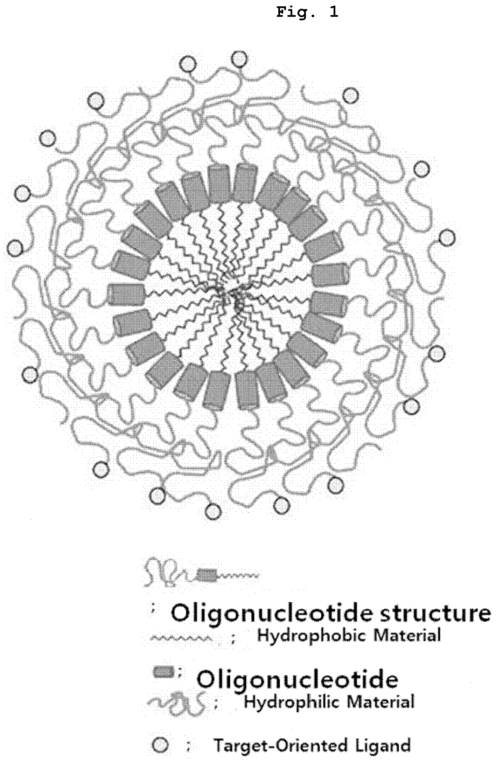

The double-helical oligo RNA structure (Mono-HEG-SAMiRNA) produced by Example 3 forms a nanoparticle, that is, micelle by a hydrophobic interaction between the hydrophobic materials bonded to the end of the double-helical oligo RNA (FIG. 1).

It was confirmed that the nanoparticle (SAMiRNA) formed of the corresponding Mono-HEG-SAMiRNA was formed by analyzing PDI (polydispersity index) of the nanoparticles formed of Mono-HEG-SAMiRNA-hCTGF, Mono-HEG-SAMiRNA-hCyr, Mono-HEG-SAMiRNA-hPlek, Mono-HEG-SAMiRNA-mCTGF, Mono-HEG-SAMiRNA-mCyr, Mono-HEG-SAMiRNA-mPlek and Mono-HEG-SAMiRNA-CONT.

### Example 4-1. Production of nanoparticle

The Mono-HEG-SAMiRNA-hCTGF was dissolved in 1.5 mℓ DPBS (Dulbecco's Phosphate Buffered Saline) at a concentration of 50 *µ*g/mℓ, the obtained mixture was freeze-dried at -75°C and 5mTorr condition for 48 hours to produce nanoparticle powder, and the nanoparticle powder was dissolved in DPBS which is a solvent to produce homogenized nanoparticles. The nanoparticles formed of Mono-HEG-SAMiRNA-hCyr, Mono-HEG-SAMiRNA-hPlek, Mono-HEG-SAMiRNA-mCTGF, Mono-HEG-SAMiRNA-mCyr, Mono-HEG-SAMiRNA-mPlek, and Mono-HEG-SAMiRNA-CONT were produced by the same method.

### Example 4-2. Measurement of size and polydispersity index (PDI) of nanoparticle

A size of the nanoparticle was measured by zeta-potential measurement. A size of the homogenized nanoparticles produced by Example 4-1 was measured by zeta-potential measurement (Nano-ZS, MALVERN, England), under conditions in which a refractive index to the material is 1.459, an absorption index is 0.001, a temperature of a solvent: DPBS is 25°C and the corresponding viscosity and refractive index are 1.0200 and 1.335, respectively. Once measurement was conducted by a size measurement including repeating 15 times and then repeating six times.

As the PDI value is decreased, the corresponding particles become uniformly distributed, and thus, it could be appreciated that the nanoparticles of the present invention have a significantly uniform size.

### Example 5. Confirmation of inhibition of target gene expression using target gene-specific siRNA for human in human fibroblast cell line (MRC-5).

Human fibroblast (MRC-5) which is a fibroblast cell line was transformed by siRNAs having sequences of SEQ ID NOs: 1 to 10, 101 to 110, 201 to 210 and 601 produced by Example 1 as the sense strand, and the expression aspect of the target gene was analyzed in the transformed fibroblast cell line (MRC-5).

### Example 5-1. Culture of human fibroblast cell line

A human fibroblast cell line (MRC-5) obtained from Korean Cell line bank (KCLB) was cultured in RPMI-1640 culture medium (GIBCO/Invitrogen, USA, 10% (v/v) fetal bovine serum, penicillin 100 units/ml and 100 *µ*g/mℓ of streptomycin) under condition of 37 °C and 5%(v/v) CO₂.

### Example 5-2. Transfection of target siRNA into human fibroblast cell line

1.8×10⁵ fibroblast cell line (MRC-5) cultured by Example 5-1 was cultured in RPMI 1640 medium for 18 hours in 6-well plate under condition of 37°C and 5 %(v/v) CO₂, and the medium was removed, and 500*µ*ℓ of Opti-MEM medium (GIBCO, USA) for each well was dispensed.

Meanwhile, 3.5*µ*ℓ of Lipofectamine™ RNAi Max (Invitrogen, USA) was mixed with 246.5*µ*ℓ of Opti-MEM medium to prepare a mixed solution, and the mixed solution was reacted at room temperature for 5 minutes. siRNA solutions each having a final concentration of 5 or 20 nM were prepared by adding 5 or 20*µ*ℓ of siRNAs (1 pmole/*µ*ℓ) having sequences of SEQ ID NOs: 1 to 10, 101 to 110, 201 to 210 and 601 by Example 1, as the sense strand, to 230*µ*ℓ of Opti-MEM medium. The Lipofectamine™ RNAi Max mixture and the siRNA solution were mixed and reacted at room temperature for 15 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO2 for 24 hours.

### Example 5-3. Quantitative analysis of target gene mRNA

cDNA was produced by extracting total RNA from the transfected cell line by Example 5-2, and an mRNA expression amount of the target gene was subjected to relative-quantification by real-time PCR.

### Example 5-3-1. RNA separation from transfected cell and cDNA production

Total RNA was extracted from the transfected cell line of Example 5-2 by RNA extraction kit (AccuPrep Cell total RNA extraction kit, BIONEER, Korea), and the extracted RNA was used to produce cDNA by RNA reverse transcriptase (AccuPower CycleScript RT Premix/dT20, Bioneer, Korea) according to the following method. Specifically, 1 *µ*g of the extracted RNA per each tube was added to AccuPower CycleScript RT Premix/dT20(Bioneer, Korea) contained in 0.25mℓ Eppendorf tube, and distilled water treated with DEPC(diethyl pyrocarbonate) was added thereto so as to have a total volume of 20*µ*ℓ. A process of hybridizing RNA and primers at 30°C for 1 minute by a gene amplifier (MyGenie™ 96 Gradient Thermal Block, BIONEER, Korea) and a process of producing cDNA at 52°C for 4 minutes were repeated 6 times, and then, an amplification reaction was completed by inactivating the enzyme at 90°C for 5 minutes.

### Example 5-3-2. Relative-quantitative analysis of target gene mRNA

A relative amount of the respiratory disease-related gene mRNA was quantified by the following method through real-time PCR having the cDNA produced by Example 5-3-1 as a template. cDNA produced by Example 5-3-1 was diluted 5 times with distilled water in each well of 96-well plate. Then, 3*µ*ℓ of the diluted cDNA, 25*µ*ℓ of 2 × GreenStar™ PCR master mix (BIONEER, Korea), 19*µ*ℓ of distilled water, and *3µ*ℓ of qPCR primers (Table 2; F and R each having 10 pmole/*µ*ℓ; BIONEER, Korea) were added thereto to prepare each mixed solution. Meanwhile, RPL13A (ribosomal protein L13a) which is a housekeeping gene (hereinafter, referred to as HK gene) was determined as a standard gene to normalize the expression amount of the target gene mRNA. The following reaction was performed on 96-well plate containing the mixture by Exicycler™ 96 Real-Time Quantitative Thermal Block (BIONEER, Korea). After the reaction at 95°C for 15 minutes to activate the enzyme and remove a secondary structure of cDNA, four processes including a process of denaturing at 94°C for 30 seconds, a process of annealing at 58°C for 30 seconds, a process of extension at 72°C for 30 seconds, and a process of SYBR green scan were repeated 42 times, and final extension was performed at 72°C for 3 minutes. Then, the temperature was maintained at 55°C for 1 minute, and a melting curve was analyzed from 55°C up to 95°C. After PCR was completed, for Ct (threshold cycle) value of each of the obtained target gene, Ct values of the target gene corrected through GAPDH gene were calculated, and then the difference (ΔCt) was obtained by using a test group treated with siRNA (SEQ ID NO: 601, siCONT) having a control sequence preventing inhibition of gene expression, as a control group.

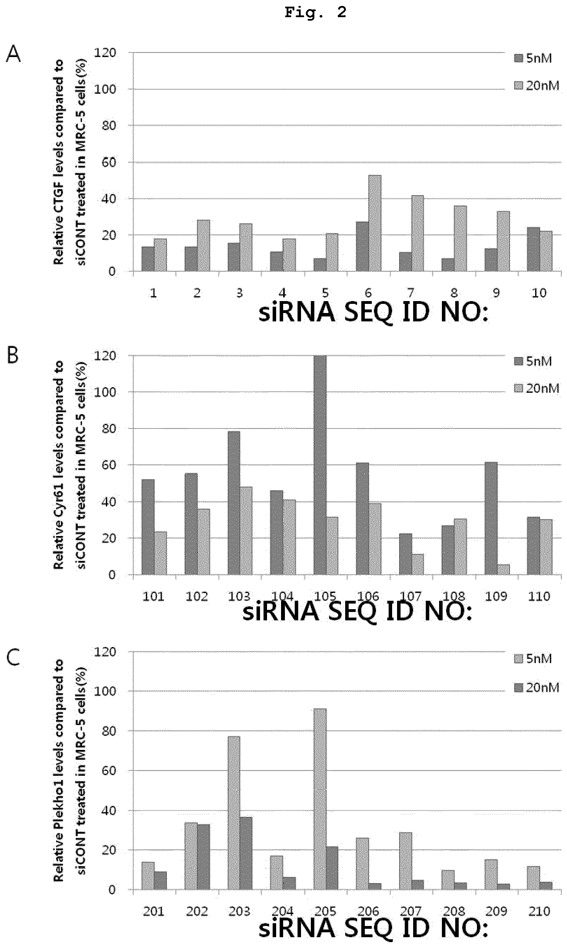

The expression amount of the target gene of the cell treated with the CTGF (Homo sapiens)-specific siRNA (having sequence of SEQ ID NOs: 1 to 10 as the sense strand) was relatively quantified by using the ΔCt value and calculation formula 2(-ΔCt)× 100 (FIG. 2A). Further, the expression amount of the target gene of the cell treated with the Cyr61(Homo sapiens)-specific siRNA (having sequence of SEQ ID NOs: 101 to 110 as the sense strand) was relatively quantified (FIG. 2B), and the expression amount of the target gene of the cell treated with the Plekho1(Homo sapiens)-specific siRNA (having sequence of SEQ ID NOs: 201 to 210 as the sense strand) was relatively quantified (FIG. 2C).

As a result, it could be confirmed that several kinds of siRNA of the present invention showed high inhibition level of target gene expression. In addition, in order to select siRNA with high efficiency, siRNAs having sequences of SEQ ID NOs: 1, 3, 4, 8, 9, 10, 102, 104, 105, 106, 107, 108, 109, 204, 206, 207, 208, 209 and 210 in which the mRNA expression amount for each gene at 5 nM concentration is significantly decreased, as the sense strand, were selected.

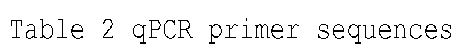

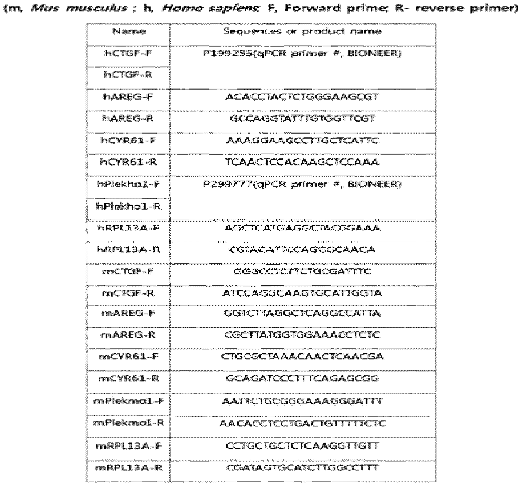

### Example 6. Selection of target gene-specific siRNA for human with high efficiency in human fibroblast cell line (MRC-5)

Human fibroblast cell line (MRC-5) was transformed by using siRNAs having sequences of SEQ ID NOs: 1, 3, 4, 8, 9, 10, 102, 104, 105, 106, 107, 108, 109, 204, 206, 207, 208, 209, 210 and 601 selected by Example 5-3-2, as the sense strand, and the expression aspect of the target gene was analyzed in the transformed fibroblast cell line (MRC-5) to select siRNA with high efficiency.

### Example 6-1. Culture of human fibroblast cell line

Human fibroblast cell line (MRC-5) obtained from Korean Cell line bank (KCLB, Korea) was cultured under the same condition as Example 5-1.

### Example 6-2. Transfection of target siRNA into human fibroblast cell line

1.8×10⁵ fibroblast cell line (MRC-5) cultured by Example 6-1 was cultured in RPMI 1640 medium for 18 hours in 6-well plate under condition of 37°C and 5 %(v/v) CO₂, and the medium was removed, and 500*µ*ℓ of Opti-MEM medium (GIBCO, USA) for each well was dispensed.

Meanwhile, 3.5*µ*ℓ of Lipofectamine™ RNAi Max (Invitrogen, USA) was mixed with 246.5*µ*ℓ of Opti-MEM medium to prepare a mixed solution, and the mixed solution was reacted at room temperature for 5 minutes. siRNA solutions each having a final concentration of 0.2 or 1 nM were prepared by adding 0.2 or 1*µ*ℓ of siRNAs (1 pmole/*µ*ℓ) having sequences of SEQ ID NOs: 1, 3, 4, 8, 9, 10, 102, 104, 105, 106, 107, 108, 109, 204, 206, 207, 208, 209, 210 and 601 by Example 1, as the sense strand, to 230*µ*ℓ of Opti-MEM medium. The Lipofectamine™ RNAi Max mixture and the siRNA solution were mixed and reacted at room temperature for 15 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO₂ for 24 hours.

### Example 6-3. Quantitative analysis of target gene mRNA

cDNA was produced by extracting total RNA from the transfected cell line by Example 6-2 through the same method as Example 4-3, and an mRNA expression amount of the target gene was subjected to relative-quantification by real-time PCR. The inhibition amount of the target gene expression according to low concentration siRNA treatment was observed to clearly confirm each siRNA efficacy, and it was confirmed that siRNAs having sequence of SEQ ID NOs: 8, 107 and 206 as the sense strand showed relatively high level of inhibition for target gene expression even at a significantly low concentration (FIG. 3).

### Example 7. Confirmation of inhibition of target gene expression using target gene-specific siRNA for human in human lung cancer cell line (A549).

Human lung cancer cell line (A549) which is a lung tumor cell line was transformed by siRNAs having sequences of SEQ ID NOs: 35, 42, 59, 602, 603, 604, 124, 153, 166, 187, 197, 212, 218, 221, 223 and 601 produced by Example 1 as the sense strand, and the expression aspect of the target gene was analyzed in the transformed lung cancer cell line (A549).

### Example 7-1. Culture of human lung cancer cell line

A human lung cancer cell line (A549) obtained from American Type Culture Collection (ATCC) was cultured in DMEM culture medium (GIBCO/Invitrogen, USA, 10% (v/v) fetal bovine serum, penicillin 100 units/ml and 100 *µ*g/mℓ of streptomycin) under condition of 37 °C and 5%(v/v) CO₂.

### Example 7-2. Transfection of target siRNA into human lung cancer cell line

1.2×10⁵ lung cancer cell line (A549) cultured by Example 7-1 was cultured in DMEM medium for 18 hours in 6-well plate under condition of 37°C and 5 %(v/v) CO₂, and the medium was removed, and 500*µ*ℓ of Opti-MEM medium (GIBCO, USA) for each well was dispensed.

Meanwhile, 3.5*µ*ℓ of Lipofectamine™ RNAi Max (Invitrogen, USA) was mixed with 246.5*µ*ℓ of Opti-MEM medium to prepare a mixed solution, and the mixed solution was reacted at room temperature for 5 minutes. siRNA solutions each having a final concentration of 5nM, 1nM, 0.5nM, 0.2nM or 0.04nM were prepared by adding 1*µ*ℓ of siRNAs (1 pmole/*µ*ℓ) having sequences of SEQ ID NOs: 35, 42, 59, 602, 603, 604, 124, 153, 166, 187, 197, 212, 218, 221 and 223 by Example 1, as the sense strand, to 230*µ*ℓ of Opti-MEM medium. The Lipofectamine™ RNAi Max mixture and the siRNA solution were mixed and reacted at room temperature for 15 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO₂ for 24 hours.

### Example 7-3. Quantitative analysis of target gene mRNA

cDNA was produced by extracting total RNA from the transfected cell line by Example 7-2, and an mRNA expression amount of the target gene was subjected to relative-quantification by real-time PCR.

### Example 7-3-1. RNA separation from transfected cell and cDNA production

Total RNA was extracted from the transfected cell line of Example 5-2 by RNA extraction kit (AccuPrep Cell total RNA extraction kit, BIONEER, Korea), and the extracted RNA was used to produce cDNA by RNA reverse transcriptase (AccuPower CycleScript RT Premix/dT20, Bioneer, Korea) according to the following method. Specifically, 1 *µ*g of the extracted RNA per each tube was added to AccuPower CycleScript RT Premix/dT20(Bioneer, Korea) contained in 0.25*m*ℓ Eppendorf tube, and distilled water treated with DEPC (diethyl pyrocarbonate) was added thereto so as to have a total volume of 20*µ*ℓ. A process of hybridizing RNA and primers at 30°C for 1 minute by a gene amplifier (MyGenie™ 96 Gradient Thermal Block, BIONEER, Korea) and a process of producing cDNA at 52°C for 4 minutes were repeated 6 times, and then, an amplification reaction was completed by inactivating the enzyme at 90°C for 5 minutes.

### Example 7-3-2. Relative quantitative analysis of target gene mRNA

A relative amount of the respiratory disease-related gene mRNA was quantified by the following method through real-time PCR having the cDNA produced by Example 7-3-1 as a template. cDNA produced by Example 6-3-1 was diluted 5 times with distilled water in each well of 96-well plate. Then, 3*µ*ℓ of the diluted cDNA, 25*µ*ℓ of 2 × GreenStar™ PCR master mix (BIONEER, Korea), 19*µ*ℓ of distilled water, and 3*µ*ℓ of qPCR primers (Table 2; F and R each having 10 pmole/*µ*ℓ; BIONEER, Korea) were added thereto to prepare each mixed solution. Meanwhile, RPL13A (ribosomal protein L13a) which is a housekeeping gene (hereinafter, referred to as HK gene) was determined as a standard gene to normalize the expression amount of the target gene mRNA. The following reaction was performed on 96-well plate containing the mixture by Exicycler™ 96 Real-Time Quantitative Thermal Block (BIONEER, Korea). After the reaction at 95°C for 15 minutes to activate the enzyme and remove a secondary structure of cDNA, four processes including a process of denaturing at 94°C for 30 seconds, a process of annealing at 58°C for 30 seconds, a process of extension at 72°C for 30 seconds, and a process of SYBR green scan were repeated 42 times, and final extension was performed at 72°C for 3 minutes. Then, the temperature was maintained at 55°C for 1 minute, and a melting curve was analyzed from 55°C up to 95°C. After PCR was completed, for Ct (threshold cycle) value of each of the obtained target gene, Ct values of the target gene corrected through GAPDH gene were calculated, and then the difference (ΔCt) was obtained by using a test group treated with siRNA (SEQ ID NO: 601, siCONT) having a control sequence preventing inhibition of gene expression, as a control group.

The expression amount of the target gene of the cell treated with the CTGF (Homo sapiens)-specific siRNA (having sequence of SEQ ID NOs: 132, 42, 59, 602, 603, 604 as the sense strand) was relatively quantified by using the ΔCt value and calculation formula 2(^{-ΔCt})× 100 (FIG. 4A). Further, the expression amount of the target gene of the cell treated with the Cyr61(Homo sapiens)-specific siRNA (having sequence of SEQ ID NOs: 124, 153, 166, 187, and 197 as the sense strand) was relatively quantified (FIG. 4B), and the expression amount of the target gene of the cell treated with the Plekho1(Homo sapiens)-specific siRNA (having sequence of SEQ ID NOs: 212, 218, 221, and 223 as the sense strand) was relatively quantified (FIG. 4C).

As a result, it could be confirmed that several kinds of siRNA of the present invention showed high inhibition level of target gene expression. In addition, in order to select siRNA with high efficiency, siRNAs having sequences of SEQ ID NOs: 42, 59, 602, 124, 153, 187, 197, 212, 218, 221 and 223 in which the mRNA expression amount for each gene at 5 nM concentration is significantly decreased, as the sense strand, were selected.

### Example 8. Inhibition of target gene expression in human lung cancer cell line (A549) by nanoparticle (SAMiRNA) formed of double-helical oligo polymer structure

Human lung cancer cell line (A549) was transformed by using the nanoparticle formed of SAMiRNA LP including siRNA having sequences of SEQ ID NOs: 42, 59, 602, 124, 153, 187, 197, 212, 218, 221 and 223 selected by Example 7-3-2, as the sense strand, and the expression aspect of the target gene was analyzed in the transformed lung cancer cell line (A549).

### Example 8-1. Culture of human lung cancer cell line

Human lung cancer cell line (A549) obtained from American Type Culture Collection (ATCC) was cultured under the same condition as Example 7-1.

### Example 8-2. Transfection of target SAMiRNA into human lung cancer cell line

1.2×10⁵ lung cancer cell line (A549) cultured by Example 8-1 was cultured in RPMI 1640 medium for 18 hours in 12-well plate under condition of 37°C and 5 %(v/v) CO₂, and the medium was removed, and the same amount of Opti-MEM medium (GIBCO, USA) for each well was dispensed. 100*µ*ℓ of Opti-MEM medium and SAMiRNALP and monoSAMiRNALP produced by Example 4-2 were added to DPBS at a concentration of 50 *µ*g/mℓ, the obtained mixture was freeze-dried at -75°C and 5mTorr condition for 48 hours by the same method as Example 5-1 to produce homogenized nanoparticles. Then, each well of the tumor cell line in which the Opti-MEM is dispensed was treated with a transfection solution at a concentration of 200 nM, and cultured at 37°C and 5 %(v/v) CO₂ for the total of 48 hours.

### Example 8-3. Relative-quantitative analysis of target gene mRNA

cDNA was produced by extracting total RNA from the transfected cell line by Example 8-2 through the same method as Example 6-3, and an mRNA expression amount of the target gene was subjected to relative quantification by real-time PCR. The inhibition amount of the target gene expression according to low concentration siRNA treatment was observed to clearly confirm each siRNA efficacy, and it was confirmed that siRNAs having sequence of SEQ ID NOs: 42, 59 and 602 as the sense strand showed relatively high level of inhibition for target gene expression even at a significantly low concentration (FIG. 5).

### Example 9. Confirmation of inhibition of target gene expression using target gene-specific siRNA for mouse in mouse fibroblast cell line (NIH3T3).

Mouse fibroblast (NIH3T3) which is a fibroblast cell line was transformed by siRNAs having sequences of SEQ ID NOs: 301 to 330, 401 to 430, 501 to 530 and 601 produced by Example 1 as the sense strand, and an expression aspect of the target gene was analyzed in the transformed fibroblast cell line (NIH3T3).

### Example 9-1. Culture of mouse fibroblast cell line

A mouse fibroblast cell line (NIH3T3) obtained from American Type Culture Collection (ATCC) was cultured in RPMI-1640 culture medium (GIBCO/Invitrogen, USA, 10% (v/v) fetal bovine serum, penicillin 100 units/ml and 100 *µ*g/mℓ of streptomycin) under condition of 37 °C and 5%(v/v) CO₂.

### Example 9-2. Transfection of target siRNA into mouse fibroblast cell line

1×10⁵ fibroblast cell line (NIH3T3) cultured by Example 9-1 was cultured in RPMI 1640 medium for 18 hours in 12-well plate under condition of 37°C and 5 %(v/v) CO₂, and the medium was removed, and 500*µ*ℓ of Opti-MEM medium (GIBCO, USA) for each well was dispensed.

Meanwhile, 1.5*µ*ℓ of Lipofectamine™ RNAi Max (Invitrogen, USA) was mixed with 248.5*µ*ℓ of Opti-MEM medium to prepare a mixed solution, and the mixed solution was reacted at room temperature for 5 minutes. 5 or 20*µ*ℓ of siRNAs (1 pmole/*µ*ℓ) having sequences of SEQ ID NOs: 301 to 330, 401 to 430, 501 to 530 and 601 produced by Example 1, as the sense strand, were added to 230*µ*ℓ of Opti-MEM medium, thereby preparing siRNA solutions each having a final concentration of 5 or 20 nM. The Lipofectamine™ RNAi Max mixture and the siRNA solution were mixed and reacted at room temperature for 20 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 *m*ℓ of RPMI 1640 culture medium was dispensed therein and cultured under condition of 37°C and 5%(v/v) CO₂ for 24 hours.

### Example 9-3. Quantitative analysis of target gene mRNA

cDNA was produced by extracting total RNA from the transfected cell line by Example 9-2 through the same method as Example 5-3, and an mRNA expression amount of the target gene was subjected to relative quantification by real-time PCR.

The expression amount of the target gene of the cell treated with the CTGF (Mus musculus)-specific siRNA (having sequence of SEQ ID NOs: 301 to 330 as the sense strand) was relatively quantified (FIG. 6A). Further, the expression amount of the target gene of the cell treated with the Cyr61(Mus musculus)-specific siRNA (having sequence of SEQ ID NOs: 401 to 430 as the sense strand) was relatively quantified (FIG. 6B), and the expression amount of the target gene of the cell treated with the Plekho1 (Mus musculus)-specific siRNA (having sequence of SEQ ID NOs: 501 to 530 as the sense strand) was relatively quantified (FIG. 6C).

As a result, it could be confirmed that several kinds of siRNA of the present invention showed high inhibition level of target gene expression. In addition, in order to select siRNA with high efficiency, siRNA having sequence of SEQ ID NO: 301, 302, 303, 305, 306, 307, 309, 317, 323 or 329 in which the mRNA expression amount for CTGF(Mus musculus) at 20 nM concentration is significantly decreased, as the sense strand, was selected, siRNA having sequence of SEQ ID NO: 409, 410, 415, 417, 418, 420, 422, 424, 427 or 429 in which the mRNA expression amount for Cyr61(Mus musculus) at 20 nM concentration is significantly decreased, as the sense strand, was selected, and siRNA having sequence of SEQ ID NO: 504, 505, 506, 507, 514, 515, 522, 523, 524 or 525 in which the mRNA expression amount for Plekho1 (Mus musculus) at 20 nM concentration is significantly decreased, as the sense strand, was selected.

Further, in order to select siRNA with more preferable efficiency, siRNA having sequence of SEQ ID NO: 301, 303, 307 or 323 in which the mRNA expression amount for CTGF(Mus musculus) at 5 nM concentration is significantly decreased, as the sense strand, was selected, siRNA having sequence of SEQ ID NO: 410, 422, or 424 in which the mRNA expression amount for Cyr61(Mus musculus) at 5 nM concentration is significantly decreased, as the sense strand, was selected, and siRNA having sequence of SEQ ID NO: 507, 515, or 525 in which the mRNA expression amount for Plekho1 (Mus musculus) at 5 nM concentration is significantly decreased, as the sense strand, was selected (FIG. 7).

### Example 10. Selection of target gene-specific siRNA for mouse with high efficiency in mouse fibroblast cell line (NIH3T3)

The expression aspect of the target gene was analyzed in the mouse fibroblast cell line (NIH3T3) using siRNAs having sequences of SEQ ID NOs: 301, 303, 307, 323, 410, 422, 424, 507, 515, 525 and 601 selected by Example 9-3, as the sense strand, to select siRNA with high efficiency.

### Example 10-1. Culture of mouse fibroblast cell line

Mouse fibroblast cell line (NIH3T3) obtained from American Type Culture Collection (ATCC) was cultured under the same condition as Example 9-1.

### Example 10-2. Transfection of target siRNA into mouse fibroblast cell line

1×10⁵ fibroblast cell line (NIH3T3) cultured by Example 10-1 was cultured in RPMI 1640 medium for 18 hours in 12-well plate under condition of 37°C and 5 %(v/v) CO₂, and the medium was removed, and 500*µ*ℓ of Opti-MEM medium (GIBCO, USA) for each well was dispensed.

Meanwhile, 1.5*µ*ℓ of Lipofectamine™ RNAi Max (Invitrogen, USA) was mixed with 248.5*µ*ℓ of Opti-MEM medium to prepare a mixed solution, and the mixed solution was reacted at room temperature for 5 minutes. 0.2, 1 or 5*µ*ℓ of siRNAs (1 pmole/*µ*ℓ) having sequences of SEQ ID NOs: 301, 303, 307, 323, 410, 422, 424, 507, 515, 525 and 601 produced by Example 1, as the sense strand, were added to 230*µ*ℓ of Opti-MEM medium, thereby preparing siRNA solutions each having a final concentration of 0.2, 1 or 5 nM. The Lipofectamine™ RNAi Max mixture and the siRNA solution were mixed and reacted at room temperature for 20 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO₂ for 24 hours.

### Example 10-3. Quantitative analysis of target gene mRNA

cDNA was produced by extracting total RNA from the transfected cell line by Example 10-2 through the same method as Example 5-3, and an mRNA expression amount of the target gene was subjected to relative quantification by real-time PCR. The expression amount of the target gene of the cell treated with the CTGF (Mus musculus)-specific siRNA (having sequence of SEQ ID NOs: 301, 303, 307, and 323 as the sense strand) was relatively quantified (FIG. 8A). Further, the expression amount of the target gene of the cell treated with the Cyr61 (Mus musculus)-specific siRNA (having sequence of SEQ ID NOs: 410, 422 and 424, as the sense strand) was relatively quantified (FIG. 8B), and the expression amount of the target gene of the cell treated with the Plekho1 (Mus musculus)-specific siRNA (having sequence of SEQ ID NOs: 507, 515, and 525 as the sense strand) was relatively quantified (FIG. 8C).

As a result, it was confirmed that each target gene-specific siRNA inhibits the expression of the target gene in a concentration-dependent manner, and it was confirmed that siRNAs having sequences of SEQ ID NOs: 307, 424 and 525 as the sense strand showed relatively high level of inhibition for target gene expression even at a significantly low concentration, to select siRNA with high efficiency.

### Example 11. Confirmation of inhibition of target gene expression using target gene-specific siRNA for human in mouse fibroblast cell line (NIH3T3).

Since biopharmaceuticals have species-specific action sites such as protein structure or gene sequence, identity of treatment drug is significantly important for securing efficiency in developing biopharmaceutical novel drug. Gene sequence homology between the target gene-specific siRNA for human and the target gene-specific siRNA for mouse designed in Example 1 was analyzed to select siRNA sequences that may confirm the inhibition effect of the target gene expression in the mouse fibroblast cell.

The selected siRNA sequences are siRNAs having sequences of SEQ ID NOs: 4, 5, 6, 8, 9, 102, 104, 105, 107, 108, 109, 202, 204, 206, 207, 208 and 209, as the sense strand, which are the target gene-specific siRNAs for human produced by Example 1, siRNAs having sequences of SEQ ID NOs: 307, 424 and 525, as the sense strand, which are the target gene-specific siRNAs for mouse, and siRNA having sequence of SEQ ID NO: 601, as the sense strand, which is a control group. The expression aspect of the target gene was analyzed in the mouse fibroblast cell line (NIH3T3) using these selected siRNAs, and efficacy thereof was confirmed in the mouse cell of siRNA designed based on the human gene.

### Example 11-1. Culture of mouse fibroblast cell line

Mouse fibroblast cell line (NIH3T3) obtained from American Type Culture Collection (ATCC) was cultured under the same condition as Example 9-1.

### Example 11-2. Transfection of target siRNA into mouse fibroblast cell line

1.8×10⁵ fibroblast cell line (NIH3T3) cultured by Example 11-1 was cultured in RPMI 1640 medium for 18 hours in 6-well plate under condition of 37°C and 5 %(v/v) CO₂, and the medium was removed, and 500*µ*ℓ of Opti-MEM medium (GIBCO, USA) for each well was dispensed.

Meanwhile, 3.5*µ*ℓ of Lipofectamine™ RNAi Max (Invitrogen, USA) was mixed with 246.5*µ*ℓ of Opti-MEM medium to prepare a mixed solution, and the mixed solution was reacted at room temperature for 5 minutes. siRNA solutions each having a final concentration of 5 or 20 nM were prepared by adding 5 or 20*µ*ℓ of siRNAs (1 pmole/*µ*ℓ) having sequences of SEQ ID NOs: 4, 5, 6, 8, 9, 102, 104, 105, 107, 108, 109, 202, 204, 206, 207, 208, 209, 307, 424, 525 and 601 produced by Example 1, as the sense strand, to 230*µ*ℓ of Opti-MEM medium. The Lipofectamine™ RNAi Max mixture and the siRNA solution were mixed and reacted at room temperature for 15 minutes, thereby preparing a solution for transfection.

Then, 500*µ*ℓ of each transfection solution was dispensed in each well of the tumor cell line containing Opti-MEM dispensed therein, and cultured for 6 hours, and the Opti-MEM medium was removed. Here, 1 mℓ of RPMI 1640 culture medium was dispensed therein and cultured under condition of 37°C and 5%(v/v) CO₂ for 24 hours.

### Example 11-3. Quantitative analysis of target gene mRNA

cDNA was produced by extracting total RNA from the transfected cell line by Example 11-2 through the same method as Example 5-3, and an mRNA expression amount of the target gene was subjected to relative quantification by real-time PCR. The expression amount of the target gene of the cell treated with the CTGF (Mus musculus)-specific siRNA (SEQ ID NO: 307) or treated with the CTGF (Homo sapiens)-specific siRNA (SEQ ID NO: 4, 5, 6, 8 or 9) was relatively quantified (FIG. 9A). The expression amount of the target gene of the cell treated with Cyr61(Mus musculus)-specific siRNA (having a sequence of SEQ ID NO: 424 as the sense strand) or treated with Cyr61(Homo sapiens)-specific siRNA (having a sequence of SEQ ID NO: 102, 104, 105, 107, 108 or 109 as the sense strand) was relatively quantified (FIG. 9B), and the expression amount of the target gene of the cell treated with Plekho1(Mus musculus)-specific siRNA (having a sequence of SEQ ID NO: 525 as the sense strand) or treated with Plekho1(Homo sapiens)-specific siRNA (having a sequence of SEQ ID NO: 202, 204, 206, 207, 208 or 209 as the sense strand) was relatively quantified (FIG. 9C).

As a result, it was confirmed that each target gene-specific siRNA for human inhibits the expression of the target gene according to sequence homology, and it was confirmed that siRNAs having sequences of SEQ ID NOs: 6, 8, 102, 104, 105, 204, 207 and 208 as the sense strand showed relatively high level of inhibition for target gene expression at 20nM, and among them, IC50(inhibition concentration 50%) was less than 20nM in siRNAs having sequences of SEQ ID NOs: 6, 102 and 207 even in mouse cell lines. Therefore, it was confirmed that relatively high level of inhibition for target gene expression was maintained even at a low concentration, that is, these siRNAs had high efficiency (FIG. 10).

### ADVANTAGEOUS EFFECTS

The CTGF, Cyr61 or Plekho1-specific siRNA according to the present invention, the double-helical oligo RNA structure containing the siRNA, and the pharmaceutical composition containing the double-helical oligo RNA structure for treatment, are capable of inhibiting expression of CTGF, Cyr61 or Plekho1 at a high efficiency without side effects to provide treatment effects for respiratory diseases, particularly, idiopathic pulmonary fibrosis and chronic obstructive pulmonary disease (COPD), which may be significantly usefully used for treating respiratory diseases in which there is no appropriate therapeutic agent at present, particularly, idiopathic pulmonary fibrosis and chronic obstructive pulmonary disease (COPD).

From the foregoing, it will be understood by those skilled in the art to which the present invention pertains that the present invention can be carried out in other concrete embodiments without changing the technical spirit or essential feature thereof. In this regard, it should be understood that the aforementioned examples are of illustrative in all aspects but not is limited. The scope of the present invention should be construed to include the meaning and scope of the appended claims, and all the alterations and modified forms which are derived from the equivalent concept thereof, rather than the detailed description.

### In the following embodiments of the present application are summarized

**[Item 1]**
A CTGF, Cyr61 or Plekho1 specific siRNA comprising a sense strand and an anti-sense strand complementary to the sense strand, wherein the sense strand and the anti-sense strand comprise any one sequence selected from the group consisting of SEQ ID NOs: 1 to 600 and 602 to 604.
**[Item 2]**
The CTGF, Cyr61 or Plekho1 specific siRNA according to item 1, wherein the sense strand or anti-sense strand has 19 to 31 nucleotides.
**[Item 3]**
The CTGF, Cyr61 or Plekho1 specific siRNA according to item 1, wherein the sense strand and the anti-sense strand complementary to the sense strand comprise any one sequence selected from the group consisting of SEQ ID NOs: 1, 2, 3, 4, 5, 6, 7, 8, 9, 10, 35, 42, 59, 101, 102, 103, 104, 105, 106, 107, 108, 109. 110, 124, 153, 166, 187, 197, 201, 202, 203, 204, 205, 206, 207, 208, 209, 210, 212, 218, 221, 223, 301, 302, 303, 305, 306, 307, 309, 317, 323, 329, 409, 410, 415, 417, 418, 420, 422, 424, 427, 429, 504, 505, 506, 507, 514, 515, 522, 523, 524, 525, 602, 603 and 604.
**[Item 4]**
The CTGF, Cyr61 or Plekho1 specific siRNA according to any one of items 1 to 3, wherein the sense strand or the anti-sense strand of the siRNA comprises more than one chemical modification.
**[Item 5]**
The CTGF, Cyr61 or Plekho1 specific siRNA according to item 4,
wherein the chemical modification is more than any one selected from the group consisting of:
substitution of -OH group at 2' carbon in a sugar structure of nucleotides with -CH₃(methyl), -OCH₃ (methoxy), -NH₂, -F(fluorine), -0-2-methoxyethyl, -O-propyl, -O-2-methylthioethyl, -0-3-aminopropyl, -0-3-dimethylaminopropyl, -O-N-methylacetamido or -O-dimethylamidooxyethyl;
substitution of oxygen in a sugar structure of nucleotides with sulfur;
modification of bindings between nucleotides to phosphorothioate boranophosphate, or methyl phosphonate; and
modification of nucleotide to peptide nucleic acid (PNA), modification to locked nucleic acid (LNA), or modification to unlocked nucleic acid (UNA).
**[Item 6]**
The CTGF, Cyr61 or Plekho1 specific siRNA according to any one of items 1 to 5, more than one phosphate group (s) is bonded to 5' end of the antisense strand of siRNA.
**[Item 7]**
A structure comprising double-helical oligo RNA, represented by the following Structural Formula (1):

Structural Formula 1 A-X-R-Y-B

wherein A is a hydrophilic material, B is a hydrophobic material, X and Y are each independently a simple covalent bond or a linker-mediated covalent bond, and R is CTGF, Cyr61, or Plekho1-specific siRNA.
**[Item 8]**
The structure according to item 7, wherein the structure comprising double-helical oligo RNA represented by the following Structural Formula (2): 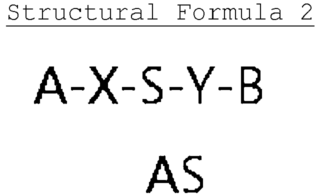 wherein S is sense strand of the siRNA of item 7, and AS is an antisense strand of item 7, A, B, X and Y are the same as being defined in item 7.
**[Item 9]**
The structure according to item 8, wherein the structure comprising double-helical oligo RNA represented by the following Structural Formula (3) or Structural Formula (4): 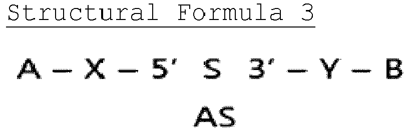 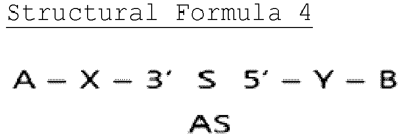 wherein A, B, X, Y S and AS are the same as being defined in item 8, and 5' and 3' mean 5' end and 3' end of the sense strand of siRNA.
**[Item 10]**
The structure according to any one of items 7 to 9, wherein the CTGF, Cyr61 or Plekho1-specific siRNA is siRNA according to any one of items 1 to 6.
**[Item 11]**
The structure according to any one of items 7 to 10, wherein the hydrophilic material has a molecular weight of 200 to 10,000.
**[Item 12]**
The structure according to item 11, wherein the hydrophilic material is any one selected from the group consisting of polyethylene glycol, polyvinyl pyrrolidone and polyoxazoline.
**[Item 13]**
The structure according to any one of items 7 to 10, wherein the hydrophilic material has the structure represented by the following Structural Formula (5) or Structural Formula (6):

Structural Formula 5 **(A'ₘ-J)ₙ**

Structural Formula 6 **(J-A'ₘ)ₙ**

wherein A' is a hydrophilic material monomer,
J is a linker for connecting between m hydrophilic material monomers, or a linker for connecting between m hydrophilic material monomers with siRNA, m is an integer of 1 to 15, n is an integer of 1 to 10,
the hydrophilic material monomer A' is a compound selected from the group consisting of compounds (1) to (3), and
the linker J is selected from the group consisting of PO₃⁻, SO₃ and CO₂.

| Compound (1) | Compound (2) | Compound (3) |
|---|---|---|
| 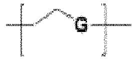 | 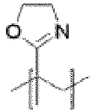 | 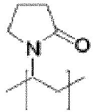 |
| G is CH₂, O, S or NH | | |

**[Item 14]**
The structure according to any one of items 7 to 10, wherein the hydrophobic material has a molecular weight of 250 to 1,000.
**[Item 15]**
The structure according to item 14, wherein the hydrophobic material is one selected from the group consisting of a steroid derivative, a glyceride derivative, glycerol ether, polypropylene glycol, C₁₂ to C₅₀ unsaturated or saturated hydrocarbon, diacylphosphatidylcholine, fatty acid, phospholipid and lipopolyamine.
**[Item 16]**
The structure according to item 15, wherein the steroid derivative is selected from the group consisting of cholesterol, cholestanol, cholic acid, cholesteryl formate, cholestanyl formate, and cholesteryl amine.
**[Item 17]**
The structure according to item 15, wherein the glyceride derivative is selected from the group consisting of mono-, di- and tri-glyceride.
**[Item 18]**
The structure according to any one of items 7 to 17, wherein the covalent bond represented by X or Y is non-degradable bond or a degradable bond.
**[Item 19]**
The structure according to item 18, wherein the non-degradable bond is amide bond or a phosphorylation bond.
**[Item 20]**
The structure according to item 18, wherein the degradable bond is a disulfide bond, an acid degradable bond, an ester bond, an anhydride bond, a biodegradable bond or an enzymatically degradable bond.
**[Item 21]**
The structure according to any one of items 7 to 20, further comprising a ligand bonded to the hydrophilic material, which is specifically bonded to a receptor that promotes target cell internalization through receptor-mediated endocytosis (RME).
**[Item 22]**
The structure according to item 21, wherein the ligand is selected from the group consisting of a target receptor-specific antibody, aptamer, peptide, N-acetyl galactosamine (NAG), glucose and mannose.
**[Item 23]**
The structure according to any one of items 7 to 20, further comprising amine group or polyhistidine introduced into the distal end bonded with the siRNA in the hydrophilic material.
**[Item 24]**
The structure according to item 23, wherein the amine group or polyhistidine is bonded to hydrophilic material or hydrophilic block with more than a linker.
**[Item 25]**

The structure according to item 23, wherein the amine group is one selected from the group consisting of primary to tertiary amine groups.
**[Item 26]**
The structure according to item 23, wherein the polyhistidine comprises 3 to 10 histidines.
**[Item 27]**
Nanoparticle(s) comprising the structure according to any one of items 7 to 26.
**[Item 28]**
The nanoparticle(s) according to item 27, wherein the structure comprising the double-helical oligo RNA containing siRNAs with different sequences, is mixed in the nanoparticle(s).
**[Item 29]**
A pharmaceutical composition comprising siRNA according to any one of items 1 to 6, the structure according to any one of items 7 to 26, or the nanoparticle(s) according to item 27 or 28 as an active ingredient.
**[Item 30]**
The pharmaceutical composition according to item 29, wherein the composition is for prevention or treatment of respiratory diseases.
**[Item 31]**
The pharmaceutical composition according to item 30, wherein the respiratory diseases is selected from the group consisting of asthma, idiopathic pulmonary fibrosis, chronic obstructive pulmonary disease (COPD), acute or chronic bronchitis, allergic rhinitis, cough and phlegm, acute lower respiratory tract infection, bronchitis, bronchiolitis, acute upper respiratory tract infection, pharyngitis, tonsillitis, and laryngitis.
**[Item 32]**
The pharmaceutical composition according to item 30, wherein the respiratory diseases is idiopathic pulmonary fibrosis or chronic obstructive pulmonary disease (COPD).
**[Item 33]**
A lyophilized formulation comprising the pharmaceutical composition according to any one items 29 to 32.
**[Item 34]**
A method of preventing or treating respiratory diseases comprising: administering siRNA, the structure comprising double-helical oligo RNA, the nanoparticle(s), the pharmaceutical composition or the formulation according to any one of items 1 to 33.
**[Item 35]**
The method according to item 34, wherein the respiratory diseases is selected from the group consisting of asthma, idiopathic pulmonary fibrosis, chronic obstructive pulmonary disease (COPD), acute or chronic bronchitis, allergic rhinitis, cough and phlegm, acute lower respiratory tract infection, bronchitis, bronchiolitis, acute upper respiratory tract infection, pharyngitis, tonsillitis, and laryngitis.
**[Item 36]**
The method according to item 35, wherein the respiratory diseases is idiopathic pulmonary fibrosis or chronic obstructive pulmonary disease (COPD).

## Claims

1. A structure comprising double-helical oligo RNA, represented by the following Structural Formula (1):
Structural Formula 1 A-X-R-Y-B
wherein A is a hydrophilic material, B is a hydrophobic material, X and Y are each independently a simple covalent bond or a linker-mediated covalent bond, and R is Cyr61-specific siRNA,
wherein the hydrophilic material has the structure represented by the following Structural Formula (5) or Structural Formula (6):
Structural Formula 5 (A'ₘ-J)ₙ
Structural Formula 6 (J-A'ₘ)ₙ
wherein A' is a hydrophilic material monomer,
J is a linker for connecting between m hydrophilic material monomers, or a linker for connecting between m hydrophilic material monomers with siRNA, m is an integer of 1 to 15, n is an integer of 1 to 10,
the hydrophilic material monomer A' is a compound selected from the group consisting of compounds (1) to (3), and
the linker J is selected from the group consisting of PO₃⁻, SO₃ and CO₂.
| Compound (1) | Compound (2) | Compound (3) |
|---|---|---|
| 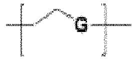 | 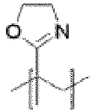 | 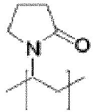 |
| G is O, S or NH | | |

2. The structure according to claim 1, wherein the structure comprising double-helical oligo RNA represented by the following Structural Formula (2): 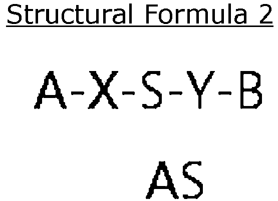 wherein S is sense strand of the siRNA of claim 1, and AS is an antisense strand of claim 1, A, B, X and Y are the same as being defined in claim 1.

3. The structure according to claim 2, wherein the structure comprising double-helical oligo RNA represented by the following Structural Formula (3) or Structural Formula (4): 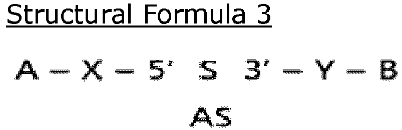 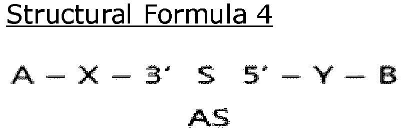 wherein A, B, X, Y, S and AS are the same as being defined in claim 2, and 5' and 3' mean 5' end and 3' end of the sense strand of siRNA.

4. The structure according to any one of claims 1 to 3, wherein the hydrophilic material has a molecular weight of 200 to 10,000, preferably the hydrophilic material is any one selected from the group consisting of polyethylene glycol, polyvinyl pyrrolidone and polyoxazoline.

5. The structure according to claim 1, wherein the Cyr61-specific siRNA is siRNA comprising a sense strand selected from the group consisting of SEQ ID NOs: 101 to 200 and 401 to 500 and an anti-sense strand complementary to the sense strand.

6. The structure according to any one of claims 1 to 5, wherein the hydrophobic material has a molecular weight of 250 to 1,000, preferably the hydrophobic material is one selected from the group consisting of a steroid derivative, a glyceride derivative, glycerol ether, polypropylene glycol, C₁₂ to C₅₀ unsaturated or saturated hydrocarbon, diacylphosphatidylcholine, fatty acid, phospholipid and lipopolyamine, preferably the steroid derivative is selected from the group consisting of cholesterol, cholestanol, cholic acid, cholesteryl formate, cholestanyl formate, and cholesteryl amine, preferably the glyceride derivative is selected from the group consisting of mono-, di- and tri-glyceride.

7. The structure according to any one of claims 1 to 6, wherein the covalent bond represented by X or Y is non-degradable bond or a degradable bond, preferably the non-degradable bond is amide bond or a phosphorylation bond, preferably the degradable bond is a disulfide bond, an acid degradable bond, an ester bond, an anhydride bond, a biodegradable bond or an enzymatically degradable bond.

8. The structure according to any one of claims 1 to 7, further comprising a ligand bonded to the hydrophilic material, which is specifically bonded to a receptor that promotes target cell internalization through receptor-mediated endocytosis (RME), preferably the ligand is selected from the group consisting of a target receptor-specific antibody, aptamer, peptide, N-acetyl galactosamine (NAG), glucose and mannose.

9. The structure according to any one of claims 1 to 8, further comprising amine group or polyhistidine introduced into the distal end bonded with the siRNA in the hydrophilic material, preferably the amine group or polyhistidine is bonded to hydrophilic material or hydrophilic block with more than a linker, preferably the amine group is one selected from the group consisting of primary to tertiary amine groups, preferably the polyhistidine comprises 3 to 10 histidines.

10. Nanoparticle(s) comprising the structure according to any one of claims 1 to 9, preferably the structure comprising the double-helical oligo RNA containing siRNAs with different sequences, is mixed in the nanoparticle(s).

11. A pharmaceutical composition comprising the structure according to any one of claims 1 to 9, or the nanoparticle(s) according to claim 10 as an active ingredient, preferably the composition is for prevention or treatment of respiratory diseases, preferably the respiratory diseases is selected from the group consisting of asthma, idiopathic pulmonary fibrosis, chronic obstructive pulmonary disease (COPD), acute or chronic bronchitis, allergic rhinitis, cough and phlegm, acute lower respiratory tract infection, bronchitis, bronchiolitis, acute upper respiratory tract infection, pharyngitis, tonsillitis, and laryngitis.

12. A lyophilized formulation comprising the pharmaceutical composition according to claim 11.
